# EUROPEAN PATENT APPLICATION

(11) **EP 1 801 097 A1**
(43) Date of publication of application: **27.06.2007**
(21) Application number: 05405723.7
(22) Date of filing: 23.12.2005
(51) Int. Cl.: C07C 69/92, C07C 229/42

(54) **Photocrosslinkable materials**

(71) Applicant: Rolic AG, 6301 Zug (CH)
(72) Inventor: Bachels, Thomas, 79639 Grenzach-Wyhlen (DE); Marck, Guy, 68440 Schlierbach (FR); Reichardt, Joachim, 79639 Grenzach-Wyhlen (DE); Seiberle, Hubert, 79576 Weil am Rhein (DE); Cherkaoui, Zoubair Mohammed, 4435 Niederdorf (CH); Muller, Olivier, 68610 Lautenbach (FR); Schuster, Andreas, 79110 Freiburg (DE); Studer, Peggy, 68330 Huningue (FR)
(74) Representative: Carreira-Staubli, Andrea

(57) **Abstract**

A diamine compound of formula (I) is proposed as well as polymers, copolymers, polyamic acids, polyamic acid esters, or polyimides based on such compound.

## Description

The invention relates to Diamine compounds, represented by the general formula (I), and also relates to oligomers and polymers from the class of polyamic acids, polyamic acid esters or polyimides (and any mixtures thereof) obtained by the reaction of a Diamine compound represented by the general formula (I) and optionally of one or more additional other Diamines, with one or more tetracarboxylic acid anhydrides, and to the use of these Diamine compounds, oligomers and polymers for the preparation of orientation layers for liquid crystals and in the construction of unstructured and structured optical elements and multi-layer systems.

Liquid crystal displays (LCDs) are becoming increasingly dominant in advanced visualization devices. LCDs offer favourable characteristics with respect to image quality (high luminance, high resolution, colour and grey scale capability), power consumption as well as dimensions and weight (flat panel displays). The use of commercial LCDs has become widespread, e.g. in automotive and telecommunication instruments, as well as in monitors of notebooks, desktop computers, television sets, etc. Today the need for LCDs in television applications is rapidly growing. Recently developed LCD modes possess high potentials in achieving fast response times, wide viewing angles and high luminance. Amongst other newly developed LCD modes, the MVA (multi-domain vertical alignment) mode appears to be the most promising for the use in modem television applications.

In the MVA mode the liquid crystal molecules are usually nearly vertically aligned with respect to the surface of the substrates. By using protrusions (or other alignment subdivisions) on the surface of the substrate, the liquid crystal molecules become locally pre-tilted within a single cell in more than one direction, leading to domains switchable in different directions. This multi-domain configuration exhibits very good display performance, with wide viewing angles of up to 160° in any direction, short response times (below 20 ms), high contrast ratios (up to 700:1) and high brightness. However, by means of using protrusions only, it is difficult to clearly define the domain space within a single pixel. Therefore the MVA mode demands additional manufacturing steps to ensure shape effects as well as electrical field effects on both the upper and lower substrate; hence all in all leading to complex manufacturing procedures.

In order to by-pass this technical challenge, the availability of an alignment layer would be desirable, which directly leads to pre-defined alignment directions within each pixel domain and having well controllable off-axis angles with respect to the normal axis of the substrate.

Methods for the preparation of orientation layers for liquid crystal materials are well known to the skilled person. Customarily used unlaxially rubbed polymer orientation layers, such as for example polyimides, however, do have a series of disadvantages, like the formation and deposition of dust during the rubbing process and concomitant partial destruction of the thin film transistors. Scratches due to brushing is another issue associated with this technique, which is particularly evident when the pixels are of the order of 10 microns or even lower, like e.g. in micro-display applications. Because of the strong optical magnification, which is required to visualize the displayed information, scratches easily become visible and are also the cause for the reduction of the contrast level. Furthermore, the rubbing process does not allow the production of structured layers.

The production procedure for obtaining orientation layers in which the direction of orientation is Induced by irradiation with polarized light is not faced With the problems inherent to the rubbing process. With the irradiation technique it is furthermore also possible to create areas having different orientation and thus to structure the orientation layer as described for example in Jpn. J. Appl. Phys., 31 (1992), 2155-64 (Schadt et al).

Using the linearly photo-polymerizable alignment (LPP) technique, the possibility of realizing a four-domain vertical aligned nematic (VAN) LCD was demonstrated some years ago (K. Schmitt, M. Schadt; Proceedings of EuroDisplay 99, 6-9 September, 1999). The four-domain VAN-LCD exhibits an excellent off-state angular brightness performance.

Apart from the current display performance requirements to be fulfilled in modem TV applications, the use of appropriate LPP materials is furthermore also guided by the necessity to achieve specific optical and electro-optical properties, e.g. with respect to the compatibility with the TFT (thin film transistors). Other important characteristics of the materials must also be taken into consideration, i.e. those crucial parameters directly related to and dependent on the molecular properties of the material.

Primarily such characteristics are:
- High voltage holding ratio (VHR), i.e. VHR of > 90 % (measured at 80° C)
- High stability of the induced pre-tilt angle against light and heat
- Low alignment energy profile (short irradiation time and/or low Irradiation energy)

In the case of LCDs of thin-film transistor type a certain amount of charge is applied over the course of a very short period of time to the electrodes of a pixel and must not subsequently drain away by means of the resistance of the liquid crystal. The ability to hold that charge and thus to hold the voltage drop over the liquid crystal is quantified by what is known as the "voltage holding ratio" (VHR). It is the ratio of the RMS-voltage (root mean square voltage) at a pixel within one frame period and the initial value of the voltage applied.

Photo-reactive materials for orientation layers with improved voltage holding ratios (VHR) are described in WO-A-99/49360, US 6,066,696, US 6,027,772, WO-A-99/15576 and WO-A-99/51662. In WO-A-99/49360, US 6,066,696 and US 6,027,772 blends of polymeric compounds are described, containing photo-reactive polymers and polyimides.

In WO-A-99/15576 and WO-A-99/51662 polyimides having photo-reactive cinnamate groups incorporated in their side chains are described. WO-A-99/15576 for instance discloses photo-active polymers which contain as side-chain specific photo-cross-linkable groups and of which a typical monomer unit is 6-{2-methoxy-4-[(1E)-3-methoxy-3-oxoprop-1-enyl]phenoxy}hexyl 3,5-Diaminebenzoate.

In the above cited references it was generally demonstrated that in order to achieve the aforementioned important parameters, molecular structures combining firstly a polyamic/polyimide backbone (i.e. delivering molecular polarity) and secondly side chains with an incorporated photo-reactive group, such as a cinnamic acid residue, are suitable for the general concept of planar orientation [requiring only slight pretilt angles, like e.g. being used in TN (twisted nematic) devices], However, these types of molecular structures, primarily developed for TN applications, cannot directly be utilized in MVA applications. From the comparative examples provided below, it can be seen that when molecular structures, providing high voltage holding ratios in the TN mode, are slightly modified in order to induce vertical alignment, for example simply by increasing the length of a peripheral alkyl chain, a strong drop of the VHR value is observed. This indicates that in case of the MVA mode not only the polyamic/polyimide backbone (being sufficient in case of the TN mode) has to be taken into consideration, but also other molecular parameters. It has surprisingly been found, that in addition to the polyamic/polyimide backbone, the introduction of an organofluorine group into a peripheral position of the polymer side groups having specific molecular architecture plays a predominant role in obtaining MVA materials having optimised properties, such as the required high voltage holding ratios, the adjustable pre-tilt angles required for the MVA mode and their stability to light and heat

Thus, the present invention relates to diamine compound of formula (I): 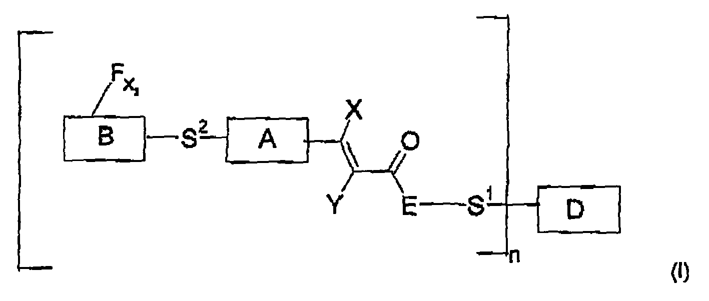 wherein,
- A: represents a unsubstituted or substituted carbocyclic or heterocyclic aromatic group selected from a monocyclic ring of five or six atoms, two adjacent monocyclic rings of five or six atoms, a bicyclic ring system of eight, nine or ten atoms, or a tricyclic ring system of thirteen or fourteen atoms;
- F: is fluorine, and
- x₁: is an integer from 1 to 15, preferably an integer from 1 to 10; more preferably 1, 2, 3, 4, 5, 6, 7, 8 or 9 and most preferred 3, 4, 5 or 7;
- B: represents a straight-chain or branched C₁-C₁₈alkyl, which is unsubstituted or substituted by di-(C₁-C₁₈alkyl)amino, C₁-C₈alkyloxy, nitro, cyano and/or chlorine or fluorine; and wherein one or more, preferably non-adjacent, -CH₂-group may independently be replaced by a linking group;
- D: represents unsubstituted or substituted aliphatic, aromatic or alicyclic diamine group having from 1 to 40 carbon atoms,
- E: represents an aromatic group, an oxygen atom, a sulphur atom, -NH-, -N(C₁-C₆alkyl)-, -CR²R³,
wherein R² and R³ are independently from each other hydrogen or a cyclic, straight-chain or branched, substituted or unsubstituted C₁-C₂₄alkyl, wherein one or more, preferably non-adjacent, -CH₂- groups may be replaced by a linking group; and with the proviso that at least one of R² and R³ is not hydrogen;
- S¹,S²: each independently from each other represents a spacer unit;
- X, Y: each independently from each other represents hydrogen, fluorine, chlorine, cyano, unsubstituted or with fluorine substituted C₁-C₁₂alkyl, in which one or more, preferably non-adjacent, -CH₂- groups may be replaced by a linking group;
- n: is 1, 2, 3 or 4,
with the proviso that if n is 2, 3, or 4, each A, B, x₁, D, E. S¹, S², X. Y may be identical or different.

The term "linking group°, as used in the context of the present invention preferably denotes -0-, -CO, -CO-O-, -O-CO-, 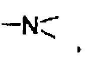 -NR¹-, -NR¹-CO-, -CO-NR¹, -NR¹ -CO-O-, -O-CO-NR¹-, -NR¹ -CO-NR¹ -CN=CH-, -C≡C-, -O-CO-O-, and -Si(CH₃)₂-O-Si(CH₃)₂-, and wherein:
R¹ represents a hydrogen atom or C₁-C₆alkyl;
   with the proviso that oxygen atoms of linking groups are not directly linked to each other.

The term "spacer unit" as used in the context of the present invention, is preferably a single bond, C₁-C₂₄alkylen, wherein one or more, preferably non-adjacent, -CH₂-groups may be replaced by a linking group and/or an alicyclic, aromatic, unsubstituted or substituted carbocyclic or heterocyclic group connected via bridging groups, preferably at the opposite positions to each other.

A bridging group as used in the context of the present invention is preferably selected from -CH(OH)-, -CH₂-, -CH(CH₃)-, -C(CH₃)₂-, -CO-, -CH₂(CO)-, -SO-, -CH₂(SO)-, -SO₂-, -CH₂(SO₂)-, -COO-, -OCO-, -COCF₂-, -CF₂CO-, -S-CO-, -CO-S-, -SOO-, -OSO-, -SOS-, -O-CO-O, -CH₂-CH₂-, -OCH₂-, -CH₂O-, -CH=CH-, -C=C-, -CH=CH-COO-, -OCO-CH=CH-, -CH=N-, -C(CH₃)=N-, -N=N- or a single bond.

Alkyl, alkyloxy, alkylcarbonyloxy, alkyloxycarbonyloxy, alkyloxycarbonyloxy methacryloyloxy, alkylvinyl, alkylvinyloxy and alkylallyloxy and alkylene, as used in the context of the present invention denotes with their alkyl residue, respectively their alkylene residue, a cyclic, straight-chain or branched, substituted or unsubstituted alkyl, respectively alkylene, in which one or more, preferably non-adjacent, -CH₂-group may be replaced by a linking group.

Further, the alkyl residue is for example C₁-C₄₀alkyl, especially C₁-C₃₀alkyl, preferably C₁-C₂₀alkyl, more preferably C₁-C₁₆alkyl, most preferably C₁-C₁₀alkyl and especially most preferably C₁-C₆alkyl. Accordingly alkylen is for example C₁-C₄₀alkylen, especially C₁-C₃₀alkylen, preferably C₁-C₂₀alkylen, more preferably C₁-C₁₆alkylen, most preferably C₁-C₁₀alkylen and especially most preferably C₁-C₆alkylen,

In the context of the present invention the definitions for alkyl given below, are applicable to alkylene in analogy.

C₁-C₆alkyl is for example methyl, ethyl, propyl, isopropyl, butyl, sec.-butyl, tert-butyl, pentyl or hexyl.

C₁-C₁₀alkyl is for example methyl, ethyl, propyl, isopropyl, butyl, sec.-butyl, tert.-bertyl, pentyl, hexyl, heptyl, octyl, nonyl, decyl.

C₁-C₁₆alkyl is for example methyl, ethyl, propyl, isopropyl, butyl, sec.-butyl, tert.-butyl, pentyl, hexyl, heptyl, octyl, nonyl, decyl, undecyl, dodecyl, tridecyl, tetradecyl, pentadecyl or hexadecyl.

C₁-C₂₀alkyl is for example methyl, ethyl, propyl, isopropyl, butyl, sec.-butyl, tert.-butyl, pentyl, hexyl, heptyl, octyl, nonyl, decyl, undecyl, dodecyl, tridecyl, tetradecyl, pentadecyl, hexadecyl, heptadecyl, octadecyl, nondecyl, eicosyl.

C₁-C₂₄alkyl is for example methyl, ethyl, propyl, isopropyl, butyl, sec.-butyl, tert.-butyl, pentyl, hexyl, heptyl, octyl, nonyl, decyl, undecyl, dodecyl, tridecyl, tetradecyl, pentadecyl, hexadecyl, heptadecyl, octadecyl, nondecyl, eicosyl.

C₁-C₃₀alkyl is for example methyl, ethyl, propyl, isopropyl, butyl, sec.-butyl, tert.-butyl, pentyl, hexyl heptyl, octyl, nonyl, decyl, undecyl, dodecyl, tridecyl, tetradecyl, pentadecyl, hexadecyl, heptadecyl, octadecyl, nondecyl, eicosyl, heneicosyl, tricosyl, tetracosy, pentacosyl, hexacosdy, heptacosyl, octacosyl, nonacosy or triacontyl.

C₁-C₄₀alkyl is for example methyl, ethyl, propyl, isopropyl, butyl, sec.-butyl, tert.-butyl, pentyl, hexyl, heptyl, octyl, nonyl, decyl, undecyl, dodecyl, tridecyl, tetradecyl, pentadecyl, hexadecyl, heptadecyl, octadecyl, nondecyl, eicosyl, heneicosyl, tricosyl, tetracosy, pentacosyl, hexacosdy, heptacosyl, octacosyl, nonacosy, triacontyl or tetracontyl.

C₁-C₂₀acryloyloxy, preferably C₁-C₁₀ acryloyloxy and more preferably C₁-C₈ acryloyloxy is for example methacryloyloxy, ethacryloyloxy, propacryloyloxy, ethacryloyloxy, propacryloyloxy, isopropacryloyloxy, butacryloyloxy, sec.-butacrytoytoxy, tert.-butacryloyloxy, pentacryloyloxy, hexacryloyloxy, haptacryloyloxy, octacryloyloxy, nonacryloyloxy, decacryloyloxy, undecacryloyloxy, dodecacryloyloxy, tridecacryloyloxy, tetradecacryloyloxy, pentadecacryloyloxy, hexadecacryloyloxy, heptadecacryloyloxy, octadecacryloyloxy, nondecacryloyloxy, eicosacryloyloxy.

An aliphatic group Is for example alkyl, alkyloxy, alkylcarbonyloxy, acryloyloxy alkyloxycarbonyloxy, alkyloxycarbonyloxy methacryloyloxy, alkylvinyl, alkylvinyloxy or alkylallyloxy.

A non-aromatic carbocyclic or heterocyclic group is for example ring systems, with 3 to 30 carbon atoms, as for example cyclopropane, cyclobutane, cyclopentane, cyclopentene, cyclohexane, cyclohexene, cyclohexadiene, decaline, tetrahydrofuran, dioxane, pyrrolidine, piperidine or a steroidal skeleton such as cholesterol.

An aromatic group as used in the context of the present invention, preferably denotes substituted or unsubstituted carbocyclic and heterocyclic aromatic groups.

A carbocyclic or heterocyclic aromatic group incorporating preferably five, six, ten or 14 ring atoms, as for example furan, benzene, pyridine, pyrimidine, naphthalene, phenanthrene, biphenylene or tetraline units, preferably naphthalene, phenanthrene, biphenylene or phenylene, more preferably naphthalene, biphenylene or phenylene, and most preferably phenylene.

The carbocyclic or heterocyclic aromatic group is for example an unsubstituted or mono- or poly-substituted. Preferred substitutents of carbocyclic or heterocyclic aromatic groups are at least one halogen, hydroxyl, a polar group, acryloyloxy, alkoxy, alkylcarbonyloxy, alkyloxycarbonyloxy, alkyloxocarbonyloxy methacryloyloxy, vinyl, vinyloxy and/or allyloxy group, having preferably from 1 to 20 carbon atoms, and more preferably having from 1 to 10 carbon atoms. Preferred polar groups are nitro, cyano or a carboxy group, and/or a cyclic, straight-chain or branched C₁-C₃₀alkyl, which is unsubstituted, mono- or poly-substituted. Preferred substitutents of C₁-C₃₀alkyl are methyl, fluorine and/or chlorine, wherein one or more, preferably non-adjacent, -CH₂-group may independently be replaced by a linking group. Preferably, the linking group is selected from -O-, -CO-, -COO- and/or -OCO-.

A monocyclic ring of five or six atoms is for example furan, benzene, preferably phenylene, pyridine, pyrimidine.

A bicyclic ring system of eight, nine or ten atoms is for example naphthalene or biphenylene.

A tricyclic ring system of thirteen or fourteen atoms is for example phenanthrene or tetraline units.

An alicyclic group as used in the context of the present invention, preferably denotes optionally substituted non-aromatic carbocyclic or heterocyclic ring systems, with 3 to 3D carbon atoms, as for example cyclopropane, cyclobutane, cyclopentane, cyclopentene, cyclohexane, cyclohexene, cyclohexadiene, decaline, tetrahydrofuran, dioxane, pyrrolidine, piperidine or a steroidal skeleton such as cholesterol.

The term "aromatic", as used in the context of the present invention, preferably denotes unsubstituted or substituted carbocyclic and heterocyclic groups, incorporating five, six, ten ot 14 ring atoms, e.g. furan, benzene, pyridine, pyrimididne, naphthalenen, phenanthrene, biphenylene or tetraline.

The term "phenylene", as used in the context of the present invention, preferably denotes a 1,2-, 1,3- or 1,4-phenylene group, which is optionally substituted, It is preferred that the phenylene group is either a 1,3- or a 1,4-phenylene group. 1,4-phenylene groups are especially preferred.

The term "halogen" denotes a chloro, fluoro, bromo or iodo substituent, preferably a chloro or fluoro substituent.

The term "polar group", as used in the context of the present invention primarily denotes a group like a nitro, cyano, or a carboxy group.

The term "hetero atom", as used in the context of the present invention primarily denotes oxygen, sulphur and nitrogen, preferably oxygen and nitrogen, in the latter case preferably in the form of -NH-.

The term "optionally substituted" as used in the context of the present invention primarily means substituted by lower alkyl, lower alkoxy, hydroxy, halogen or by a polar group as defined above.

The term "diamine" or "diamine compound" is to be understood as designating a chemical structure which has at least two amino groups, i.e. which may also have 3 or more amino groups. The at least two amino groups are preferably able to react with e.g. anhydrides as outlined in more detail below.

With respect to straight chain or branched alkyl, alkylene, alkoxy, alkoxycarbonyl, alkylcarbonyl, alkylcarbonyloxy groups it is repeatedly pointed out that some or several of the -CH₂- groups may be replaced e.g. by heteroatoms, but also by other groups. In such cases it is generally preferred that such replacement groups are not directly linked to each other. It is alternatively preferred that heteroatoms, and in particular oxygen atoms are not directly linked to each other.

With respect to the possibility of having several side-chains (i.e. n>1) connected to residue D, it has to be mentioned that the side chains [i.e, structures (I) without the group D] can either be linked to the group D at one atomic position within group D, e.g. two or three side chains connected to one single carbon atom within group D, or they can be linked to group D at different atomic positions within group D, e.g. at adjacent atomic positions within group D but also spaced further apart.

Further preferred is compound (I), wherein if n>1, then the side chains [i.e. structures (I) without the group D] can either be linked to the group D at one atomic position within group D, e.g. two or three side chains connected to one single carbon atom within group D, or they can be linked to group D at different atomic positions within group D, e.g. at adjacent atomic positions within group D but also spaced further apart.

Preferably, A is unsubstituted or substituted phenanthrylene, naphthylene, biphenylene or phenylene, which is preferably mono- or poly-substituted by a halogen atom, hydroxy group and/or by a polar group, wherein the polar group is preferably nitro, cyano, carboxy; and/or by acryloyloxy, methacryloyloxy, vinyl, vinyloxy, allyl, allyloxy, and/or by a cyclic, straight-chain or branched alkyl, which is unsubstituted, mono- or poly-substituted by fluorine and/or chlorine, having from 1 to 20 carbon atoms, wherein one or more, preferably non-adjacent -CH₂- groups may independently be replaced by a linking group and or an aromatic or an alicyclic group, preferably the linking group is selected from -O-, -CO-, -CO-O, -O-CO-,

More preferably A is substituted or unsubstituted naphthylene, biphenylene or phenylene, which are mono- or poly-substituted by a halogen atom, hydroxy group and/or by acryloyloxy, methacryloyloxy, straight-chain or branched alkyl, alkoxy, alkylcarbonyloxy, and/or alkyloxycarbonyl groups having from 1 to 20 carbon atoms.

Most preferably A is substituted or unsubstituted phenylene, preferably 1,4-phenylen, which are mono- or poly-substituted by a halogen atom, and/or by acryloyloxy or methacryloyloxy, and/or by an alkoxy, alkylcarbonyloxy, and/or alkyloxycarbonyl groups having from 1 to 10 carbon atoms.

Preferably B is a straight-chain or branched C₁-C₁₀alkyl, wherein one or more, preferably non-adjacent, -CH₂- group may be replaced by a group selected from -O-, -CO, -CO-O-, -O-CO-, -NR¹-, -NR¹-CO-, -CO-NR¹-, -NR¹-CO-O-, -O-CO-NR¹-, -NR¹-CO-NR¹-, -CH=CH-, -C≡C-, -O-CO-O-, and -Si(CH₃)2-O-Si(CH₃)₂-, an aromatic or an alicyclic group; and wherein:
R¹ represents a hydrogen atom or C₁-C₆alkyl;
with the proviso that oxygen atoms are not directly linked to each other.

More preferably, B is a straight-chain or branched C₁-C₁₂alkyl, wherein one or more, preferably non-adjacent, the -CH₂- group may be replaced by a group selected from from -O-, -CO, -CO-O-, -O-CO-, -NR¹-, -NR¹-CO-, -CO-NR¹- or -CH=CH- wherein:
R¹ represents a hydrogen atom or C₁-C₆alkyl;
with the proviso that oxygen atoms are not directly linked to each other.

Most preferably, B is a straight-chain or branched C₁-C₅alkyl, wherein one or more, preferably non-adjacent, the -CH₂- group may be replaced by a group selected from from -O-, -CO, -CO-O-, -O-CO-, -NR¹-, -NR¹-CO-, -CO-NR¹- or -CH=CH- wherein:
R¹ represents a hydrogen atom or C₁-C₆alkyl;
with the proviso that oxygen atoms are not directly linked to each other.

Especially most preferably, B is a straight-chain or branched C₁-C₈alkyl, wherein one or more, preferably non-adjacent, the -CH₂- group may be replaced by a group selected from -O-, -CO-, -CO-O-, -O-CO-, - and -CH=CH-, with the proviso that oxygen atoms are not directly linked to each other,

Preferably Fx₁ and Bare:
trifluoromethyl; 2,2,2-trifluoroethyl; pentafluoroethyl; 2,2-tetrafluorethyl; 3,2-tetrafluorethyl; 3,3,3-trifluoropropyl; 2,2,3,3-tetrafluoropropyl; 2,2,3,3,3-pentafluoropropyl; hexafluoropropyl; heptafluoropropyl; 4,4,4-trifluorobutyl; tetrafluorobutyl; 3,3,4,4,4-pentafluorobutyl; hexafluorobutyl; 2,2,3,3,4,4,4-heptafluorobutyl; 5,5,5-trifluoropentyl; tetrafluoropentyl; 4,4,5,5,5-pentafluoropentyl; hexafluoropentyl; 3,3,4,4,5,5,5-heptafluoropentyl; 6,6,6-trifluorohexyl; tetrafluorohexyl; 5,5,6,6,6-pentafluorohexyl; hexafluorohexyl; 4,4,5,5,6,6,6-heptafluorohexyl;
trifluoromethoxy; 2,2,2-trifluoroethoxy; pentafluoroethoxy; 1,1,2,2-tetrafluoroethoxy; 2,2,2,1-tetrafluoroethoxy; 3,3,3-trifluoropropoxy; 2,2,3,3-tetrafluoropropoxy, 2,2,3,3,3-pentafluoropropoxy; hexafluoropropoxyl; heptafluoropropoxy; 4,4,4-trifluorobutoxy; tetrafluorobutoxy; 3,3,4,4,4-pentafluorobutoxy; 2,2,3,3,4,4-hexafluorobutoxy; 2,2,3,3,4,4,4-heptafluorobutoxy; 5,5,5-trifluoropentoxy; tetrafluoropentoxy; 4,4,5,5,5-pentafluoropentoxy; hexafluoropentoxy; 3,3,4,4,5,5,5-heptafluoropentoxy; 6,6,6-trifluorohexoxy; tetrafluorohexoxy; 5,5,6,6,8-pentafluorohexoxy; hexafluorohexoxy; 4,4,5,5,6,6,6-heptafluorahexoxy;
trifluoromethionyloxy; 2,2,2-trifluoroethionyloxy; pentafluoroethionyloxy; 1,1,2,2-tetrafluorethionyloxy; 2,2,2,1-tetrafluorethionyloxy; 3,3,3-trifluoropropionyloxy; tetrafluoropropionyloxy; 2,2,3,3,3-pentafluoropropionyloxy; hexafluoropropionyloxy; 1,1,2,2,3,3,3-heptafluoropropionyloxy; 4,4,4-trifluorobutonyloxy; tetrafluorobutionyloxy; 3,3,4,4,4-pontafluorobutionyloxy; hexafluorobutionyloxy; 2,2,3,3,4,4,4-heptafluorobutionyloxy; 5,5,5-trifluoropentionyloxy; tetrafluoropentionyloxy; 4,4,5,5,5-pentafluoropentionyloxy; hexafluoropentionyloxy; 3,3,4,4,5,6,5-heptafluoropentionyloxy; 6,6,6-trifluorohexionyloxy; tetrafluorohexionyloxy; 5,5,6,6,6-pentafluorohexionyloxy; hexafluorohexionyloxy; 4,4,5,5,6,6,6-heptafluorohexionyloxy; trifluoroacetyl;
4,4,4-trifluorobut-2-enyl; 5,5,5-trifluoropent-1-onyl; 6,6,6-trifluorohex-1-enyl; 7,7,7-trifluorohept-1-enyl;
trifluoroacetylaminomethoxy; trifluoroacetylaminoethoxy; trifluoroacetylaminopropoxy; trifluoroacetylaminobutoxy;
2-fluoroethyl; 3-fluoropropyl; 4-fluorobutyl; 5-fluoropentyl; 6-fluorohexyl; 2-fluoroethoxy; 3-fluoropropoxy; 4-fluorobutoxy; 5-fluoropentoxy; 6-fluorohexyloxy; 4-fluorobut-1-enyl; 5-fluoropent-1-anyl; 6 fluorohex-1-enyl;
7-fluorohept-1-enyl;
4,4,4-trifluoro-3-(trifluoromethyl)butoxy; 4,5,5-trifluoropent-4-enoxy; 4,5,5-trifluoropent-4-enoyloxy; 5,6,6-trifluorohex-5-enoxy or 5,6,6-trifluoropent-5-enoloxy.

D is preferably selected from formula (III):

HN(R⁵)-(sp¹)ₖ₁-(x¹)ₜ₁-(Z³-C³)ₐ₃-(Z⁴-C⁴)ₐ₄-(X²)ₜ₂-(Sp²)ₖ₂- N(R⁶)H (III)

wherein:
- R⁵, R⁶: each independently from each other represents a hydrogen atom or C₁-C₆alkyl;
- Sp¹, Sp²: each independently represents an optionally substituted straight-chain or branched C₁-C₂₀alkylene, in which one or more, preferably non-adjacent, CH₂-atoms may be replaced by a linking group, and
- k¹, k²: each independently is an integer having a value of 0 or 1; and
- X¹, X²: each independently represents a linking spacer, preferably selected from -O-, -S-, -NH-, -N(CH₃)-, -CH(OH)-, -CO-, -CH₂(CO)-, -SO-, -CH₂(SO)-, -SO₂-, -OH₂(SO₂)-, -COO-, -OCO-, -OCO-O-, -S-CO-, -CO-S-, -SOO-, -OSO-. -SOS-, -CH₂-CH₂-, -OCH₂-, -CH₂O-, -CH=CH-, or -C≡C- or a single bond; and
- t¹, t²: each independently is an integer having a value of 0 or 1; and
- C³, C⁴: each independently represents a non-aromatic, aromatic, substituted or unsubstituted carbocyclic or heterocyclic group, and
- Z³: represents a bridging group; and.
- Z⁴: represents a bridging group, or represents a substituted or unsubstituted straight-chain or branched C₁-C₂₀alkylene group, in which one or more, preferably non-adjacent, -CH₂- groups may be replaced by a heteroatom and/or by a bridging group; and
- a³, a⁴: are independently integers from 0 to 3, such that a³ + a⁴ *≤* 4; and wherein
- D: is at least once linked to at least once group S¹ in formula (I) via group Sp¹ and/or group C³ and/or group Z⁴ and/or group C⁴ and/or group Sp²; and at least one of k¹, k², a³ and a⁴ is not equal to zero; and wherein
linking group and bridging group are as defined above;
and wherein preferably, if n>1, then the side chains [i.e. structures (I) without the group D] can either be linked to the group D at one atomic position within group D, e.g. two or three side chains connected to one single carbon atom within group D, or they can be linked to group D at different atomic positions within group D, e.g. at adjacent atomic positions within group D but also spaced further apart.

More preferably D is selected from formula (III), wherein:
- k¹, k²: are 0 or 1, and
- t¹, t²: are 0, and
- C³, C⁴: independently from each other are selected from a compound of group G²,
wherein group G² denotes: 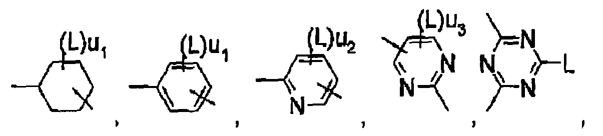 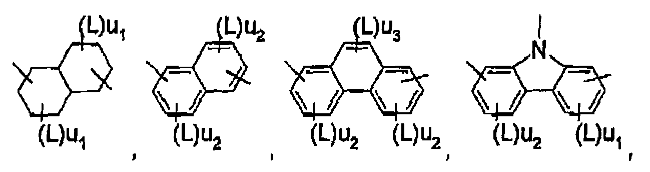 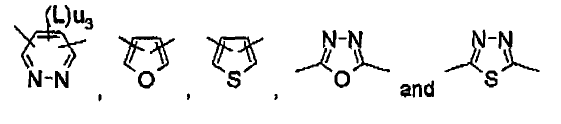 wherein:
- "___": denotes the connecting bonds of C³ and C⁴ to the adjacent groups; and
- L: is -CH₃, -COCH₃, -OCH₃, nitro, cyano, halogen, CH₂=CH-, CH₂=C(CH₃)-, CH₂=CH-(CO)O-, CH₂=CH-O-, CH₂=C(CH₃)-(CO)O- or CH₂=C(CH₃)-O-,
- u₁: is an integer from 0 to 4; and
- u₂: is an integer from 0 to 3; and
- u₃: is an integer from 0 to 2; and
- Z³: represents a group selected from -CH(OH)., -CH(CH₃)-, -C(CH₃)₂-, -CO-, -COO-, -COCF₂-, -CF₂CO- or a single bond; and
- Z⁴: has one of the meanings of Z³ or represents an unsubstituted or substituted straight chain or branched C₁-C₁₄alkylene group, in which one or more, preferably non-adjacent, -CH₂- groups may be replaced by an oxygen atom and/or one or more carbon-carbon single bonds are replaced by a carbon-carbon double or a carbon-carbon triple bond; and
- a³, a⁴: each independently represents an integer from 0 to 2 such that a³ + a⁴ ≤ 3;
- D: is linked to S₁ in formula (I) via group Sp¹ and/or group C³ and/or group Z⁴ and/or group C⁴ and/or group Sp²; and with the proviso that at least one of k¹, k², a³ and a⁴ is not equal to zero;
and wherein preferably, if n>1, then the side chains [i.e. structures (I) without the group D] can either be linked to the group D at one atomic position within group D, e.g. two or three side chains connected to one single carbon atom within group D, or they can be linked to group D at different atomic positions within group D, e.g. at adjacent atomic positions within group D but also spaced further apart.
- D: is most preferably selected from the following group of structures: substituted or unsubstituted o-phenylenediamine, p-phenylenediamine, m-phenylenediamine, biphenyldiamine, aminophenyl-Z⁴-phenylamino, wherein Z⁴ has the same meaning as given above; naphthylenediamine, benzidine, diaminofluorene, 3,4-diaminobenzoic acid, 3,4-diaminobenzyl alcohol dihydrochloride, 2,4-diaminobenzoic acid, L-(+)-threo-2-amino-1-(4-aminophenyl)-1,3-propanediol, p-aminobenzoic acid, [3,5-3h]-4-amino-2-methoxybenzoic acid, L-(+)-threo-2-(N,N-dimethylamino)-1-(4-aminophenyl)-1,3-propanediol, 2,7-diaminofluorene, 4,4'-diaminooctafluorobiphenyl, 3,3'-diaminobenzidine, 2,7-diamino-9-fluorenone, 3,5,3',5'-tetrabromo-biphenyl-4,4'-diamine, 2,2'-dichloro[1,1'-biphenyl]-4,4'-diamine, 3,9-diamino-1,11-dimethyl-5,7-dlhydro-dibenzo(a,c)cyclohepten-6-one, dibenzo(1,2)dithiine-3,8-diamine, 3.3'-diaminobenzophenone, 3,3'-diaminodiphenylmethane, 4,4-bis-(3-amino-4-hydroxyphonyl)-valeric acid, 2,2-bis(3-amino-4-hydroxyphenyl)hexafluoropropane, 2,2-bis(3-amino-4-methylphenyl)hexafluoropropane, tetrabromo methylenedianiline, 2,7-diamino-9-fluorenone, 2,2-bis(3-aminophonyl)-hexafluoropropane, bis-(3-amino-4-chloro-phenyl)-methanone, bis-(3-amino-4-dimethylamino-phonyl)-methanone, 3-[3-amino-5-(trifluoromethyl)benzy)]-5-(trifluoromethyl)aniline, 1,5-diaminonaphthalane, benzidine-3,3'-dicarboxylic acid, 4,4'-diamino-1,1'-binaphthyl, 4,4'-diaminodiphenyl-3,3'-diglycolic acid, dihydroethidium, o-dianisidine, 2,2'-dichloro-5,5'-dimethoxybenzidine, 3-methoxybenzidine, 3,3'-dichlorobenzidine (diphenyl-d6), 2,2'-bis(trifluoromethyl)benzidine, 3,3'-bis(trifluoromethyl)benzidine, 3,3'-dichlorobenzidine-d6, tetramethylbenzidine, di-(aminophenyl)alkylen
and from amino compounds listed below, which do not carry two amino groups and are taken as derivatives with at least one additional amino group: aniline, 4-amino-2,3,5,6-tetrafluorobenzoic add, 4-amino-3,5-diiodobenzoic acid, 4-amino-3-methylbenzoic acid, 4-amino-2-chlorobenzoic acid, 4-aminosalicylic acid, 4-aminobenzoic acid, 4-aminophthalic acid, 1-(4-aminophenyl)ethanol, 4-aminobenzyl alcohol, 4-amino-3-methoxybenzoic acid, 4-aminophenyl ethyl carbinol, 4-amino-3-nitrobenzoic acid, 4-amino-3,5-dinitrobenzoic acid, 4-amino-3,5-dichlorobenzoic acid, 4-amino-3-hydroxybenzoic acid, 4-aminobenzyl alcohol hydrochloride, 4-aminobenzoic acid hydrochloride, pararosaniline base, 4-amino-5-chloro-2-methaxybenzoic add, 4-(hexafluoro-2-hydroxyisopropyl)aniline, piperazine-p-amino benzoate, 4-amina-3,5-dibromobenzoic acid, isonicotinic acid hydrazide p-amino-salicylate salt, 4-amino-3,5-diiodosalicylic acid, 4-amino-2-methoxybenzoic acid, 2-[2-(4-aminophenyl)-2-hydroxy-1-(hydroxymethyl)ethyl]isoindoline-1,3-dione, 4-amino-2-nitrobenzoic acid, ethyl 2-(4-aminophenyl)-3,3,3-trifluoro-2-hydroxypropanoate, ethyl 2-(4-amino-3-methylphenyl)-3,3,3-trifluoro-2-hydroxypropanoate, ethyl 2-(4-amino-3-methoxyphenyl)-3,3,3-trifluoro-2-hydroxypropanoate, 4-aminonaphthalene-1,8-dirarboxylic acid, 4-amino-3-chloro-5-methylbenzoic acid, 4-amino-2,6-dlmethylbenzoic acid, 4-amino-3-fluorobenzoic acid, 4-amino-6-bromo-2-methoxybenzenecarboxylic acid,
3,3'-tolidine-5-sulfonic acid,
or their derivatives, again with the proviso that compounds listed which do not carry two amino groups are taken as derivatives with at least one additional amino group.

The diamine groups D are commercial available or accessible by known methods. The second amino group is for example by substitution reaction accessible.

D is especially most preferably selected from the group of compound: 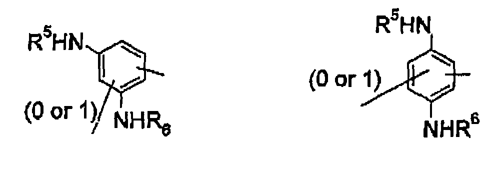 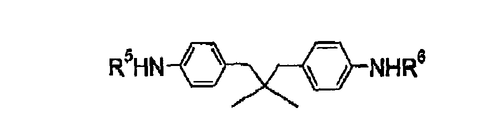 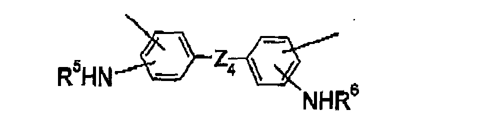 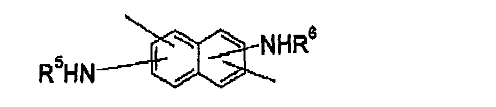 wherein
R⁵, R⁶ and Z⁴ have the same meanings as given above.
(E preferably represents an phenylene, an oxygen atom or a -N(H)- group, more preferred E is oxygen or a -N(H)- group, and most preferred E is oxygen.

Preferably, S¹, S² each Independently from each other represents a single bond or a spacer unit, which is a cyclic, straight-chain or branched, substituted or unsubstituted C₁-C₂₄alkylen, in which one or more, preferably non-adjacent, -CHz- group may be replaced by a linking group residue; or a non-aromatic, aromatic, unsubstituted or substituted carbocyclic or heterocyclic group of formula (IV):

-(Z¹-C¹)ₐ₁-(Z²-C²) ₐ₂- (IV)

wherein:
- C¹, C²: each independently represents a non-aromatic, aromatic, optionally substituted carbocyclic or heterocyclic group, connected to each other via the bridging groups Z¹ and Z², preferably C¹ and C² are connected at the opposite positions via the bridging groups Z¹ and Z², so that groups S¹ and/or S² have a long molecular axis, and
- Z¹, Z²: each independently represents a bridging group, preferably selected from -CH(OH)-, -CH₂-, -O-, -CO-, -CH₂(CO)-, -SO-, -CH₂(SO)-, -SO₂-, -CH₂(SO₂)-, -COO-, -OCO-, -COCF₂-, -CF₂CO-, -S-CO-, -CO-S-, -SOO-, -OSO-, -SOS-, -CH₂-CH₂-, -OCH₂-, -CH₂O-, -CH=CH-, -C≡C-, -CH=CH-COO-, -OCO-CH=CH-, -CH=N-, -C(CH₃)=N-, -O-CO-O-, -N=N- or a single bond; and
- a¹,a²: each independently represents an integer from 0 to 3, such that a¹ + a² ≤ 4, wherein preferably S² is linked to A via Z¹.

More preferred S¹ represents a single bond or a straight-chain or branched C₁-C₂₄alkylen, wherein one or more -CH₂- groups may independently be replaced by a linking group or/and a group represented by the formula (IV), wherein;
- C¹, C²: are selected from a compound of group G¹, wherein group G¹ is: 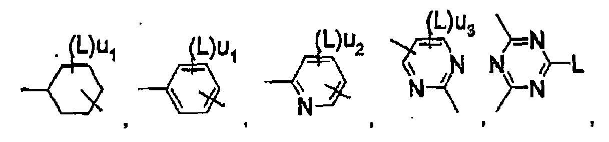 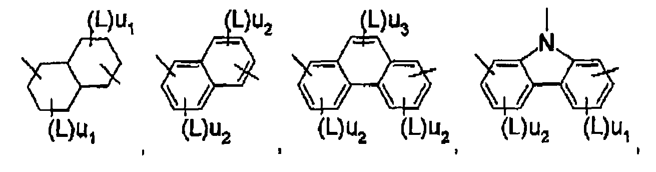 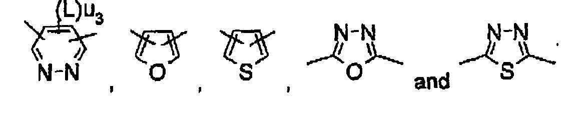 wherein:
- "___": denotes the connecting bonds of C¹ and C² to the adjacent groups; and
- L: is -CH₃, -OCH₃, -COCH₃, nitro, cyano, halogen, CH₂=CH-, CH₂=C(CH₃)-, CH₂=CH-(CO)O-, CH₂=CH-O-, CH₂=C(CH₃)-(CO)O-, or CH₂=C(CH₃)-O-,
- u₁: is an integer from 0 to 4; and
- u₂: is an integer from 0 to 3; and
- u₃: is an integer from 0 to 2; and
- Z¹, Z²: each independently represents -O-, -CO-, -COO-, -OCO-, -COCF₂-, -CF²CO-, -CH₂-CH₂-, -OCH₂-, -CH₂O-, -CH=CH-, -C≡C-, -CH=CH-COO-, -OCO-CH=CH- or a single bond; with the proviso that heteroatoms are not directly linked to each other, and
- a¹, a²: each independently represents an integer from 0 to 3, such that a¹ + a² ≤ 4.

Most preferred S¹ represents a single bond or a spacer unit such a straight-chain or branched C₁-C₁₄alkylen, wherein one or more, preferably non adjactent, -CH₂- groups may independently be replaced by a linking group and/or a group represented by formula (IV), wherein:
- C¹, C²: each independently represents a 1,4-phenylene, 2-methoxy-1,4-phenylene, 1,4-cyclohexylene or a 4,4'-biphenylene group; and
- Z¹, Z²: each independently represents -COO-, -OCO-, -CH₂-CH₂-, -OCH₂-, -CH₂O-, -CH=CH-, -C≡C-, -CH=CH-COO-, -OCO-CH=CH- or a single bond; and
- a¹, a²: are independently 0 or 1.

Especially most preferred S¹ represents a single bond or a spacer unit such a straight-chain C₁-C₁₂alkylen, wherein one -CH₂- groups may be replaced by -O-, -O(CO)-, -(CO)O-, -NR₁CO-, -CONR₁-, wherein R₁ is hydrogen or C₁-C₆alkyl or a group of formula (IV), wherein:
- C¹, C²: each independently represents 1,4-phenylene ; and
- Z¹, Z²: each independently represents -COO-, -OCO-, -CH₂-CH₂-, -OCH₂-, -CH₂O-, -CH=CH-, -C≡C-, -CH=CH-COO-, -OCO-CH=CH-, or a single bond; and
- a¹, a²: each independently represents 0 or 1.

More preferred S² represents a spacer unit such as a straight-chain or branched C₁-C₂₄alkylen, wherein one or more -CH₂- groups is independently replaced by a group represented by the formula (IV), wherein:
- C¹, C²: are selected from group G¹, with the avove given meaning; and
- Z¹, Z²: each independently represents -O-, -CO-, -COO-, -OCO-, -COCF₂-, -CF₂CO-, -OH₂-CH₂-, -OCH₂-, -CH₂O-, -CH=CH-, -C≡C-, -CH=CH-COO-, -OCO-CH=CH- or a single bond; with the proviso that heteroatoms are not directly linked to each other, and
- a¹, a²: each independently represents an integer from 0 to 3, such that a¹ + a² ≤ 4, wherein preferably S² is linked to A via Z¹.

Most preferred S² represents a spacer unit such as a straight-chain or branched C₁-C₁₂alkylen, wherein one -CH₂- groups is independently be replaced by a group represented by the formula (IV), wherein
- C¹, C²: each independently represents a 1,4-phenylene which is unsubstituted or mono or poly-substituted by a halogen atom, and/or by an alkoxy, alkylcarbonyloxy or an alkyloxycarbonyl group, having form 1 to 10 carbon atoms, 1,4-cyclohexylene or a 4,4'-biphenylene group; and
- Z¹, Z²: each independently represents -COO-, -OCO-, -CH₂-CH₂-, -OCH₂-, -CH₂O-, -CH=CH-, -C≡C-, -CH=CH-COO-, -OCO-CH=CH- or a single bond; and
- a¹, a²: are independently 0 or 1, wherein preferably S² is linked to A via Z¹.

Especially most preferred S² represents a group of formula (IV), wherein:
- C¹: represents a 1,4-phenylene which is unsubstituted or mono or poly-substituted by a halogen atom, and/or by an alkoxy, alkylcarbonyloxy or an alkyloxycarbonyl group, having form 1 to 10 carbon atoms,
- Z¹: -COO-, -OCO-, -CH₂-CH₂-,-OCH₂-, -CH₂O-, -CH=CH-, -C≡C-, -CH=CH-COO-, -OCO-CH=CH-, or a single bond;
- a 1: represents 1, wherein preferably S² is linked to A via Z¹.

Another preferred embodiment of the present invention relates to diamine compounds (I), referring to any of the preceding definitions comprising these diamine compounds wherein
- A: represents phenanthrylene, biphenylene, naphthylene, or phenylene, which is unsubstituted or mono- or poly-substituted by a halogen atom, hydroxy group and/or by a polar group, preferably nitro, cyano, carboxy; and/or by acryloyloxy, methacryloyloxy, vinyl, vinyloxy, allyl, allyloxy, and/or by a cyclic, straight-chain or branched C₁-C₁₂alkyl residue, which is unsubstituted, mono- or poly-substituted by fluorine and/or chlorine, wherein one or more, preferably non-adjacent, -CH₂- groups may Independently be replaced by a linking group, preferably selected from -O-, -CO-, -CO-O-, -O-CO-, -NH-CO-O-, -OCO-NH-, -NHCO-, -CONH-;
- F: is fluorine, and
- x₁: is an integer from 1 to 10; more preferably x is 1, 2, 3, 4, 5, 6, 7, 8 or 9 and most preferred x is 3, 4, 5 or 7;
- B: represents a straight-chain or branched C₁-C₁₂alkylen, which is unsubstituted or substituted by di-(C₁-C₁₆alkyl)amino, C₁-C₆alkyloxy, nitro, cyano and/or chlorine or fluorine,
wherein one or more, preferably non-adjacent, -CH₂- group may independently be replaced by a linking group selected from -O-, -CO-, -CO-O-, -O-CO-, -NR¹-, -NR¹-CO-, -CO-NR¹- and -CH=CH-, wherein:
R¹ represents a hydrogen atom or C₁-C₆alkyl;
with the proviso that oxygen atoms are not directly linked to each other;
- D: represents an optionally substituted aliphatic, aromatic or alicyclic Diamine group having from 1 to 40 carbon atoms, preferably selected from formula (III) as defined above, wherein:
- k¹, k²: are 0 or 1, and
- t¹, t²: are 0, and
- R⁵, R⁶: are identical and represent a hydrogen atom, a methyl, an ethyl or an isopropyl group; and
- C³, C⁴: independently from each other are selected from compound of a group G² as defined above:
- Z³: represents a group selected from -CH(OH)-, -CH(CH₃)-, -C(CH₃)₂-, -CO-,-COO-, -COCF₂-, -CF₂CO- or a single bond; and
- Z⁴: has one of the meanings of Z³ or represents a substituted or unsubstituted straight-chain or branched C₁-C₂₀alkylene group, in which one or more, preferably non-adjacent, -CH₂- groups may be replaced by a heteroatom and/or by an oxygen atom; and/or one or more carbon-carbon single bonds are replaced by a carbon-carbon double or a carbon-carbon triple bond;
- a³, a⁴: each independently represents an integer from 0 to 2 such that a³ +-a⁴ s 3;
- E: represents an phenylene, an oxygen atom or a -N(H)- group;
- S¹: represents a single bond or a spacer unit such as a straight-chain or branched C₁-C₂₄alkylen, wherein one or more -CH₂- groups may independently be replaced by a linking group and/or a group represented by the formula (IV) as defined above, wherein:
- C¹, C²: are selected from a compound of group G¹ as defined above, and
- Z¹, Z²: each independently represents -O-, -CO-, -COO-, -OCO-, -COCF₂-, -CF₂CO-, -CH₂-CH₂-, -OCH₂-, -CH₂O-, -CH=CH-, -C≡C-, -CH=CH-COO-, -OCO-CH=CH- or a single bond; with the proviso that heteroatoms are not directly linked to each other; and
- a¹, a²: each independently represents an integer from 0 to 3, such that a¹ + a² ≤ 4;
- S²: represents a spacer unit such as a straight-chain or branched C₁-C₂₄alkylen, wherein one or more -CH₂- groups is independently replaced by a group represented by the formula (IV) as defined above, wherein preferably S² is linked to A via Z¹; and wherein:
C¹, C² are selected from a compound of group G¹ as defined above, and
- Z¹, Z²: each independently represents -O-, -CO-, -COO-, -OCO-, -COCF₂-, -CF₂CO-, -CH₂-CH₂-, -OCH₂-, -CH₂O-, -CH=CH-, -C≡C-, -CH=CH-COO-,
-OCO-CH=CH- or a single bond; with the proviso that heteroatoms are not directly linked to each other, and
- a¹, a²: each independently represents an integer from 0 to 3, such that a¹ + a² ≤ 4;
- X, Y: are hydrogen atoms, and
- n: is 1,2 or 3,
with the proviso that if n is 2 or 3 each A, B, X₁, D, E, S¹, S², X, Y may be identical or different.

A more preferred embodiment of the present invention relates to Diamine compounds (I), referring to any of the preceding definitions, and to alignment materials comprising these Diamine compounds wherein
- A: represents a biphenylene, naphthylene or phenylene group, which is unsubstituted or mono- or poly-substituted by a halogen atom, a hydroxy group, and/or by acryloyloxy, and/or methacryloyloxy groups, and/or by straight-chain or branched alkyl, alkoxy, alkylcarbonyloxy, and/or alkyloxycarbonyl groups having from 1 to 20 carbon atoms.
- F: is fluorine, and
- x₁: is 1, 2, 3, 4, 5, 6, 7, 8 or 8 and most preferred x₁ is 3, 4, 5 or 7;
- B: represents a straight-chain or branched C₁-C₁₀alkyl residue, wherein one -CH₂- group may independently be replaced by a group selected from -O-, -CO-, -CO-O-, -O-CO-, -NR¹-, -NR¹-CO-, -CO-NR¹- and -CH=CH-, wherein:
- R¹: represents a hydrogen atom or C₁-C₆alkyl; with the proviso that oxygen atoms are not directly linked to each other; and
- D: represents an optionally substituted aliphatic, aromatic or alicyclic diamine group having from 1 to 40 carbon atoms, represented by formula (III) and is most preferably selected from the following group of structures: substituted or unsubstituted o- phenylenediamine, p-phenylenediamine, m-phenylenediamine, aminophenyl-Z⁴-phenylamino, wherein Z⁴ has the above given meaning, benzidine, diaminofluorene, 3,4-diaminobenzoic acid, 3,4-diaminobenzyl alcohol dihydrochloride, 2,4-diaminobenzoic acid, L-(+)-threo-2-amino-1-(4-aminophenyl)-1,3-propanediol, p-aminobenzoic acid, [3,5-3h]-4-amino-2-methoxybenzoic acid, L-(+)-threo-2-(N,N-dimethylamino)-1-(4-aminophenyl)-1,3-propanediol, 2,7-diaminofluorene, 4,4'-diaminooctafluorobiphenyl, 3,3'-diaminobenzidine, 2,7-diamine-9-fluorenone, 3,5,3',5'-tetrabromo-biphenyl-4,4'-diamine, 2,2'-dichloro[1,1'-biphenyl]-4,4'-diamine, 3,9-diamine-1,11-dimethyl-5,7-dihydro-dibenzo(a, c)cyclohepten-6-one, dibenzo(1,2)dithiine-3,8-diamine, 3,3'-diaminobenzophenone, 3,3'-diaminediphenyl methane, 4,4-bis-(3-amino-4-hydroxyphenyl)-valeric acid, 2,2-bis(3-amino-4-hydroxyphenyl)hexafluoropropane, 2,2-bis(3-amino-4-methylphenyl)hexafluoropropane, tetrabromo methylenedianiline, 2,7-diamine-9-fluorenone, 2,2-bis(3-aminophenyl)hexafluoropropane, bis-(3-amino-4-chloro-phenyl)-methanone, bis-(3-amino-4-dimethylamino-phenyl)-methanone, 3-[3-amino-5-(trifluoromethyl)benzyl]-5-(trifluoromethyl)aniline, 1,5-diaminonaphthalene, benzidine-3,3'-dicarboxylic acid, 4,4'-diamino-1,1'-binaphthyl, 4,4'-diaminediphenyl-3,3'-diglycolic acid, dihydroethidium, o-dianisldine, 2,2'-dichloro-5,5'-dimethoxybenzidine, 3-methoxybenzidine, 3,3'-dichlorobenzidine (diphenyl-d6), 2,2'-bis(trifluoromethyl)benzidine, 3,3'-bis(trifluoromethyl)benzidine, 3,3'-dichlorobenzidine-d6, tetramethylbenzidine, di-(aminophenyl)alkylen
and from amino compounds listed below, which do not carry two amino groups and are taken as derivatives with at least one additional amino group: aniline, 4-amino-2,3,5,6-tetrafluorobenzoic acid, 4-amino-3,5-diiodobenzoic acid, 4-amino-3-methylbenzoic acid, 4-amino-2-chlorobenzoic acid, 4-aminosalicylic acid, 4-aminobenzoic acid, 4-aminophthalic acid, 1-(4-aminophenyl)ethanol, 4-aminobenzyl alcohol, 4-amino-3-methoxybenzoic acid, 4-aminophenyl ethyl carbinol, 4-amino-3-nitrobenzoic acid, 4-amino-3,5-dinitrobenzoic acid, 4-amino-3,5-dichlorobenzoic acid, 4-amino-3-hydroxybenzoic acid, 4-aminobenzyl alcohol hydrochloride, 4-aminobenzoic acid hydrochloride, pararosaniline base, 4-amino-5-chloro-2-methoxybenzoic acid, 4-(hexafluoro-2-hydroxyisopropyl)aniline, piperazine-p-amino benzoate, 4-amino-3,5-dibromobenzoic acid, isonicotinic acid hydrazide p-amino-salicylate salt, 4-amino-3,5-dilodosalicylic acid, 4-amino-2-methoxybenzoic acid, 2-[2-(4-aminophenyl)-2-hydroxy-1-(hydroxymethyl)ethyl]isoindoline-1,3-dione, 4-amino-2-nitrobenzoic acid, ethyl 2-(4-aminophenyl)-3,3,3-trifluoro-2-hydroxypropanoate, ethyl 2-(4-amino-3-methylphenyl)-3,3,3-trifluoro-2-hydroxypropanoate, ethyl 2-(4-amino-3-methoxyphenyl)-3,3,3-trifluoro-2-hydroxypropanoate, 4-aminonaphthalene-1,8-dicarboxylic acid, 4-amino-3-chloro-5-methylbenzoic acid, 4-amino-2,6-dimethylbenzoic acid, 4-amino-3-fluorobenzoic acid, 4-amino-5-bromo-2-methoxybenzenecarboxylic acid, 3,3'-tolidine-5-sulfonic acid,
or their derivatives, again with the proviso that compounds listed which do not carry two amino groups are taken as derivatives with at least one additional amino group, and
- E: represents an oxygen atom or a -N(H)- group;
- S¹: represents a single bond or a spacer unit such a straight-chain or branched C₁-C₁₄alkylone groups, wherein one or more -CH₂- groups may independently be replaced by a group represented by formula (IV) as defined above,
wherein:
- C¹, C²: each independently represents a 1,4-phenylene, 2-methoxy-1,4-phenylene, 1,4-cyclohexylene or a 4,4'-biphenylene group; and
- Z¹, Z²: each independently represents -COO-, -OCO-, -CH₂-CH₂-, -OCH₂-, -CH₂O-, -CH=CH-, -C≡C-, -CH=CH-COO-, -OCO-CH=CH- or a single bond; and
- a¹, a²: are independently 0 or 1;
- S²: represents a spacer unit such as a straight-chain or branched C₁-C₈alkylen, wherein one -CH₂- groups is independently be replaced by a group represented by the formula (IV) as defined above, wherein preferably S² is linked to A via Z¹; and wherein
C¹, C² each independently represents a 1,4-phenylene which is unsubstituted or mono or poly-substituted by a halogen atom, and/or by an alkoxy, alkylcarbonyloxy or an alkyloxycarbonyl group, having form 1 to 10 carbon atoms, 1,4-cyclohexylene or a 4,4'-biphenylene group; and
- Z¹, Z²: each independently represents -COO-, -OCO-, -CH₂-CH₂-, -OCH₂-, -CH₂O-, -CH=CH-, -C≡C-, -CH=CH-COO-, -OCO-CH=CH- or a single bond; and
- a¹, a²: are independently 0 or 1;
- n: is 1 or 2,
with the proviso that if n is 2 or 3 each A, B, x₁, D, E, S¹, S², X, Y may be identical or different.

Another preferred embodiment of the present invention relates to diamine compounds represented by one of the general formula (I), referring to any of the preceding definitions, and to alignment materials comprising these diamine compounds wherein
- A: represents 1,4-phenylene, which is unsubstituted or mono- or poly-substituted by a halogen atom, and/or by acryloyloxy or methacryloyloxy, and/or by an alkoxy, alkylcarbonyloxy or an alkyloxycarbonyl group, having from 1 to 10 carbon atoms,
- F: is fluorine, and
- x₁: is 1, 2, 3, 4, 5, 6, 7, 8 or 9;
- B: represents a straight-chain or branched C₁-C₈alkyl residue,
wherein one -CH₂- group may independently be replaced by a group selected from -O-, -CO-, -CO-O-, -O-CO-, - and -CH=CH-, with the proviso that oxygen atoms are not directly linked to each other,
- D: represents an unsubstituted o-phenylenediamine, p-phenylenediamine, m-phenylenediamine, biphenyldiamine, aminophenyl-Z⁴-phenylamino, naphthylenediamine,
wherein
- Z⁴: is as defined in claim 5; and
is preferably selected from the following group of structures: 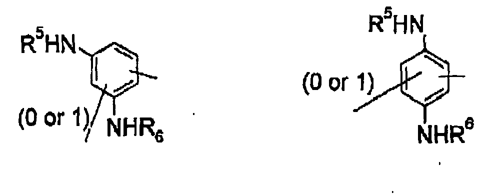 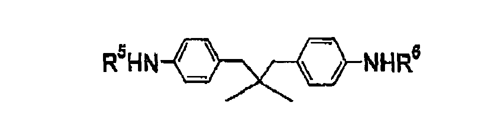 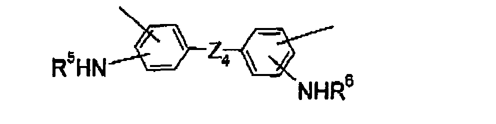 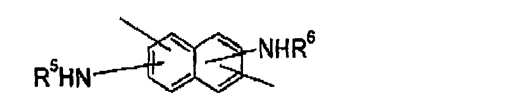 wherein
- R⁵, R⁶ and Z⁴: are as defined in claim 5;
- E: represents an oxygen atom;
- S¹: represents a single bond or a straight-chain C₁-C₈alkylene group, wherein one -CH₂- groups may be replaced by a group of formula (IV) as defined above, wherein:
- C¹, C²: each independently represents 1,4-phenylene; and
- Z¹, Z²: each independently represents -COO-, -OCO-, -CH₂-CH₂-, -OCH₂-, -CH₂O-, -CH=CH-, -C≡C-, -CH=CH-COO-, -OCO-CH=CH-, or a single bond; and
- a¹, a²: each independently represents 0 or 1, and
- S²: is replaced by a group of formula (IV) as defined above, wherein:
- C¹: represents a 1,4-phenylene which is unsubstituted or mono or poly-substituted by a halogen atom, and/or by an alkoxy, alkylcarbonyloxy or an alkyloxycarbonyl group, having form 1 to 10 carbon atoms,
- Z¹: represents -COO-, -OCO-, -CH₂-CH₂-,-OCH₂-, -CH₂O-, -CH=CH-, -C=C-, -CH=CH-COO-, -OCO-CH=CH-, or a single bond;
- a¹: represents 1, and
- S²: is linked to A via Z¹
- n: is 1 or 2,
with the proviso that if n is 2 each A, B, x₁, D, E, S¹ and S² may be identical or different.

Most preferred embodiment of the present invention relates to diamine compounds represented by one of the general formula (I), referring to any of the preceding definitions, and to alignment materials comprising these diamine compounds wherein
- S²: Is replaced by a group of formula (IV, wherein:
- C¹: represents 1,4-phenylene; and
- Z¹: represents -COO-, -OCO-, -CH₂-CH₂-,-OCH₂-, -CH₂O-, -CH=CH-, -C≡C-, -CH=CH-COO-, -OCO-CH=CH-, or a single bond;
- a¹: represents 1, and
- S²: is linked to A via Z¹,

Especially most preferred embodiment of the present invention relates to Diamine compounds of formulae (VI), (VII), (VIII), (IX), (X) and (XI) 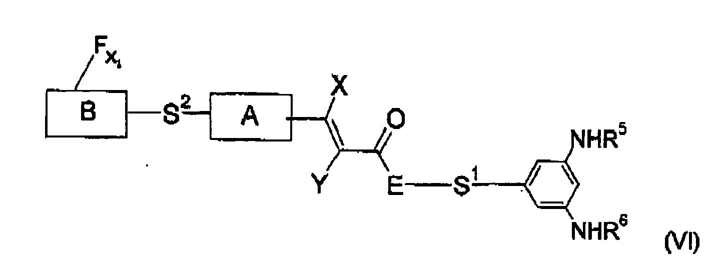 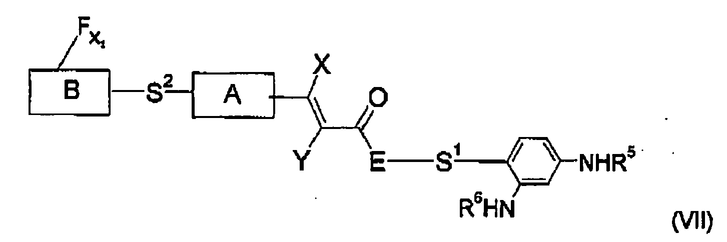 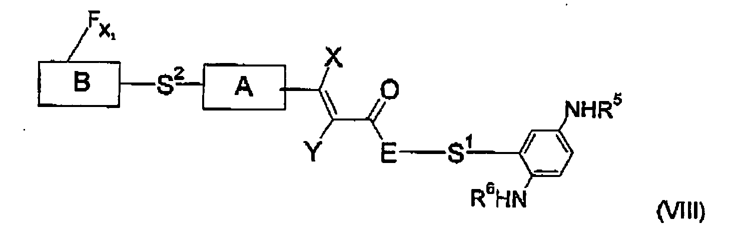 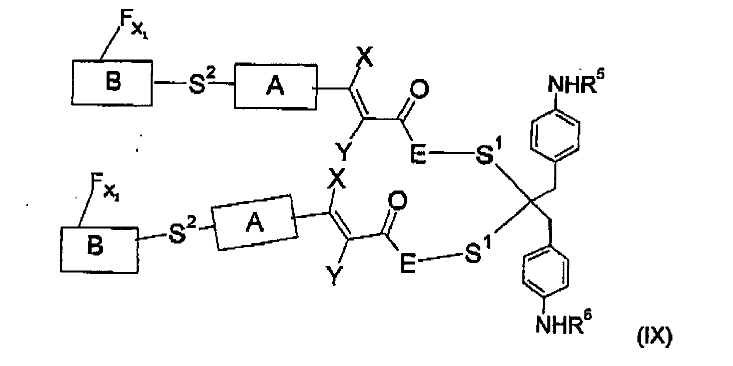 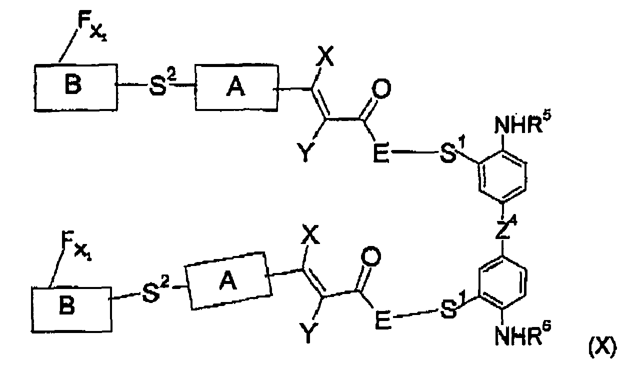 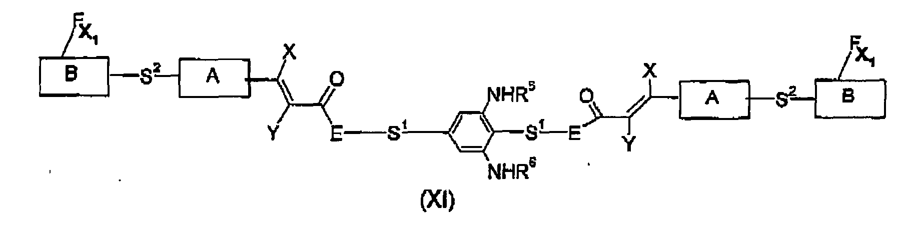 wherein
A, B, x₁, n, D, E, S²,S¹, X, Y and Z⁴ have the above given meanings and preferences.

Further, especially most preferred embodiment of the present invention relates to diamine compounds of formula (XII) 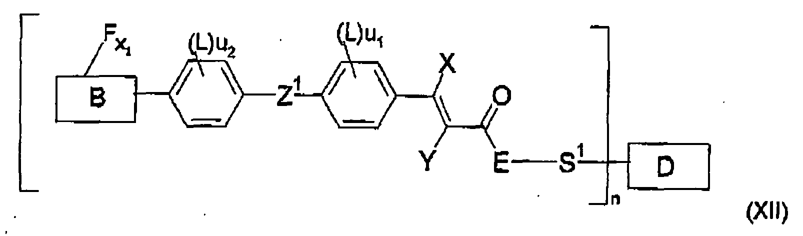 wherein x₁, n, D, E, S¹, X, Y, Z¹, L, u₁ and u₂ have the above given meanings and preferences.

Preferred diamine compounds of formula (XII) are compounds of formula (XIII) 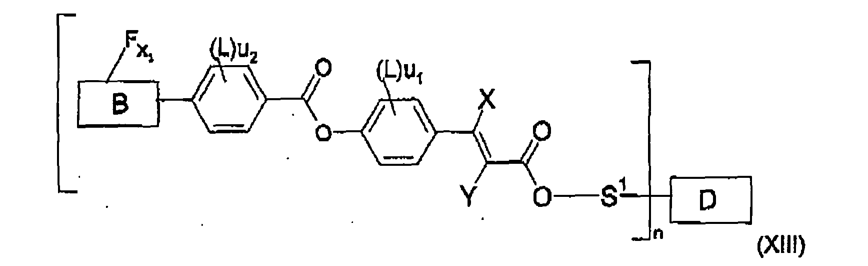 wherein x₁, n, D, S¹, X, Y, Z¹, L, u₁ and u₂ have the above given meanings and preferences.

Another preferred embodiment of the present invention relates to diamine compounds represented by the general formula (I), which may be used in the subsequent manufacturing processes as such or in combination with one or more additional other diamines.

A further embodiment of the present invention is a composition comprising diamine of compound (I) and optionally a further diamine, which is different from diamine of compound (I).

Preferred examples of additional other Diamines are:
ethylenediamine, 1,3-propylenediamine, 1,4-butylenediamine, 1,5-pentylenediamine, 1,6-hexylenediamine, 1,7-heptylenediamine, 1,8-octylenediamine, 1,9-nonylenediamine, 1,10-decylenediamine, 1,11-undecylenediamine, 1,12-dodecylenediamine, α,α'-diamino-*m*-xylene, α,α'-diamino-*p*-xylene, (5-amino-2,2,4-trimethylcyclopentyl)methylamine, 1,2-diaminocyclohexane, 4,4'-diaminodicyclohexylmethane, 1,3-bis(methylamino)cyclohexane, 4,9-dioxadodecane-1, 12-diamine, 3,5-diaminobenzoic acid methyl ester, 3,5-diaminobehzoic acid hexyl ester, 3,5-diaminobenzoic acid dodecyl ester, 3,5-diaminobenzoic, acid isopropyl ester, 4,4'-methylenedianiline, 4,4'-ethylenedianiline, 4,4'-diamino-3.3'-dimethyldiphenylmethane, 3,3',5,5'-tetramethylbenzidine, 4,4'-diaminodiphenyl sulfone, 4,4'-diaminodiphenyl ether, 1,5-diaminonaphthalene,3,3'-dimethyl-4,4'-diaminobiphenyl, 3,4'-diaminodiphenyl ether, 3,3'-diaminobenzophenone, 4,4'-diaminobenzophenone, 4,4'-diamino-2,2'-dimethylbibenzyl, bis[4-(4-aminophenoxy)phenyl] sulfone, 1,4-bis(4-aminophenoxy)benzene, 1,3-bis(4-aminophenoxy)benzene, 1,3-bis(3-aminophenoxy)benzene, 2,7-diaminofluorene, 9,9-bis(4-aminophenyl)fluorene, 4,4'-methylenebis(2-chloroaniline), 4,4'-bis(4-aminophenoxy)biphenyl, 2,2',5,5'-tetrachloro-4,4'-diaminobiphenyl, 2,2'-dichloro-4,4'-diamino-5,5'-dimethoxybiphenyl, 3,3'-dimethoxy-4,4'-diaminobiphenyl, 4,4'-(1,4-phenyleneisopropylidene)bisaniline, 4,4'-(1,3-phemylanalsopropylidene)bisaniline, 2,2-bis[4-(4-aminophenoxy)phenyl]propane, 2,2-bis[3-(4-aminophenoxy)phenyl]hexafluoropropane, 2,2-bis[3-amino-4-methylphenyl]hexafluoropropane, 2,2-bis(4-aminophenyl)hexafluoropropane, 2,2'-bis[4-(4-amino-2-trifluoromethylphenoxy)phenyl]hexafluoropropane, 4,4'-diamino-2,2'-bis(trifluoromethyl)biphenyl, and 4,4'-bis[(4-amino-2-trifluoromethyl)phenoxy]-2,3,5,6,2',3',5',6'-actefluorobiphenyl;
as well as diamines disclosed in US 6,340,506, WO 00/59966 and WO 01/53384, all of which are explicitely incorporated herein by reference.

A further preferred embodiment of the present invention relates to a polymer material or oligomer material from the class of polyamic acids, polyamic acid esters or polyimides, (and any mixtures thereof) obtained by or obtainable by the reaction of at least one diamine compound represented by the general formula (I) and optionally of one or more additional other diamines (as e.g. given above), with one or more tetracarboxylic acid anhydrides of the general formula (V) 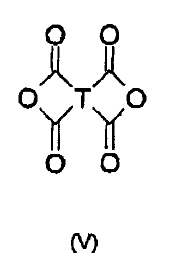 wherein,
T represents a tetravalent organic radical.

The tetravalent organic radical T is preferably derived from an aliphatic, alicyclic or aromatic tetracarboxylic acid dianhydride.

Preferred examples of aliphatic or alicyclic tetracarboxylic acid dianhydrides are:
1,1,4,4-butanetetracarboxylic acid dianhydride,
ethylenemaleic acid dianhydride,
1,2,3,4-cyclobutanetetracarboxylic acid dianhydride,
1,2,3,4-cyclopentanetetracarboxylic acid dianhydride,
2,3,5-tricarboxycyclopentylacetic acid dianhydride,
3,5,6-tricarboxynorbomylacetic acid dianhydride,
2,3,4,5-tetrahydrofurantetracarboxylic acid dianhydride,
rel-[1S,5R,6R]-3-oxabicyclo[3,2,1]octane-2,4-dione-6-spiro-3'-(tetrahydrofuran2',5'-dione).
4-(2,5-dioxotetrahydrofuran-3-yl)tetrahydronaphthalene-1,2-dicarboxylicacid dianhydride,
5-(2,5-dioxotetrahydrofuran-3-yl)-3-methyl-3-cyclohexene-1,2-dicarboxylicacid dianhydride,
bicyclo[2.2.2]oct-7-ene-2,3,5,6-tetracarboxylic acid dianhydride,
bicyclo[2.2.2]octane-2,3,5,6-tetracarboxylic acid dianhydride,
1,8-dimethylbicyclo[2.2.2]oct-7-ene-2,3,5,6-tetracarboxylic acid dianhydride, pyromellitic acid dianhydride,
3,3',4,4'-benzophenonetetracarboxylic acid dianhydride,
4,4'-oxydiphthalic acid dianhydride,
3,3',4,4'-diphenylsulfonetetracarboxylic acid dianhydride,
1,4,5,8-naphthalenetetracarboxylic acid dianhydride,
2,3,6,7-naphthalenetetracarboxylic acid dianhydride,
3,3',4,4'-dimethyldiphenylsilanetetracarboxylic acid dianhydride,
3,3',4,4'-tetraphenylsilanetetracarboxylic acid dianhydride,
1,2,3,4-furantetracarboxylic acid dianhydride,
4,4'-bis(3,4-dicarboxyphenoxy)diphenyl sulfide dianhydride,
4,4'-bis(3,4-dicarboxyphenoxy)diphenyl sulfone dianhydride,
4,4'-bis(3,4-dicarboxyphenoxy)diphenylpropane dianhydride,
3,3',4,4'-biphenyltetracarboxylic acid dianhydride, ethylene glycol bis(trimellitic acid) dianhydride,
4,4'-(1,4-phenylene)bis(phthalic acid) dianhydride,
4,4'-(1,3-phenylene)bis(phthalic acid) dianhydride,
4,4'-(hexafluoroisopropylidene)diphthalic acid dianhydride,
4,4'-oxydi(1,4-phenylene)bis(phthalic acid) dianhydride, and
4,4'-methylenedi(1,4-phenylene)bis(phthalic acid) dianhydride.

Preferred examples of aromatic tetracarboxylic acid dianhydrides are:
pyromellitic acid dianhydride,
3,3',4,4'-benzophenonetetracarboxylic acid dianhydride,
4,4'-oxydiphthalic acid dianhydride,
3,3',4,4'-diphenylsulfonetetracarboxylic acid dianhydride,
1,4,5,8-naphtholenetetracarboxylic acid dianhydride,
2,3,6,7-naphthalenotetracarboxylic acid dianhydride,
3,3',4,4'-dimethyldiphenylsilanetetracerboxylic acid dianhydride,
3,3',4,4'-tetraphenylsilanetetracarboxylic acid dianhydride,
1,2,3,4-furantetracarboxylic acid dianhydride,
4,4'-bis(3,4-dicarboxyphenoxy)diphenyl sulfide dianhydride,
4,4'-bis(3,4-dicarboxyphenoxy)diphenyl sulfone dianhydride,
4,4'-bis(3,4-dicarboxyphenoxy)diphenylpropane dianhydride,
3,3',4,4'-biphenyltetracarboxylic add dianhydride,
ethylene glycol bis(trimellitic acid) dianhydride,
4,4'-(1,4-phenylene)bis(phthalic acid) dianhydride,
4,4'-(1,3-phenylene)bis(phthalic acid) dianhydride,
4,4'-(hexafluoroisopropylidene)diphthalic acid dianhydride,
4,4'-oxydi(1,4-phenylene)bis(phthalic acid) dianhydride,
4,4'-methylenedi(1,4-phenylene)bis(phthalic acid) dianhydride,
and the like.

More preferably the tetracarboxylic acid dianhydrides used to form the tetravalent organic radical T are selected from:
1,2,3,4-cyclobutanetetracarboxylic acid dianhydride,
1,2,3,4-cyclopentanetetracarboxylic acid dianhydride,
2,3,5-tricarboxycyclopentylacetic acid dianhydride,
5-(2,5-dioxotetrahydrofuran-3-yl)-3-methyl-3-cyclohexene-1,2-dicarboxylic acid dianhydride,
4-(2,5-dioxotetrahydrofuran-3-yl)tetrahydronaphthalene-1,2-dicarboxylic acid dianhydride,
4,4¹-(hexafluoroisopropylidene)diphthalic acid dianhydride and
bicyclo[2.2.2]oct-7-ene-2,3,5,6-tetracarboxylic acid dianhydride.

Preferred polyamic acids, polyamic acid esters or polyimides (and any mixtures thereof) of the present invention relate to those which comprise as side-chains a photo-reactive group that can be photo-isomerized and/or photo-dimerized on exposure to visible light, UV light or laser light. It is preferred that at least 30 % of the repeating units include a side chain with a photo-reactive group.

Preferably, the photo-reactive groups are able to undergo photo-cyclization, in particular [2+2]-photo-cyclisation.

Preferably, the photo-reactive groups are sensitive to visible and/or UV light, in particular to linearly polarized UV light.

The side-chain polymers or oligomers according the invention can be present in the form of homopolymers as well as in the form of copolymers. The term "copolymers" is to be understood as meaning especially statistical copolymers.

The diamine compounds according to the present invention may be prepared using methods that are known to a person skilled in the art.

Further the present invention relates to a process for the preparation of diamine compounds (XII) and (XIII) as defined above comprising contacting a acrylic acid compound of formula (XIV) 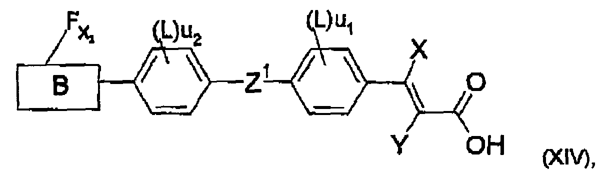 preferably 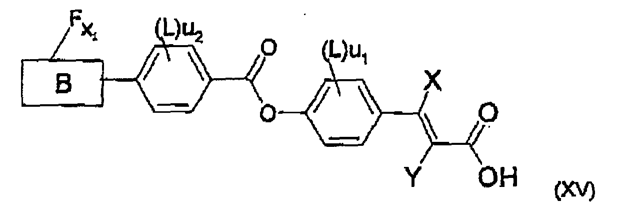 With a compound of formula (XVI) 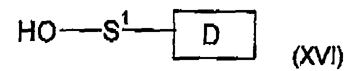 and optionally an additional other diamine,
wherein
F, x₁, B, S², X, Y, S¹ and D have the same meanings as in claim 1, and Z¹, L, u₁ and u₂ have the same meanings and preferences as given above.

The reaction of compounds (XIV) and (XV) can be conducted in many known ways (see J. March, Advanced Organic Chemistry, second edition, pages 363 and 365.

Usually, compounds (XIV) and (XV) are contacted with a dehydrating agent.

Commenly known dehydrating agents can be used. Preferred are EDC, 1-(3-Dimethylaminopropyl)-3-ethylcarbodiimide hydrochloride or DCC, dicyclohexylcarbodiimide, trifluoroacetic anhydride, H₃BO₃-H₂SO₄, polymer protected AlCl₃, pyridinium salts-Bu₃N or N,N-carbonyldiimidazole.

In general the reaction of compounds (XIV) and (XV) are made in a solvent. Usually organic solvents, such as for example toluene, xylene, pyridine, halogenalkane, such as dichlormethan, trichlorethan, acetone or dimethylformamide.

Further, the present invention relates to acrylic acid of formulae (XIV) and (XV) as above.

The polyamic acids, polyamic acid esters and polyimides according to the present invention may be prepared in line with known methods, such as those described in Plast. Eng. 36 (1996), (Polyimides, fundamentals and applications), Marcel Dekker, Inc.

For example, the poly-condensation reaction for the preparation of the polyamic acids is carried out in solution in a polar aprotic organic solvent, such as γ-butyrolactone, N,N-dimethylacetamide, N-methylpyrrolidone or N,N-methylformamide. In most cases equimolar amounts of the dianhydride and the Diamine are used, i.e, one amino group per anhydride group. If it is desired to stabilize the molecular weight of the polymer or oligomer, it is possible for that purpose to either add an excess or a less-than-stolchiometric amount of one of the two components or to add a mono-functional compound in the form of a dicarboxylic acid monoanhydride or in the form of a mono-amine. Examples of such mono-functional compounds are maleic acid anhydride, phthalic acid anhydride, aniline and the like. Preferably the reaction is carried out at temperatures of less than 100 °C.

The cyclisation of the polyamic acids to form the polyimides can be carried out by heating, i.e. by condensation with removal of water or by other imidisation reactions using appropriate reagents. When carried out purely thermally, the imidisation of the polyamic acids may not always be complete, i.e. the resulting polyimides may still contain proportions of polyamic acid. In general the imidisation reactions are carried out at temperatures between 60 and 250 °C, preferably at temperatures of less than 200 °C. In order to achieve imidisation at lower temperatures additional reagents that facilitate the removal of water are added to the reaction mixture. Such reagents are, for example, mixtures consisting of acid anhydrides, such as acetic acid anhydride, propionic acid anhydride, phthalic acid anhydride, trifluoroacetic acid anhydride or tertiary amines, such as triethylamine, trimethylamine, tributylamine, pyridine, N,N-dimethylaniline, lutidine, collidine etc. The amount of aforementioned additional reagents that facilitate the removal of water is preferably at least four equivalents of acid anhydride and two equivalents of amine per equivalent of polyamic acid to be condensed.

The imidisation reaction can be carried out prior or after the application to a support.

The polyamic acids and the polyimides of the present invention have an intrinsic viscosity preferably in the range of 0.05 to 10 dUg, more preferably in the range of 0.05 to 5 dL/g. Herein, the intrinsic viscosity (ηᵢₙₕ= In ηrel/C) is determined by measuring a solution containing a polymer or an oligomer in a concentration of 0.5 g/100 ml solution for the evaluation of its viscosity at 30 °C using N-methyl-2-pyrrolidone as solvent.

The polyamic acid chains or polyimide chains of the present invention preferably contain from 2 to 2000 repeating units, especially from 3 to 200 repeating units.

Additives such as silane-containing compounds and epoxy-containing crosslinking agents may be added to the polymers or the oligomers of the Invention In order to improve the adhesion of the polymer or the oligomer to the substrates.

Suitable silane-containing compounds are described in Plast. Eng. 36 (1996), (Polyimides, fundamentals and applications), Marcel Dekker, Inc.

Suitable epoxy-containing cross-linking agents include 4,4¹-methylene-bis-(N,N-diglycidylaniline), trimethylolpropane triglycidyl ether, benzene-1,2,4,5-tetracarboxylic acid 1,2,4,5-N,N'-diglycidyldiimide, polyethylene glycol diglycidyl ether, N,N-diglycidylcyclohexylamine and the like.

Additional additives such one or more photo-sensitizers and/or one or more photo-radical generators and/or one or more cationic photo-initiators may also be added to the polymers or oligomers of the invention.

Suitable photo-active additives include 2,2-dimethoxyphonylethanome, a mixture of diphenylmethanone and N,N-dimethylbenzenamine or ethyl 4-(dimethylamino)-benzoate, xanthone, thioxanthone, Irgacure^{®} 184, 369, 500, 651 and 907 (Ciba), Michler's ketone, triaryl sulfonium salt and the like.

The polymers or oligomers according to the invention may be used in form of polymer layers or oligomer layers alone or in combination with other polymers, oligomers, monomers, photo-active polymers, photo-active oligomers and/or photo-active monomers, depending upon the application to which the polymer or oligomer layer is to be added. Therefore it is understood that by varying the composition of the polymer or oligomer layer it is possible to control specific and desired properties, such as an induced pre-tilt angle, good surface wetting, a high voltage holding ratio, a specific anchoring energy, etc.

Polymer or oligomer layers may readily be prepared from the polymers or oligomers of the present invention and a further embodiment of the invention relates to a polymer or oligomer layer comprising a polymer or oligomer according to the present invention in a cross-linked form.

The polymer or oligomer layer is preferably prepared by applying one or more polymers or oligomers according to the invention to a support and, after any imidisation step which may be necessary, cross-linking the polymer or oligomer or polymer mixture or or oligomer mixture by irradiation with linearly polarized light. It is possible to vary the direction of orientation and the tilt angle within the polymer or oligomer layer by controlling the direction of the irradiation of the linearly polarized light. It is understood that by selectively irradiating specific regions of the polymer or oligomer layer it is possible to align very specific regions of the layer and to provide layers with a defined tilt angle. The induced orientation and tilt angle are retained in the polymer or oligomer layer by the process of cross-linking.

It is understood that the polymer or oligomer layers of the present invention (in form of a polymer gel, a polymer network, a polymer film, etc.) can also be used as orientation layers for liquid crystals and a further preferred embodiment of the invention relates to an orientation layer comprising one or more polymers or oligomers according to the invention in a cross-linked form. Such orientation layers can be used in the manufacture of unstructured or structured optical-or electro-optical elements, preferably in the production of hybrid layer elements.

The orientation layers are suitably prepared from a solution of the polymer or oligomer material. The polymer or oligomer solution is applied to a support optionally coated with an electrode [for example a glass plate coated with indium-tin oxide (ITO)] so that homogeneous layers of 0.05 to 50 µm thickness are produced. In this process different coating techniques like spin-coating, meniscus-coating, wire-coating, slot-coating, offset-printing, flexo-printing, gravur-printing may be used. Then, or optionally after a prior imidisation step, the regions to be oriented are irradiated, for example, with a high-pressure mercury vapour lamp, a xenon lamp or a pulsed UV laser, using a polarizer and optionally a mask for creating images of structures.

The irradiation time is dependent upon the output of the individual lamps and can vary from a few seconds to several hours: The photo-reaction (dimerisation; polymerization, cross-linking) can also be carried out, however, by irradiation of the homogeneous layer using filters that, for example, allow only the radiation suitable for the cross-linking reaction to pass through.

It is understood that the polymer or oligomer layers of the invention may be used in the production of optical or electro-optical devices having at least one orientation layer as well as unstructured and structured optical elements and multi-layer systems.

A further embodiment of the invention relates to an optical or electro-optical device comprising one or more polymers or oligomers according to the present Invention in cross-linked form. The electro-optical devices may comprise more than one layer. The layer, or each of the layers may contain one or more regions of different spatial orientation.

### EXAMPLE 1

### Synthesis

### Preparation of (2E)-3-(4-{[4-(4,4,4-trifluorobutoxy)benzoyl]oxy}phenyl)acrylic acid

1.1 Preparation of 4-Formylphenyl-4-(4,4,4-trifluorobutoxy)benzoate 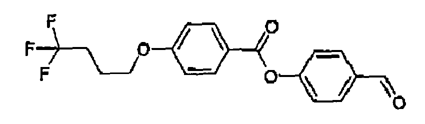 6.89 g (56.4 mmol) of 4-hydroxybenzaldehyd, 14.0 g (56.4 mmol) of 4-(4,4,4-trifluorobutoxy)benzoic acid, 0.69 g (5.6 mmol) of 4-Dimethylaminopyridine were dissolved in 100 ml of dichloromethane. 11.89 g (62.0 mmol) of N-(3-Dimethylaminopropyl)-N'-ethylcarbodiimide hydrochloride (EDC hydrochloride) were added at 0°C, The solution was stirred for 1 h at 0°C and allowed to stir at room temperature overnight. After 22 hours at room temperature the reaction mixture was partitioned between dichloromethane and water; the organic phase was washed repeatedly with water, dried over sodium sulphate, filtered and concentrated by rotary evaporation. Crystallization form 2-propanol at 0°C give 17.1 g 4-formylphenyl-4-(4,4,4-trifluorobutoxy)benzoate as colorless crystals.
1.2 Preparation of (2E)-3-(4-{[4-(4,4,4-trifluorobutoxy)benzoyl]oxy}phenyl)acryilc acid 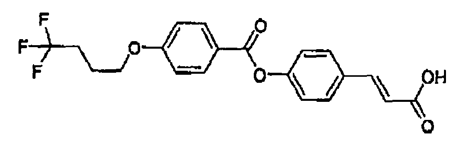 5.00 g (14.2 mMol) of 4-formylphenyl 4-(4,4,4-trifluorobutoxy)benzoate and 3.00 g (28.4 mMol) of Malonic acid were dissolved in 18 ml (227.1 mMol) of Pyridin.1.21 g (14.2 mMol) of Piperidin were added to the suspension which was allowed to react at 100°C under argon for 1.5 h. The yellow solution was then thrown on ice. The solution was carefully acidified to pH=1-2 with a 25% HCl solution and was stirred for 15 min. The product was filtrated off and dried at room temperature under vacuum for 10 h to give 5.2 g of (2E)-3-(4-{[4-(4,4,4-trifluorobutoxy)benzoyl]oxy}phenyl)acrylic acid as white powder.

### The following acrylic acid were synthesized in an analogous manner

(2E) 3-{4-[(4-trifluoromethoxybenzoyl)oxy]phenyl}acrylic acid
(2E) 3-{4-[(4-(2,2,2-trifluoroethoxy)benzoyl)oxy]phenyl}acrylic acid
(2E) 3-{4-[(4-(2,2,2-trifluoropropoxy)benzoyl)oxy]phenyl}acrylic acid
(2E) 3-{4-[(4-(5,5,5-trifluoropentyloxy)benzoyl)oxy]phenyl}acrylic acid
(2E) 3-{4-[(4-(4,4,5,5,5-pentafluoropentyloxy)benzoyl)oxy]phenyl}acrylic acid.
(2E) 3-{4-[(4-(1,1,2,2-tetrafluoroethoxy)benzoyl)oxy]phenyl}acrylic acid
(2E) 3-{4-[(4-(1,1,2,2-tetrafluoropropoxy)benzoyl)oxy] phenyl}acrylic acid
(2E) 3-{4-[(4-(4,4,5,5,6,6,6-heptafluorohexyloxy)benzoyl)oxy]phenyl}acrylic acid

### EXAMPLE 2

### Synthesis

### Preparation of 6-{[((2E)-3-{4-[(4-(4,4,4-thffuorobutoxy)benzoyl)oxy]phenyl}prop-2-enoyl)oxy]}hexyl 3,5-diaminobenzoate

2,1 Preparation of 4-(4,4,4-trifluorobutoxy)benzoic acid 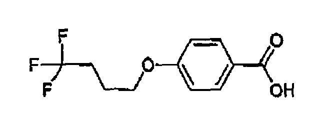 55.00 g (0.408 Mol) 4,4,4-tiffluorobutan-1-ol were dissolved in 550 ml tetrahydrofurane, 142 ml (0.102 Mol) triethylamine were added at room temperature.38 ml (0.490 Mol) methanesulfonyl chloride were added dropwise under nitrogen. The mixture was stirred for 1 h at 0-5°C. The beige suspension was Hyflo-filtrated and washed with tetrahydrofurane. The filtrate was concentrated. The residue was dissolved in 1.4 l 1-methyl-2-pyrrolidone 62.70 g (0.408 Mol) of methyl 4-hydroxybenzoate and 226.00 g (1.43 Mol) of potassium carbonate were added to the lightly brown solution. The reaction suspension was allowed to react at 80°C for 14 h. 1 l (1.0 Mol) of a 1 N NaOH solution was added to the above mixture. The suspension was heated at reflux temperature for 30 min until the reaction was completed. The reaction mixture was allowed to cool at room temperature and thrown in cold water. The solution was carefully acidified with a 25% HCl solution and was stirred for 15 min. The product was filtrated off, washed with water and dried overnight at room temperature under vacuum to give 99.00 g (98%)of 4-(4,4,4-trifluorobutoxy)benzoic acid as a white solid.
2.2 Preparation of (2E)-3-{4-[(ethoxycarbonyl)oxy]phenyl}acrylic acid 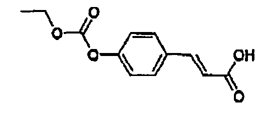 67 g (0.41 mol) p-cumaric acid were added to a mixture of 50.4 g (0.90 mol) potassium hydroxide and 600 ml water. 53.1g (0.50 mol) ethyl chloroformate were added dropwise at 0°C. The reaction temperature rise to 10°C, The reaction mixture was subsequently allowed to react for 2 hours at 25°C and acidified to pH=1 with 200 ml hydrochloric acid 7 N. The product was filtered off, washed with water and dried under vacuum to give 95.3 g of (2E)-3-{4-[(ethoxycarbonyl)oxy]phenyl}acrylic acid as white powder.
2.3 Preparation of 6-hydroxyhexyl 3,5-dinitrobenzoate 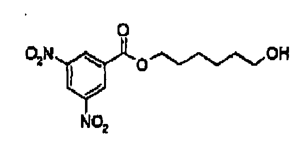 357.70 g (1.686 Mol) of 3,5-dinitrobenzoic acid were suspended in 750 ml of 1-methy-2-pyrrolidone. The suspension was stirred up to 50°C. 386.36 g (4.599 Mol) of sodium hydrogen carbonate were added and the mixture was heated up to 90°C. 22.50 g (0.150 Mol) of sodium iodide and 204.0 ml (1.533 Mol) of 6-chlorohexanol were added to the reaction mixture which was heated to 100°C for 1 h. After 1 h of reaction, the reaction was complete and the orange suspension was thrown on 2 1 of ice and 1 l of water. The product was filtrated, washed water and dried at 50°C under vacuum for 24 h to give 425.0 g (91%) of 6-hydroxyhexyl 3,5-dinitrobenzoate as a rose powder.
2.4 Preparation of 6-[({2E)-{4-[(ethoxycarbonyl)oxy]phenyl}prop-2-enoyl)oxy]hexyl 3,5-dinitrobenzoate 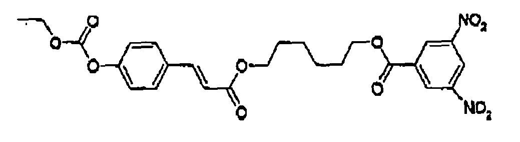 4.53 g (0.0145 Mol) of 6-hydroxyhexyl 3,5-dinitrobenzoate, 3.44 g (0.0145 Mol) of 4-ethylcarbonatecinnamic acid, 0.177 g (0.0015 Mol) of 4-Dimethylaminopyridine were dissolved in 40 ml of dichloromethane, 3.04 g (0.0159 Mol) of N-(3-Dimethylaminopropyl)-N'-ethylcarbodiimide hydrochloride (EDC hydrochloride) were added at 0°C. The solution was stirred for 1 h at 0°C and allowed to stir at room temperature overnight. After 22 hours at room temperature the reaction mixture was partitioned between dichloromethane and water, the organic phase was washed repeatedly with water, dried over sodium sulphate, filtered and concentrated by rotary evaporation. The residue was dissolved ethyl acetate. The product was precipitated with Hexane at 0°C. The precipitated was filtrated and dried under vacuum overnight to give 4.2 g (55%) of 6-[((2E)-{4[(ethoxycarbonyl)oxy]phenyl}prop-2-enoyl)oxy]hexyl 3,5-dinitrobenzoate as a light-yellow powder.
2.5 Preparation of B-[((2E)-{4-hydroxyphenyl}prop-2-enoyl)oxy]hexyl 3,5-dinitrobenzoate 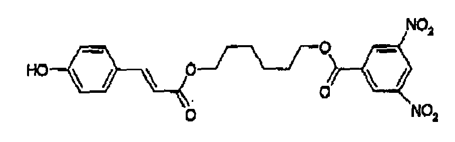 43.20 g (0.081 Mol) of 6-[((2E)-{4[(ethoxycarbonyl)oxy]phenyl}prop-2-enoyl}oxy]hexyl 3,5-dinitrobenzoate were dissolved in 66 ml (0.815 Mol) of pyridine and 400 ml of acetone at room temperature. 61 ml (0.815 Mol) of ammonium hydroxide solution 25% were added dropwise to the solution at room temperature. After 12h reaction, the mixture was thrown on water and acidified by the addition of HCl 25% (up to pH=3-4). A paste was obtained which was filtrated and dissolved in ethyl acetate and extracted with water. The organic phase was dried with sodium sulfate, filtrated, concentrated by rotary evaporation. Filtration of the residue over silica gel with *tert*-Butyl methyl ether as eluant and crystallization of the residue in 200 ml of ethyl acetate and 1200 ml of hexane at 0°C give 15.84 g of 6-[((2E)-(4-hydroxyphenyl)prop-2-enoyl)oxy]hexyl 3,5-dinitrobenzoate as yellow crystals.
2.6 Preparation of 6-{[((2E)-3-{4-[(4-(4,4,4-trifluorobutoxy)benzoy)oxy]phenyl}prop-2-enoyl)oxy]}hexyl 3,5-dinitrobenzoate 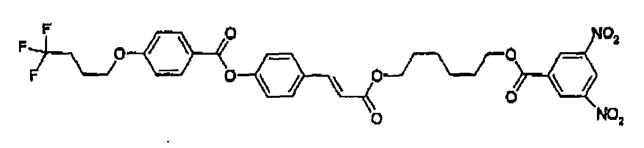 8.61 g (0.0347 Mol) of 4-(4,4,4-trifluorobutoxy)benzolo acid were suspended In 100 ml of dichloromethane, 0.42 g (0.0035 Mol) of 4-Dimethylaminopyridine were added at room temperature. 7.98 g (0.04163 Mol) of N-(3-Dimethylaminopropyl)-N'-ethylcarbodiimide hydrochloride (EDC hydrochloride) were added at 0°C. The solution was stirred for 1 h at 0°C. 15.90g (0.0347 Mol) of 6-[((2E)-{4-hydroxyphenyl}prop-2-enoyl)oxy]hexyl 3,5-dinitrobenzoate dissolved in 50 ml of dichloromethane were added dropwise to the solution at 0°C and allowed to stir at room temperature overnight. After 22 hours at room temperature the reaction mixture was partitioned between dichloromethane and water. The mixture was acidified with HCl 25%. The organic phase was washed repeatedly with water, dried over sodium sulphate, filtered and concentrated by rotary evaporation. Chromatography of the residue on 600 g silica gel using toluene:ethyl acetate (99:1) as eluant and crystallization from ethyl acetate/hexane (1:2) yielded 18.82 g (79 %) of 6-{[((2E)-3{4-[(4-(4,4,4-trifluorobutoxy)benzoyl)oxy]phenyl}prop-2-enoyl)oxy]}hexyl 3,5-dinitrobenzoate as white crystals.
2.7 Preparation of 6-{[((2E)-3-{4-[(4-(4,4,4-trifluorobutoxy)benzoyl)oxy]phenyl}prop-2-enoyl)oxy]}hexyl 3,5-diaminobenzoate 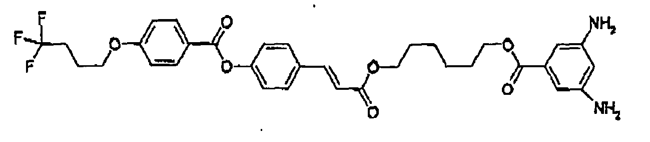 18.80 g (0.027 Mol) of 6-{[((2E)-3-{4-[(4-(4,4,4-trifluorobutoxy)benzoyl)oxy]phenyl} prop-2-enoyl)oxy]}hexyl 3,5-dinitrobenzoate were dissolved in a mixture of 350 ml of N,N-dimethylformamide and 25 ml water. 44.28 g (0.164 Mol) ferric chloride hexahydrate were added. 17.85 g (0.273 Mol) Zinc powder were added portionwise within 40 min. The mixture was allowed to react for 2 hours. The reaction mixture is then partitioned between ethyl acetate and water and filtered. The organic phase is washed repeatedly with water, dried over sodium sulfate, filtered and concentrated by rotary evaporation. Chromatography of the residue on 400 g silica gel using toluene:ethyl acetate(2:1) as eluant yielded 15.39 g (91%) of 6-{[((2E)-3-{4-[(4-(4,4,4-trifluorobutoxy)benzoyl)oxy]phenyl}prop-2-enoyl)oxy]}hexyl 3,6-diaminobenzoate as yellowish crystals.

### The following diamines were synthesized in an analogous manner:

2-{[((2E)-3-{4-[4-[(4-(trifluoroethoxy)benzoyl)oxy]}ethyl}prop-2-enoyl)oxy]}ethyl 3,5-diaminobenzoate.
3-{[((2E)-3{4-[(4-(trifluoroethoxy)benzoyl)oxy]phenyl}prop-2-enoyl)oxy]}propyl 3,5-diaminobenzoate.
4-{[((2E)-3-{4-[(4-(trifluoroethoxy)benzoyl)oxy]phenyl}prop-2-enoyl)oxy]}butyl 3,5-diaminobenzoate.
5-{[((2E)-3-{4-[(4-(trifluoroethoxy)benzoyl)oxy]phenyl}prop-2-enoyl)oxy]}pentyl 3,5-diaminobenzoate
7-{[((2E)-3-{4-[(4-(trifluoroethoxy)benzoyl)oxy]phenyl}prop-2-enoyl)oxy]}heptyl 3,5-diaminobenzoate.
8-{[((2E)-3-{4-[(4-(trifluoroethoxy)benzoyl)oxy]phenyl]prop-2-enoyl)oxy]}octyl 3,5-diaminobenzoate.
11-{[((2E)-3-{4-[(4-(trifluoroethoxy)benzoyl)oxy]phenyl}prop-2-enoyl)oxy]} undecyl 3,5-diaminobenzoate.
2-{[((2E)-3-{4-[(4-(trifluoromethoxy)benzoyl)oxy]phenyl}prop-2-enoyl)oxy]}ethyl 3,5-diaminobenzoate.
3-{[((2E)-3-{4-[(4-(trifluoromethoxy)benzoyl)oxy]phenyl}prop-2-enoyl)oxy]}propyl 3,5-diaminobenzoate.
4-{[((2E)-3-{4-[(4-(trifluoromethoxy)benzoyl)oxy]phenyl}prop-2-enoyl)oxy]}butyl 3,5-diaminobenzoate.
5-{[((2E)-3-{4-[(4-(trifluoromethoxy)benzoyl)oxy]phenyl}prop-2-enoyl)oxy]}pentyl 3,5-diaminobenzoate
6-{[((2E)-3-{4-[(4-(trifluoromethoxy)benzoyl)oxy]phenyl}prop-2-enoyl)oxy]}hexyll 3,5-diaminobenzoate
7-{[((2E)-3-{4-[(4-(trifluoromethoxy)benzoyl)oxy]phenyl}prop-2-enoyl)oxy]}heptyl 3,5-diaminobenzoate.
8-{[((2E)-3-{4-[(4-(triftuoromethoxy)benzoyl)oxy]phenyl}prop-2-enoyl)oxy]}octyl 3,5-diaminobenzoate.
2-{[((2E)-3-{4-[(4-(trifluoromethyl)benzoyl)oxy]phenyl}prop-2-enoyl)oxy]}ethyl 3,5-diaminobenzoate,
3-{[((2E)-3-{4-[(4-(trifluoromethyl)benzoyl)oxy]phenyl}prop-2-enoyl)oxyl]}propyl 3,5-diaminobenzoate.
4-{[((2E)-3-{4-[(4-(trifluoromethyl)benzoyl)oxy]phenyl}prop-2-enoyl)oxy]}butyl 3,5-diaminobenzoate.
5-{[((2E)-3-{4-[(4-(trifluoromethyl)benzoyl)oxy]phenyl}prop-2-enoyl)oxy]}pentyl 3,5-diaminobenzoate
6-{[((2E)-3-{4-[(4-(trifluoromethyl)benzoyl)oxy]phenyl}prop-2-enoyl)oxy]}hexyl) 3,5-diaminobenzoate
8-{[((2E)-3-{4-[(4-(trifluoromethyl)benzoyl)oxy]phenyl}prop-2-enoyl)oxy]}octyl 3,5-diaminobenzoate
11-{[((2E)-3-{4-[(4-(trifluoromethyl)benzoyl)oxy]phenyl}prop-2-enoyl)oxy]}undecyll 3,5-diaminobenzoate
2-[2-{[((2E)-3-{4-[(4-(trifluoroethoxy)benzoyl)oxy]phenyl}prop-2-enoyl)oxY]}ethoxy]ethyl 3,5-diaminobenzoate
2{2-[2-{[((2E)-3-{4-[(4-(trifluoroethoxy)benzoyl)oxy]phenyl}prop-2-enoyl)oxy]}ethoxy]ethoxy}ethyl 3,5-diaminobenzoate
2,2-dimethyl-3-{[((2E)-3-{4-[(4-(trifluoroethoxy)benzoyl)oxy]phenyl}prop-2-enoyl)oxy]}propyl 3,5-diaminobenzoate
2-{[((2E)-3-{4-[(4-(3,3,3-trifluoropropoxy)benzoyl)oxy]phenyl}prop-2-enoyl)oxy]}ethyl 3,5-diaminobenzoate
3-{[((2E)-3-{4-[(4-(3,3,3-trifluoropropoxy)benzoyl)oxy]phenyl}prop-2-enoyl)oxy]}propyl 3,5-diaminobenzoate
4-{[((2E)-3-{4-[(4-(3,3,3-trifluoropropoxy)benzoyl)oxy]phenyl}prop-2-enoyl)oxy]}butyl 3,5-diaminobenzoate.
6-{[((2E)-3-{4-[(4-(3,3,3-trifluoropropoxy)benzoyl)oxy]phenyl}prop-2-enoyl)oxy]}hexyl 3,5-diaminobenzoate
7-{[((2E)-3-{4-[(4-(3,3,3-trifluoropropoxy)benzoyl)oxy]phenyl}prop-2-enoyl)oxy]}heptyl 3,5-diaminobenzoate
8-{[((2E)-3-{4-[(4-(3,3,3-trifluoropropoxy)benzoyl)oxy]phenyl}prop-2-enoyl)oxly]}octyl 3,5-diaminobanzoate
11-{[((2E)-3-{4-[(4-(3,3,3-trifluaropropoxy)benzoyl)oxy]phenyl}prop-2-anoyl)oxy]} undecyl 3,5-diaminobenzoate
2-[2-{[((2E)-3-{4-[(4-(3,3,3-trifluoropropoxy)benzoyl)oxy]phenyl}prop-2-enoyl)oxyl]}ethoxy]ethyl 3,5-diaminobenzoate
2{2-[2-{[((2E)-3-{4-[(4-(3,3,3-trifluoropropoxy)benzoyl)oxy]phenyl}prop-2-enoyl)oxy]}ethoxy]ethoxy}ethyl 3,5-diaminobenzoate
2,2-dimethyl-3-{[((2E)-3{4-[(4-(3,3,3-trifluoropropoxy)benzoyl)oxy]phenyl}prop-2-enoyl)oxy]}propyl 3,5-diaminobenzoate
2-{[((2E)-3-{4-[(4-(4,4,4-trifluorobutoxy)benzoyl)oxy]phenyl}prop-2-anoyl)oxy]}ethyl 3,5-diaminobanzoate
3-{[((2E)-3-{4-[(4-(4,4,4-trifluorobutoxy)benzoyl)oxy]phenyl}prop-2-enoyl)oxy]}propyl 3,5-diaminobenzoate.
4-{[((2E)-3-{4-[(4-(4,4,4-trifluorobuloxy)benzoyl)oxy]phenyl}prop-2-enoyl)oxy]}butyl 3,5-diaminobenzoate.
5-{[((2E)-3-{4-[(4-(4,4,4-trifluorobutoxy)benzoyl)oxy]phenyl}prop-2-enoyl)oxy]}pentyl 3,5-diaminobenzoate
7-{[((2E)-3-(4-[(4-(4,4,4-trifluorobutoxy)benzoyl}oxy]phenyl}prop-2-enoyl)oxyl]}heptyl 3,5-diaminobenzoate.
8-{[((2E)-3-{4-[(4-(4,4,4-trifluorobutoxy)benzoyl)oxy]phenyl}prop-2-enoyl)oxy]}octyl 3,5-diaminobenzoate.
11-{[((2E)-3-{4-[(4-(4,4,4-trifluorobutoxy)benzoyl)oxy]phenyl}prop-2-enoyl)oxy]} undecyl 3,5-diaminobenzoate.
2-[2-{[((2E)-3-{4-[(4-(4,4,4-trifluorobutoxy)banzoyl)oxy]phenyl}prop-2-enoyl)oxy]}ethoxy]ethyl 3,5-diaminobenzoate
2{2-[2-{[((2E)-3-{4-[(4-(4,4,4-trifluorobutoxy)benzoyl)oxy]phenyl}prop-2-enoyl)oxy]}ethoxy]ethoxy}ethyl 3,5-diaminobenzoate
2,2-dimethyl-3{[((2E)-3-{4-[(4-(4,4,4-trifluorobutoxy)benzoyl)oxy]phenyl}prop-2-enoyl)oxy]}propyl 3,5-diaminobenzoate
2-{[((2E)-3-{4-[(4-(5,5,5-trifluoropentoxy)benzoyl)oxy]phenyl}prop-2-enoyl)oxy]}ethyl 3,5-diaminobenzoate
3-{[((2E)-3-{4-[(4-(5,5,5-trifluoropentoxy)benzoyl)oxy]phenyl}prop-2-enoyl)oxyl]}propyl 3,5-diaminobenzoate
4-{[((2E)-3-{4-[(4-(5,5,5-trifluoropentoxy)benzoyl)oxy]pheny)}prop-2-enoyl)oxy]}butyl 3,5-diaminobenzoate
5-{[((2E)-3-{4-[(4-(5,5,6-trifluoropentoxy)benzoyl)oxy]phenyl}prop-2-enoyl)oxy]}pentyl 3,5-diaminobenzoate
6-{[((2E)-3-{4-[(4-(5,5,5-trifluoropentoxy)benzoyl)oxy]phenyl}prop-2-enoyl)oxy]}hexyl 3,5-diaminobenzoate
8-{[((2E)-3-{4-[(4-(5,5,5-trifluoropentoxy)benzoyl)oxy]phenyl}prop-2-enoyl)oxy]}octyl 3,5-diaminobenzoate
11-{[((2E)-3-{4-[(4-(5,5,5-trifluoropentoxy)benzoyl)oxy]phenyl}prop-2-enoyl)oxy]} undecyl 3,6-diaminobenzoate
2-[2-{[((2E)-3-{4-[(4-(5,5,5-trifluoropentoxy)benzoyl)oxy]phenyl}prop-2-enoyl)oxy]}ethoxy]ethyl 3,5-diaminobenzoate
2{2-[2-{{((2E)-3-{4-[(4-(5,5,5-trifluoropentoxy)benzoyl)oxy]phenyl}prop-2-enoyl)oxy]}ethoxy]ethoxy}ethyl 3,5-diaminobanzoate
2,2-dimethyl-3-{[((2E)-3-{4-[(4-(5,5,5-trifluoropentoxy)benzoyl)oxy]phenyl}prop-2-enoyl)oxy]}propyl 3,5-diaminobenzoate
2-{[((2E)-3-{4-[(4-(6,6,6-trifluorohexyloxy)benzoyl)oxy]phenyl}prop-2-enoyl)oxy]}ethyl 3,5-diaminobenzoate
3-{[(((2E)-3-{4-[(4-(6,6,6-trifluorohexyloxy)benzoyl)oxy]phenyl}prop-2-enoyl)oxy]}propyl 3,5-diaminobenzoate
4-{[((2E)-3-{4-[(4-(6,6,6-trifluorohexyloxy)benzoyl)oxy]phenyl}prop-2-enoyl)oxy]}butyl 3,5-diaminobenzoate
5-{[((2E)-3-{4-[(4-(6,6,6-trifluorohexyloxy)benzoyl)oxy]phenyl}prop-2-enoyl)oxy]}pentyl 3,5-diaminobenzoate
7-{[((2E)-3-{4-[{4-(6,6,6-trifluorohexyloxy)benzoyl)oxy]phenyl}prop-2-enoyl)oxy]}heptyl 3,5-diaminobenzoate
8-{[((2E)-3-{4-[(4-(6,6,6-trifluorohexyloxy)benzoyl)oxy]phenyl}prop-2-enoyl)oxy]}octyl 3,5-diaminobenzoate
11-{[((2E)-3-{4-[{4-(6,6,6-trifluorohexyloxy)benzoyl)oxy]phenyl}prop-2-enoyl)oxy]} undecyl 3,5-diaminobenzoate
2-[2-{[((2E)-3-{4-[(4-(6,6,6-trifluorohexyloxy)benzoyl)oxy]phenyl}prop-2-enoyl)oxy]}ethoxy]ethyl 3,5-diaminobenzoate
2{2-[2-{[((2E)-3-{4-[(4-(6,6,6-trifluorohexyloxy)benzoyl)oxy]phenyl}prop-2-enoyl)oxy]}ethoxy]ethoxy}ethyl 3,5-diaminobenzoate
2,2-dimethyl-3-([((2E)-3-{4-[(4-(6,6,6-trifluorohexyloxy)benzoyl)oxy]phenyl}prop-2-enayl)oxy]}propyl 3,5-diaminobenzoate
3-{[((2E)-3-{4-[(3-methoxy 4-(3,3,3-trifluoropropoxy)benzoyl)oxy]phenyl}prop-2-enoyl)oxy]}propyl 3,5-diaminobenzoate
8-{[((2E)-3-{4-[(3-methoxy 4-(3,3,3-trifluompropoxy)benzoyl)oxy]phanyorop-2-enoyl)oxy]}octyl 3,5-diaminoberizoate
11-{[((2E)-3-{4-[(3-methoxy 4-(3,3,3-trifluoropropoxy)benzoyl)oxy]phenyl}prop-2-enoyl)oxy]} undecyl 3,5-diaminobenzoate
6-{[((2E)-3-{4-[(3-methoxy 4-(4,4,4-trifluorobutoxy)benzoyl)oxy]phenyl}prop-2-enoyl)oxy]}hexyll 3,5-diaminobenzoate
8-{[((2E)-3-{4-[(3-methoxy 4-(4,4,4-trifluorobutoxy)benzoyl)oxy]phenyl}prop-2-enoyl)oxy]}octyl 3,5-diaminobenzoate
11-{[((2E)-3-{4-[(3-methoxy 4-(4,4,4-trifluorobutoxy)benzoyl)oxy]phenyl}prop-2-anoyl)oxy]} undecyl 3,5-diaminobenzoate
4-{[((2E)-3-{4-[(3-methoxy 4-(5,5,5-trifluoropentoxy)benzoyl)oxy]phenyl}prop-2-enoyl)oxy]}butyl 3,5-diaminobenzoate
6-{[((2E)-3-{4-[(3-methoxy 4-(5,5,5-trifluoropentoxy)benzoyl)oxy]phenyl}prop-2-enoyl)oxy]}hexyl3,5-diaminobenzoate
4-{[((2E)-3-{4-[(3-methoxy 4-(6,6,6-trifluorohexyloxy)benzoyl)oxy]phenyl}prop-2-enoyl)oxy]}butyl 3,5-diaminobenzoate
6-{[((2E)-3-{4-[(3-methoxy 4-(6,6,6-trifluorohexyloxy)benzoyl)oxy]phenyl}prop-2-enoyl)oxy]}hexyl 3,5-diaminobenzoate
2-{[((2E)-3-{4-[(4-{4,4,5,5,5-pentafluoropentoxy)phenzoyl)oxy]phenyl}prop-2-enoyl)oxyl]}ethyl 3,5-diaminobenzoate
3-{[((2E)-3-{4-[(4-(4,4,5,5,5-pentafluoropentoxy)benzoyl)oxy]phenyl}prop-2-enoyl)oxy]}propyl 3,5-diaminobenzoate
4-{[((2E)-3-{4-[(4-(4,4,5,5,5-pentafluoropentoxy)benzoy)oxy]pheny}prop-2-enoyl)oxy]}butyl 3,5-diaminobanzoate
6-{[((2E)3-{4-[(4-(4,4,5,5,5-pentafluoropentoxy)benzoyl)oxy]phenyl}prop-2-enoyl)oxy]}hexyl 3,5-diaminobenzoate
7-{[((2E)-3-{4-{(4-(4,4,5,5,5-pentafluoropentoxy)benzoyl)oxy]phenyl}prop-2-enoyl)oxy]}heptyl 3,5-diaminobenzoate
8-{[((2E)-3-{4-[(4-(4,4,5,5,5-pentafluoropentoxy)benzoyl)oxy]pheny}prop-2-enoyl)oxy]}octyl 3,6-diaminobenzoate
11-{[((2E)-3-{4-[(4-(4.4,5,5,5-pentafluoropentoxy)benzoyl)oxy]phenyl}prop-2-enoyl)oxy]} undecyl 3,5-diaminobenzoate
2-[2-{[((2E)-3-{4-[(4-(4,4,5,5,5-pentafluoropentoxy)benzoyl)oxy]phenyl}prop-2-enoyl)oxy]}ethoxy]ethyl 3,5-diaminobenzoate
2{2-[2-{[((2E)-3-{4-[(4-(4,4,5,5,5-pentafluoropentoxy)benzoyl)oxy]phenyl}prop-2-enoyl)oxy]}ethoxy]ethoxy}ethyl 3,5-diaminobenzoate
2,2-Dimethyl-3-{[((2E)-3-{4-[(4-(4,4,5,5,5-pentafluoropentoxy)benzoyl)oxy]phenyl}prop-2-enoyl)oxy]}propyl 3,5-diaminobenzoate
2-{[((2E)-3-{4-[(4-(1,1,2,2-tetrafluoroethoxy)benzoyl)oxy]phenyl}prop-2-enoyl)oxy]}ethyl 3,5-diaminobenzoate
3-{[((2E)-3-{4-[(4-(1,1,2,2-tetrafluoroethoxy)benzoyl)oxy]phenyl}prop-2-enoyl)oxyl]}propyl 3,5-disminobenzoate
4-{[((2E)-3-{4-[(4-(1,1,2,2-tetrafluoroethoxy)benzoyl)oxy]phenyl}prop-2-enoyl)oxy]}butyl 3,5-diaminobenzoate
5-{[((2E)-3-{4-[(4-(1,1,2,2-tetrafluoroathoxy)benzoyl)oxy]phenyl}prop-2-enoyl)oxy]}pentyl 3,5-diaminobenzoate
6-{[((2E)-3-{4-[(4-(1,1,2,2-tetrafluoroethoxy)benzoyl)oxy]phenyl}prop-2-enoyl)oxy]}hexyll 3,5-diaminobenzoate
7-{[((2E)-3-{4-[(4-(1,1,2,2-tetrafluoroethoxy)benzoyl)oxy]phenyl}prop-2-enoyl)oxy]}heptyl 3,5-diaminobenzoate.
8-{[((2E)-3-{4-[(4-(1,1,2,2-tetrafluoroethoxy)benzoyl)oxy]phenyl}prop-2-enoyl)oxy]}octyl 3,5-diaminobenzoate
11-{[((2E)-3-{4-[(4-(1,1,2,2-tetrafluoroethoxy)benzoyl)oxy]phenyl}prop-2-enoyl)oxy]}undecy l 3,5-diaminobenzoate
6-{[((2E)-3-{4-[(4-{[(4,4,4-trifluorobutoxy)carbonyl]amino}benzoyl)oxy]phenyl}prop-2-enoyl)oxy]}hexyll 3,5-diaminobenzoate
6-{[((2E)-3-{4-[(4-{[(4,4,5,5,5-pentafluoropentoxy)carbonyl]amino}benzoyl)oxy] phenyl}prop-2-enoyl)oxy]}hexyll 3,5-diaminobenzoate
6-{[((2E)-3-{4-[(4-({[(4,4,5,5,6,6,6-heptafluorohexy)carbonyl]amino})benzoyl)oxy] phenyl}prop-2-enoyl)oxy]}hexyll 3,5-diaminobenzoate

### EXAMPLE 3

### Synthesis

### Preparation of 3,5-Diaminobenzyl (2E) 3-{4-(4-(4,4,4-trifluorobutoxy)benzoyl)oxy] phenyl}acrylate

3.1 Preparation of 3,5-dinitrobenzyl (2E)3-{4-[(4-(4,4,4-trifluorobutoxy) benzoyl)oxy]phenyl}acrylate 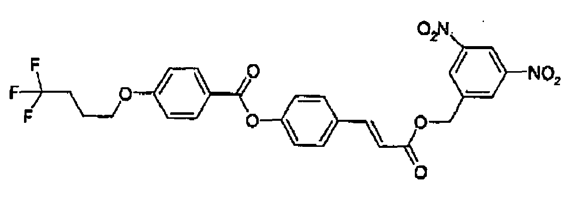 1.00 g (51.0 mmol) of 3,5-dinitrobenzylalcohol, 2.00 g (51.0 mmol) of (2E)-3-(4-{[4-(4,4,4-trifluorobutoxy)benzoyl]oxy}phenyl)acrylic acid, 62 mg (0.51 mmol) of 4-Dimethylaminopyridine were dissolved in 10 ml of dichloromethane. 1.07 g (58.0 mmol) of N-(3-Dimethylaminopropyl)-N'-ethylcarbodiimide hydrochloride (EDC hydrochloride) were added at 0°C. The solution was stirred for 1 h at 0°C and allowed to stir at room temperature overnight. After 22 hours at room temperature the reaction mixture was partitioned between dichloromethane and water. The organic phase was washed repeatedly with water, dried over sodium sulphate, filtered and concentrated by rotary evaporation to yield 3,5-dinitrobenzyl (2E)3-{4-[(4-(4,4,4-trifluorobutoxy) benzoyl)oxy]phenyl}acrylate 2.1 g as colorless crystals.
3.2 Preparation of 3,5-Diaminobenzyl (2E) 3-{4-[(4-(4,4,4-trifluorobutoxy)benzoyl)oxy]phenyl}acrylate 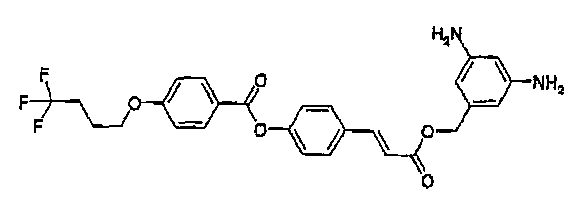 5.30 g (9.22 mol) of (2E)3-{4-[(4-(4,4,4-trifluorobutoxy) benzoyl)oxy]phenyl}acrylate were dissolved in a mixture of 55 ml of N,N-dimethylformamide and 6 ml water. 14.98 g (55.3 mmol) ferric chloride hexahydrate were added. 6.03 g (55.35 mmol) Zinc powder were added portionwise within 40 min. The mixture was allowed to react for 2 hours. The reaction mixture is then partitioned between ethyl acetate and water and filtered. The organic phase is washed repeatedly with water, dried over sodium sulfate, filtered and concentrated by rotary evaporation. Chromatography of the residue on 200 g silica gel using toluene:ethyl acetate(1:1) as eluant and crystallization form ethylacetate:hexane mixture yielded 3.8 g 3,5-Diaminobenzyl (2E) 3-{4-[(4-(4,4,4-trifluorobutoxy)benzoyl)oxy]phenyl}acrylate as yellowish crystals

### The following diamines were synthesized in an analogous manner:

3,5-Diaminobenzyl (2E) 3-{4-[(4-trifluoromethoxybenzoyl)oxy]phenyl}acrylate
3,5-Diaminobenzyl (2E) 3-{4-[(4-(2,2,2-trifluoroethoxy)benzoyl)oxy]phenyl}acrylate
3,5-Diaminobenzyl (2E) 3-{4-[(4-(5,5,5-trifluoropentyloxy)benzoyl)oxy]phenyl}acrylate
3,5-Diaminobenzyl (2E) 3-{4-[(4-(4,4,5,5,5-pentafluoropentyloxy)benzoyl)oxy] phenyl}acrylate
3,5-Diaminobenzyl (2E) 3-{4-[(4-(1,1,2,2-tetrafluoroethoxy)benzoyl)oxy] phenyl}acrylate
3,5-Diaminobenzyl (2E) 3-{4-[{4-(1,1,2,2-tetrafluoropropoxy)benzoyl)oxy] phenyl}acrylate
3,5-Diaminobanzyl (2E) 3-{4-[(4-(4,4,5,5,6,6,6-heptafluorohexyloxy)benzoyl)oxy] phenyl}acrylate
3,5-Diaminobenzyl (2E) 3-{4-{ [(4-(4,4,5,5,6,6,6-heptafluorohexyloylVoxy)benzoyl oxy]phenyl}acrylate
3,5-Diaminobenryl (2E)3-{4-[(4-[(4,4,4-trifluorobutoxy)carbonyl]amino)benzoyl)oxy] phenyl}acrylate
3,5-Diaminobenzyl (2E)3-{4-[4-(4,4,5,5,5,-pentafluoropentyloyloxy)benzoyl oxy]phenyl}acrylate
2-(3,5-Diaminophanyl)ethyl (2E) 3-{4-[(4-trifluoromethoxybenzoyl)oxy]phenyl}acrylate
2-(3,5-Diaminophenyl)ethyl (2E) 3-{4-[(4-(2,2,2-trifluoroethoxy)benzoyl)oxy]phenyl} acrylate
2-(3,5-Diaminophenyl)ethyl (2E) 3-{4-[(4-(4,4,4-trifluorobutoxy)benzoyl)oxy]phenyl} acrylate
2-(3,5-Diaminophenyl)ethyl (2E) 3-{4-[(4-(5,5,5-trifluoropentyloxy)benzoyl)oxy]phenyl} acrylate
2-(3,5-Diaminophenyl)ethyl (2E) 3-{4-[(4-(4,4,5,5,5-pentafluoropentoxy)benzoyl)oxy] phenyl}acrylate
2-(3,5-Diaminophenyl)ethyl (2E) 3-{4-[(4-(1,1,2,2-tetrafluoroethoxy)benzoyl)oxy] phenyl}acrylate
2-(3,5-Diaminophenyl)ethyl (2E) 3-{4-[(4-(4,4,4-trifluorobutanoyl)oxy)benzoyl]oxy} phanyl}acrylate
2-(2,5-Diaminophenyl)ethyl (2E) 3-{4-[(4-trifluoromethoxybenzoyl)oxy]phenyl}acrylate
2-(2,5-Diaminophenyl)ethyl (2E) 3-{4-[(4-(2,2,2-trifluoroethoxy)benzoyl)oxy]phenyl} acrylate
2-(2,5-Diaminophenyl)ethyl (2E) 3-{4-[(4-(4,4,4-trifluorobutoxy)benzoyl)oxy]phenyl} acrylate
2-(2,5-Diaminophenyl)ethyl (2E) 3-{4-[(4-(5,5,5-trifluoropentyloxy)benzoyl)oxy]phenyl} acrylate
2-(2,5-Diaminophenyl)ethyl (2E) 3-{4-[(4-(4,4,5,5,5-pentafluoropentoxy)benzoyl)oxy] phenyl}acrylate
2-(2,5-Diaminophenyl)ethyl (2E) 3-{4-[(4-(1,1,2,2-tetrafluoroethoxy)benzoyl)oxy] phenyl}acrylate
2-(2,5-Diaminophenyl)ethyl (2E) 3-{4-[(4-(4,4,4-trifluorobutanoyl)oxy)benzoyl]oxy] phenyl}acrylate
3-(3,5-Diaminophenyl)propyl (2E) 3-{4-[(4-trifluoromethoxybenzoyl)oxy]phenyl} acrylate
3-(3,5-Diaminophenyl)propyl (2E) 3-{4-[(4-(2,2,2-trifluoroethoxy)benzoyl)oxy]phenyl} acrylate
3-(3,5-Diaminophenyl)propyl (2E) 3-{4-[(4-(4,4,4-trifluorobutoxy)benzoyl)benzoyl)oxy]phenyl} acrylate
3-(3,5-Daminophenyl)propyl (2E) 3-{4-[(4-(5,5,5-trifluoropentyloxy)benzoyl)oxy]phenyl} acrylate
3-(3,5-Diaminophenyl)propyl (2E) 3-{4-[(4-(4,4,5,5,5-pentafluoropentoxy)benzoyl)oxy] phenyl}acrylate
3-(3,5-Diaminophenyl)propyl (2E) 3-{4-[(4-(1,1,2,2-tetraf)uoroethoxy)benzoyl)oxy] phenyl}acrylate
3-(3,5-Diaminophenyl)propyl (2E) 3-{4-[(4-(4.4.4-trifluorobutanoyl)oxy)benzoyl]oxy] phenyl}acrylate
2-(2,5--Diaminophenyl)ethyl (2E) 3-{4-[(3--methoxy-4-trifluoromethoxybenzayl)oxy]phenyl}acrylate
2-(2,5-Diaminophenyl)ethyl (2E) 3-{4-[(3-methoxy 4-(2,2-,2-trifluoroethoxy)benzoyl)oxy]phenyl] acrylate
2-(2,5-Diaminophenyl)ethyl (2E) 3-{4-[(3-methoxy 4-(4,4,4-trifluorobutoxy)benzoyl)oxy]phenyl} acrylate
2-(2,5-Diaminophenyl)ethyl (2E) 3-{4-[(3-methoxy 4-(5,5,5-trifluoropentyloxy)benzoyl)oxy]phenyl} acrylate
2-(2,5-Diaminophenyl)ethyl (2E) 3-{4-[(3-methoxy 4-(4,4,5,5,5-pentafluoropentoxy)benzoyl)oxy] phenyl}acrylate
2-(2,5-Diaminophenyl)ethyl (2E) 3-{4-[(3-methoxy 4-(1,1,2,2-tetrafluorethoxy)benzoyl)oxy] phenyl}acrylate
2-(2,5-Diaminophenyl)ethyl (2E) 3-{4-[(3-methoxy 4-(4,4,4-trifluorobutanoyl)oxy)benzoyl]oxy} phenyl}acrylate
2-(2,5-Diaminophenyl)ethyl (2E) 3-{4-[(4-trifluoromethoxybenzoyl)oxy]-3-methoxyphenyl}acrylate
2-(2,5-Diaminophenyl)ethyl (2E) 3-{4-[(4-(2,2,2-triflucroethoxy)benzoyl)oxy]-3-methoxyphenyl} acrylate
2-(2,5-Diaminophenyl)ethyl (2E) 3-{4-[(4-(4,4,4-trifluorobutoxy)benzoyl)oxy]-3-methoxy phenyl} acrylate
2-(2,5-Diaminophenyl)ethyl (2E) 3-{4-[(4-(5,5,5-trifluoropentyloxy)benzoytl)oxy]-3-methoxyphenyl} acrylate
2-(2,5-Diaminophenyl)ethyl (2E) 3-{4-[(4-(4,4,5,5,5-pentafluoropentoxy)benzoyl)oxy]-3-methoxyphenyl}acrylate
2-(2,5-Diaminophenyl)ethyl (2E) 3-{4-[(4-(1,1,2,2-tetrafluoroethoxy)benzoyl)oxy] -3-methoxyphenyl}acrylate
2-(2,5-Diaminophenyl)ethyl (2E) 3-{4-[(4-(4,4,4-trifluorobutanoyl)oxy)benzoyl]oxy}-3-methoxyphenyl}acrylate
2-(2,5-Diaminophenyl)ethyl (2E) 3-{4-[(4-trifluoromethoxyphenoxy)carbonyl]phenyl}acrylate
2-(2,5-Diaminophenyl)ethyl (2E) 3-{4-[(4-(2,2,2-trifluoroethoxy)phenoxy)carbonyl]phenyl} acrylate
2-(2,5-Diaminophenyl)ethyl (2E) 3-{4-[(4-(4,4,4-trifluorobutoxy)phenoxy)carbonyl]phenyl} acrylate
2-(2,5-Diaminophenyl)ethyl (2E) 3-{4-[(4-(5,5,5-trifluoropentyloxy)phenoxy)carbonyl]phenyl} acrylate
2-(2,5-Diarnincphenyl)ethyl (2E) 3-{4-[(4-(4,4,5,5,5-pentafluoropentoxy)phenoxy)carbonyl] phenyl}acrylate
2-(2,5-Diaminophenyl)ethyl (2E) 3-{4-[(4-(1,1,2,2-tetrafluoroethoxy)phenoxy)carbonyl] phenyl}acrylate
2-(2,4-Diaminophonyl)ethyl (2E) 3-{4-[(4-trifluoromethoxybenzoyl)oxy]phenyl}acrylate
2-(2,4-Diaminophanyl)ethyl (2E) 3-{4-[(4-(2,2,2-trifluoroethoxy)benzoyl)oxy]phenyl} acrylate
2-(2,4-Diaminophenyl)ethyl (2E) 3-{4-[(4-(4,4,4-trifluorobutoxy)benzoyl)oxy]phenyl} acrylate
2-(2,4-Diaminophenyl)ethyl (2E) 3-{4-[(4-(5,5,5-trifluoropentyloxy)benzoyl)oxy]phenyl} acrylate
2-(2,4-Diaminophenyl)ethyl (2E) 3-{4-[(4-(4,4,5,5,5-pentafluoropentoxy)benzoyl)oxy] phenyl}acrylate
2-(2,4-Diaminophenyl)ethyl (2E) 3-{4-[(4-(1,1,2,2-tetrafluoroethoxy)benzoyl)oxy] phenyl}acrylate
2-(2,4-Diaminophenyl)ethyl (2E) 3-{4-[(4-(1,1,2,2-tetrafluoropropoxy)benzoyl)oxy] phenyl}acrylate
2-(2,4-Diaminophenyl)ethyl (2E) 3-{4-[(4-(4,4.5.5.6.6,6-heptafluorohexyloxy)benzoyl)oxy] phenyl}acrylate
2-(2,4-Diaminophenyl)ethyl (2E) 3-{4-[(4-(4,4,4-trifluorobutanoyl)oxy)benzoyl]oxy} phenyl}acrylate
3-(2,4-Diaminophenyl)propyl (2E) 3-{4-[(4-(4,4,4-trifluorobutoxy)benzoyl)oxy]phenyl} acrylate
3-(2,4-Diaminophenyl)propyl (2E) 3-{4-[(4-(4,4,5,5,5-pentafluoropentoxy)benzoyl)oxy] phenyl}acrylate
3-(2,4-Diaminophenyl)propyl (2E) 3-(4-[(4-(1,1,2,2-tetrafluoroethoxy)benzoyl)oxy] phenyl}acrylate
3-(2,4-Diaminophenyl)propyl (2E) 3-{4-[(4-(4,4,4-trifluorobutanoyl)oxy)banzoyl]oxy} phenyl}acrylate
2-(2,4-Diaminophenyl)ethyl (2E) 3-{4-[(3-methoxy-4-trifluoromethoxybenzoyl)oxy]phenyl}acrylate
2-(2,4-Diaminophenyl)ethyl (2E) 3-{4-[(3-methoxy 4-(2,2,2-trifluoroethoxy)benzoyl)oxy]phenyl} acrylate
2-(2,4-Diaminophanyl)ethyl (2E) 3-{4-[(3-methoxy 4-(4,4,4-trifluorabutoxy)benzoyl)oxy]phenyl} acrylate
2-(2,4-Diaminophenyl)ethyl (2E) 3-{4-[(3-methoxy 4-(5,5,5-trifluoropentyloxy)benzoyl)oxy]phenyl} acrylate
2-(2,4-Diaminophenyl)ethyl (2E) 3-{4-[(3-methoxy 4-(4,4,5,5,5-pentafluoropentoxy)benzoyl)oxy] phenyl}yacrylate
2-(2,4-Diaminophenyl)ethyl (2E) 3-{4-[(3-methoxy 4-(1,1,2,2-tetrafluoroethoxy)benzoyl)oxy] phenyl}acrylate
2-(2,4-Diaminophenyl)ethyl (2E) 3-{4-[(3-methoxy 4-(4,4,4-trifluorobutanoyl)oxy)benzoyl]oxy)phenyl}acrylate
2-(2,4-Diaminophenyl)ethyl (2E) 3-{4-[(4-(4,4,4-trifluorobutoxy)benzoyl)oxy]-3-methoxy phenyl} acrylate
2-(2,4-Diaminophenyl)ethyl (2E) 3-{4-[(4-(5,5,5-trifluoropentyloxy)benzoyl)oxy]-3-methoxyphenyl} acrylate
2-(2,4-Diaminophenyl)ethyl (2E) 3-{4-[(4-(4,4,5,5,5-pentafluoropentoxy)benzoyl)oxy]-3-methoxyphenyl}acrylate
2-(2,4-Diaminophenyl)ethyl (2E) 3-{4[(4-(1,1,2,2-tetrafluomethoxy)benzoyl)oxy] -3-methoxyphenyl}acrylate
2-(2,4-Diaminophenyl)ethyl (2E) 3-{4-[(4-(4,4,4-trifluorobutanoyl)oxy)benzoyl]oxy}-3-methoxyphenyl}acrylate
2-(2,4-Diaminophenyl)ethyl (2E) 3-{4-[(4-(4,4,4-trifluorobutoxy)phenoxy)carbonyl]phenyl} acrylate
2-(2,4-Diaminophenyl)ethyl (2E) 3-{4-[(4-(5,5,5-trifluoropentyloxy)phenoxy)carbonyl]phenyl} acrylate
2-(2,4-Diaminophenyl)ethyl (2E) 3-{4-[(4-(4,4,5,5,5-pentafluoropentoxy)phenoxy)carbonyl] phenyl}acrylate
2-(2,4-Diaminophenyl)ethyl (2E) 3-{4-[(4-(1,1,2,2-tetrafluoroethoxy)phenoxy)carbony] phenyl}acrylate

### EXAMPLE 4

### Synthesis

### Preparation of 2,2-bis(4-aminobenzyl)-1,3 di[(2E)-3-{4-[(4-(4,4,4-trifluorobutoxy)benzoyl)oxy]phenyl}prop-2-enoyl] propanediol

4.1 Preparation of 2,2-dimethyl-5,5-bis(4-nitrobenzyl)-1,3-dioxane-4,6-dione 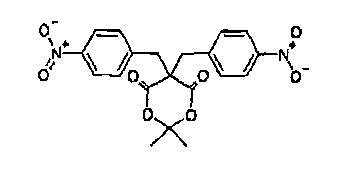 15.0 g (69.4 mmol) of 4-nitrobenzylbromide and 5.00 g (34.7 mmol) of Meldrum's acid were dissolved in 100 ml 2-butanone. 4.40 g (104.1 mmol) potassium carbonate were added, the resulting suspension was heated to 50°C and allowed to react for 2.5 hours. After cooling to room temperature, 100 ml water were added. The product was collected by filtration and washed with a lot of water. 12,3 g (85 %) of 2,2-dimethyl-5,5-bis(4-nitrobenzyl)-1,3-dioxana-4,6-dione as yellowish powder was used without further purification.
4.2 Preparation of 2,2-bis(4-nitrobenzyl)malonic acid 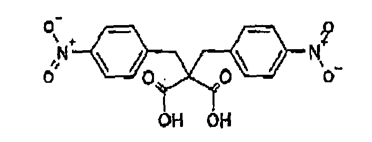 2.185 g (52.07 mmol) of lithium hydroxide were added to a suspension of 10.79 g (26.04 mmol) of 2,2-dimethyl-5,5-bis(4-nitrobenzyl)-1,3-dioxane-4,6-dione and 110 ml mixture of tetrahydrofurane: water 9:1. The mixture was subsequently allowed to react for 21.5 hours at 25°C, added to 500 ml water and acidified to pH=1 with 20 ml hydrochloric acid 3N. The mixture was partitioned between water and ethyl acetate; the organic phase was washed repeatedly with water, dried over sodium sulfate, filtered and concentrated by rotary evaporation. The residue 9.54 g (98%) of 2,2-bis(4-nitrobenzyl)malonic acid as white powder was used without further purification.
4.3 Preparation of 2,2-bis(4-nitrobenzyl)-1,3-propandiol 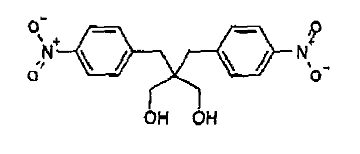 4.00 g (10.69 mmol) 2,2-bis(4-nitrobenzyl)malonic acid were dissolved in 40 ml tetrahydrofuran and added dropwise in a the course of 2 hours to 64.1 ml (64.1 mmol) of a borane-tetrahydrofuran complex 1.0 M solution in tetrahydrofuran. After 19 hours at 25°C, 50 ml water were carefully added. The reaction mixture was then partitioned between ethyl acetate and water; the organic phase was washed repeatedly with water" dried over sodium sulfate, filtered and concentrated by rotary evaporation. The residue, 3.77 g (97%) of 2,2-bis(4-nitrobenzyl)-1,3-propandiol as white powder was used without further purification.
4.4 Preparation 2,2-bis(4-nitrobenzyl-1,3 di[(2E)-3-{4-[(4-(4,4,4-trifluorobutoxy) benzoyl) oxy]phenyl}prop-2-enoyl] propanediol 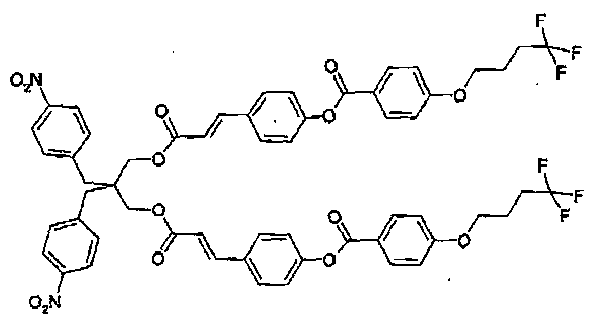 1.76 g (5.07 mmol) of 2,2-bis(4-nitrobenzyl)-1,3-propandiol, 4.00 g (10.14 mmol) of (2E)-3-(4-{[4-(4,4,4-trifluorobutoxy)benzoy]oxy}phenyl)acrylic acid,124 mg (1.01 mmol) of 4-Dimethylaminopyridine were dissolved in 100 ml of dichloromethane. 2.14 g (11.16 mmol) of N-(3-Dimethylaminopropyl)-N'-ethylcarbodiimide hydrochloride (EDC hydrochloride) were added at 0°C. The solution was stirred for 1 h at 0°C and allowed to stir at room temperature overnight. After 22 hours at room temperature the reaction mixture was partitioned between dichloromethane and water. The organic phase was washed repeatedly with water, dried over sodium sulphate, filtered and concentrated by rotary evaporation. Chromatography of the residue on 150 g silica gel using toluene:ethyl acetate 9:1 as eluant to yield 2.20 g 2,2-bis(4-nitrobenzyl)-1.3 di[(2E)-3-{4-[(4-(4,4,4-trifluorobutoxy)benzoyl)oxy]phenyl}prop-2-enoyl) propanediol as white crystals.
4.5 Preparation of 2,2-bis(4-aminobenzyl)-1,3 di[(2E)-3-{4-[(4-(4,4,4-trifluorobutoxy)benzoyl)oxy]phenyl}prop-2-enoyl) propanediol 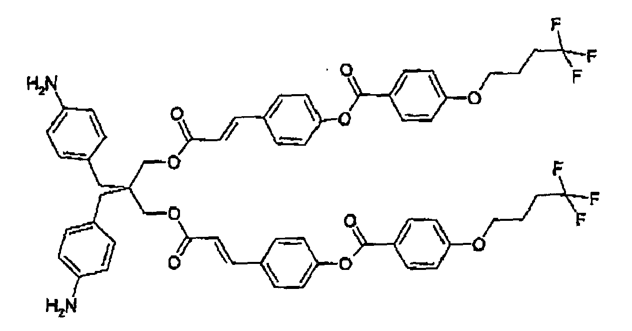 2.20 g (2.00 mol) of (2E)3-{4-[(4-(4,4,4-trifluorobutoxy) benzoyl)oxy]phenyl}acrylate were dissolved in a mixture of 25 ml of N,N-dimethylformamide and 3 ml water. 3.25 g (12.01 mmol) ferric chloride hexahydrate were added. 1.31 g (20.02 mmol) Zinc powder were added portionwise within 40 min. The mixture was allowed to react for 2 hours. The reaction mixture is then partitioned between ethyl acetate and water and filtered. The organic phase is washed repeatedly with water, dried over sodium sulfate, filtered and concentrated by rotary evaporation. Chromatography of the residue on 100 g silica gel using toluene:ethyl acetate 1:1 as eluant and crystallization form ethylacetate:hexane mixture to yield 1.20 g 2,2-bis(4-aminobenzyl)-1,3 di[(2E)-3-{4-[(4-(4,4,4-trifluorobutoxy)benzoyl)oxy]phenyl}prop-2-encyl] propanediol

The following diamines were synthesized in an analogous manner
2,2-bis(A-aminobenzyl)-1,3 di[(2E) 3-{4-[(4-trifluoromethoxybenzoyl)oxy]phenyl}prop-2-enoyl] propanediol
2,2.bis(4-aminobenzyl)-1,3 di[(2E) 3-{4-[(4-(2,2,2-trifluoroethoxy)benzoyl)oxy]phenyl} prop-2-enoyl] propanediol
2,2-bis(4-aminobenzyl)-1,3 di[(2E)3-{4-[(4-(4,4,4-trifluorobutoxy)cenzoyl)oxy]phenyl} prop-2-enoyl] propanediol
2,2-bis(4-aminobenzyl)-1,3 di[(2E) 3-(4-[(4-(5.5,5-trifluoropentyloxy)benzoyl)oxy]phenyl}prop-2-anoyl]propanediol
2,2-bis(4-aminobenzyl)-1,3di[(2E)3-{4-[(4-(4,4,5,5,5-pentafluoropentoxy)benzoyl)oxy] phenyl}prop-2-enoyl] propanediol
2,2-bis(4-aminobenzyl)-1,3 di[(2E) 3-{4-[(4-(1,1,2,2-tetrafluoroethoxy)benzoyl)oxy] phenyl}prop-2-enoyl] propanediol
2,2-bis(4-aminobenzyl)-1,3 di[(2E) 3{4-[(4-(4,4,4-trifluorobutanoyl)oxy)benzoyl]oxy} phenyl}prop-2-enoyl] propanediol
2,2-bis(4-aminobenzyl)-1,3 di[(2E) 3-{4-[(3-methoxy-4-trifluoromethoxybenzoyl)oxy]phenyl}prop-2-enoyl] propanediol
2,2.bis(4-aminobenzyl)-1,3 di[(2E) 3-{4-[(3-methoxy 4-(2,2,2-trifluoroethoxy}benzoyl)oxy]phenyl} prop-2-enoyl] propanediol
2,2-bis(4-aminobenzyl)-1,3 di[(2E) 3-{4-[(3-methoxy 4-(4,4,4-trifluorobutoxy)benzoyl)oxy]phenyl} prop-2-enoyl] propanediol
2,2-bis(4-aminobenzyl)-1,3 di[(2E) 3-{4-[(3-methoxy 4-(5,5,5-trifluoropentyloxy)benzoyl)oxy]phenyl} prop-2-enoyl] propanediol
2,2-bis(4-aminobenzyl)-1,3 di[(2E) 3-{4-[(3-methoxy 4-(4,4,5,5,5-pentafluoropentoxy)benzoyl)oxy] phenyl}prop-2-enoyl] propanediol
2.2-bis(4-aminobenzyl)-1,3 di[(2E) 3-{4-[(3-methoxy 4-(1,1,2,2-tetrafluoroethoxy)benzoyl)oxy] phenyl}prop-2-enoyl] propanediol
2,2-bis(4-aminobenzyl)-1,3 di[(2E) 3-{4-[(3-methoxy 4-(4,4,4-trifluorobutanoyl)oxy)benzoyl]oxy} phenyl}prop-2-enoyl] propanediol
2,2-bis(4-aminobenzyl)-1,3 di[(2E) 3-{4-[(4-trifluoromethoxybenzoyl)oxy]-3-methoxyphenyl}prop-2-enoyl] propanediol
2,2-bis(4-aminobenzyl)-1,3 di[(2E) 3-{4-[(4-(2,2,2-trifluoroethoxy)benzoyl)oxy]-3-methoxyphenyl} prop-2-enoyl] propanediol
2,2-bis(4-aminobenzyl)-1,3 di[(2E) 3-{4-[(4-(4,4,4,trifluorobutoxy)benzoyl)oxy]-3-methoxy phenyl} prop-2-enoyl] propanediol
2,2-bis(4-aminobanzyl)-1,3 di[(2E) 3-{4-[(4-(5,5,5-trifluoropentyloxy)benzoyl)oxy]-3-methoxyphenyl} prop-2-enoyl] propanediol
2,2-bis(4-aminobenzyl)-1,3 di[(2E) 3-{4-[(4-(4,4,5.5.5-pentafluoropentoxy)benzoyl)oxy]-3-methoxyphenyl}prop-2-enoyl] propanediol
2,2-bis(4-aminobenzyl)-1,3 di[(2E) 3-{4-[(4-(1,1,2,2-tetrafluoroethoxy)benzoyl)oxy] -3-methoxyphenyl}prop-2-enoyl] propanediol
2,2-bis(4-aminobenzyl)-1,3 di[(2E) 3-{4-{(4-(4,4,4-trifluorobutanoyl)oxy)benzoyl]oxy}-3-methoxyphenyl}prop-2-enoyl] propanediol
2,2-bis(4-aminobenzyl)-1,3 di[(2E) 3-{4-[(4-trifluoromethoxyphenoxy)carbonyl]phenyl}prop-2-enoyl] propanediol
2,2-bis(4-aminobenzyl)-1,3 di[(2E) 3-{4-[(4-(2,2,2-trifluoroethoxy)phenoxy)carbonyl]phenyl} prop-2-enoyl] propanediol
2,2-bis(4-aminobenzyl)-1,3 di[(2E) 3-{4[(4-(4,4,4-trifluorobutoxy)phenoxy)carbonyl]phenyl}prop-2-enoyl] propanediol
2.2-bis(4-aminobenzyl)-1,3 di[(2E) 3-{4-[(4-(5,5,5-trifluoropentyloxy)phenoxy)carbonyl]phenyl} prop-2-enoyl] propanediol
2,2-bis(4-aminobenzyl)-1,3 di[(2E) 3-{4-[(4-(4,4,5,5,5-pentafluoropentoxy)phenoxy)carbonyl] phenyl}prop-2-enoyl] propanediol
2,2-bis(4-amlnobanzyl)-1,3 di[(2E) 3-{4-[(4-(1,1,2,2-tetrafluoroethoxy)phenoxy)carbonyl] phenyl}prop-2-enoyl] propanediol

### EXAMPLE 5

### Synthesis

Preparation of 6-{[((2E)-3-{4-[(4-(4,4,4-trifluorobutoxy)benzoyl)oxy]phenyl}prop-2-enoyl)oxy]}hexyl 3,5-Diamino-4-[6-{[((2E)-3-{4-(4,4,-trifluorobutoxy)benzoyl)oxy]phenyl}prop-2-enoyl)oxy]}hexyloxy]benzoate
4.1 Preparation of 6-{[((2E)-3-{4-[(4-(4,4,4-trifluorobutoxy)benzoyl)oxy]phenyl}prop-2-enoyl)oxy]}hexyl 3,5-dinitro-4-[6-{[((2E)-3-{4-[(4-(4,4,4-trifluorobutoxy)benzoyl)oxy]phenyl}prop-2-enoyl)oxy]}hexyloxy]benzoate 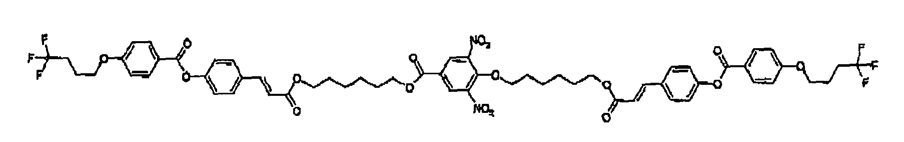 6.50 g (11.67 mmol) of 6-hydroxyhexyl 4-(6-hydroxyhexyloxy)-3,5-dinitrobenzoate, 9.67 g (24.53 mmol) of (2E)-3-(4-{[4-(4,4,4-trifluorobutoxy)benzoyl]oxy}pheny)acrylic acid,.290 mg (2.34 mmol) of 4-Dimethyfaminopyridine were dissolved in 100 ml of dichloromethane. 5.14 g (26.87 mmol) of N-(3-Dimethylaminopropyl)-N'-ethylcarbodiimide hydrochloride (EDC hydrochloride) were added at 0°C. The solution was stirred for 1 h at 0°C and allowed to stir at room temperature overnight. After 22 hours at room temperature the reaction mixture was partitioned between dichloromethane and water. The organic phase was washed repeatedly with water, dried over sodium sulphate, filtered and concentrated by rotary evaporation. Chromatography of the residue on 500 g silica gel using toluene:ethyl acetate 95:5 as eluant and crystallization form ethyl acetate:hexane mixture to yield 7,70 g of 6-{[((2E)-3-{4-[(4-(4,4,4-trifluorobutoxy)benzoyl)oxy]phenyl}prop-2-enoyl)oxy]}hexy 3,5-dinitro-4-[6-{[((2E)-3-{4-[(4-(4,4,4-trifluorobutoxy)benzoyl)oxy]phenyl}prop-2-enoyl)oxy]hexyloxy]benzoate as yellow crystals
4.2 Preparation of 6-{[((2E)-3q4-[(4-(4,4,4-trifluorobutoxy)benzoyl)oxy]phenyl)prop-2-enoyl)oxy]}hexyl 3,5-Diamino-4-[6-U((2E)-3-0-[(4-(4,4,4-trifluorobutoxy)benzoyl)oxy]phenyl}prop-2-enoyl)oxy]}hexyloxy]benzoate 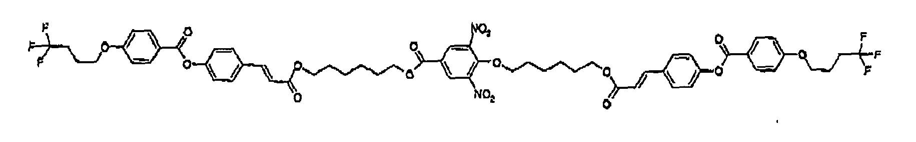 7.70 g (6.5 mol) of 6-{[((2E)-3-{4-[(4-(4,4,4-trifluorobutoxy)benzoyl)oxy]phenyl}prop-2-enoyl)oxy]}hexyl 3,5-dinitro-4-[6-{[((2E)-3-{4-[(4-(4,4,4-trifluorobutoxy)benzoyl) oxy]phenyl}prop-2-enoyl)oxy]}hexyloxy]benzoate were dissolved in a mixture of 90 ml of N,N-dimethylformamide and 7 ml water. 10.6 g (39.2 mmol) ferric chloride hexahydrate were added. 4.27 g (65.36 mmol) Zinc powder were added portionwise within 40 min. The mixture was allowed to react for 2 hours. The reaction mixture is then partitioned between ethyl acetate and water and filtered. The organic phase is washed repeatedly with water, dried over sodium sulfate, filtered and concentrated by rotary evaporation. Chromatography of the residue on 200 g silica gel using toluene:ethyl acetate 2:1 as eluant and crystallization form methanol:ethyl acetate mixture to yield 4.92 g 6-{[((2E)-3-{4-[(4-(4,4,4-trifluorobutoxy)benzoyl)oxy] phenyl}prop-2-enoyl)oxy]}hexyl 3,5-Diamino-4-[6-{[((2E)-3-{4-[(4-(4,4,4-trifluorobutoxy)benzoyl)oxy]phenyl}prop-2-enoyl]}hexyloxy]benzoate as colorless crystals.

### EXAMPLE 6

### Polymerization Step A (formation of the Polyamic Acid)

2.25g (11.47 mmol) of 1,2,3,4-cyclobutantetracarboxylic acid dianhydride was added to a solution of 8.030 g (12.77 mmol) of 6-{[((2E)-3-{4-[(4-(4,4,4-trifluorobutoxy)benzoyl)oxy]phenyl}prop-2-enoyl)oxy]}hexyl 3,5-Diaminobenzoate in 56.0 ml of tetrahydrofuran. Stirring was then carried out at 0 °C for 2 hours. Then another 0.255 g (1.30 mmol) of 1,2,3,4-cyclobutantetracarboxylic acid dianhydride were added. The mixture was subsequently allowed to react for 21 hours at room temperature. The polymer mixture was diluted with 56 ml THF, precipitated into 2000 ml diethyl ether and collected by filtration. The polymer was reprecipitated form THF (160 ml) into 3500 ml water to yield, after drying at room temperature under vacuum, 9.42 g of *Polyamic Acid 1* in the from of a white powder; [η] = 0.50 dL/g

Analogous to EXAMPLE 6 the following diamines were used for the preparation of Polyamic Acid with 1.2.3,4-cyclobutantetracarboxylic acid dianhydride
6-{[((2E)-3-{4-[(4-(3,3,3-trifluoropropoxy)benzoyl)oxy]phenyl}prop-2-enoyl)oxy]}hexyl 3,5-Diaminobenzoate 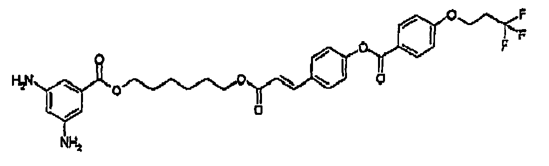 yield Polyamic acid 2 as white powder; [η] = 0.24 dUg
6-{[((2E)-3-{4-[(3-methoxy 4-(4,4,4-trifluorobutoxy)benzoyl)oxy]phenyl}prop-2-enoyl)oxy]}hexyll 3,5-Diaminobenzoate 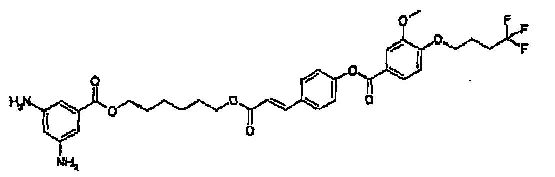 yield Polyamic acid 3 as white powder, [η] = 0.25 dL/g.
8-{[((2E)-3-{4-[(4,4,4-trifluorobutoxy)benzoyl)oxy]phenyl}prop-2-enoyl)oxy]}octyl 3,5-Diaminobenzoate 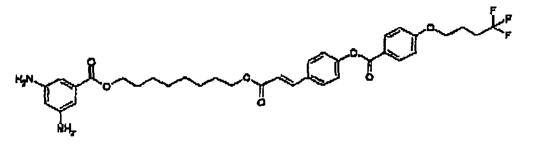 yield Polyamic acid 4 as white powder; [η] = 1.09 dUg.
4-{[((2E)-3-{4-[(4-(4,4,4-trifluorobutoxy)benzoyl)oxy]phenyl}prop-2-enoyl)oxy]}butyl 3,5-Diaminobenzoate 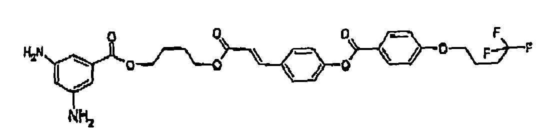 yield Polyamic acid 5 as white powder, [η] = 0.21 dL/g.
2-[2-{[((2E)-3-{4-[(4-(4,4,4-trifluorobutoxy)benzoyl)oxy]phenyl}prop-2-enoyl)oxy]}ethoxy]ethyl 3,5-Diaminobenzoate 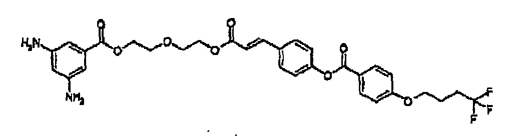 yield Polyamic acid 6 as white powder; [η] = 0.87 dL/g.
2-{[((2E)-3-{4-[(4,4,4-trifluorobutoxy)benzoyl)oxy]phenyl}prop-2-enoyl)oxy]}ethyl 3,5-Diaminobenzoate 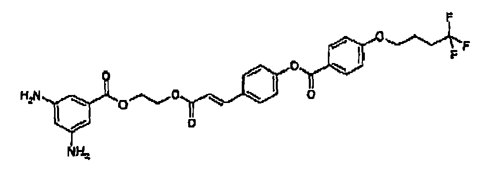 yield Polyamic acid 7 as white powder; [η] = 0.48 dL/g.
3-{[((2E)-3-{4-[(4-(4,4,4-trifluorobutoxy)benzoyl)oxy]phenyl}prop-2-enoyl)oxy]}propyl 3,5-Diaminobenzoate yield 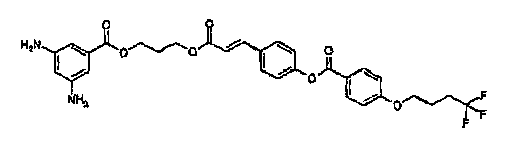 Polyamic acid 8 as white powder, [η] = 0.83 dUg.
6-{[((2E)-3-{4-[(4-(4,4,5,5,5-pentafluoropentoxy)benzoyl)oxy]phenyl}prop-2-enoyl)oxy]}hexyl 3,5-Diaminobenzoate 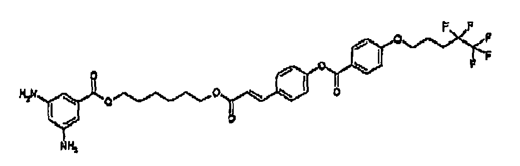 yield Polyamic acid 9 as white powder, [η] = 0.26 dUg.
6-{[((2E)-3-{4-[(4-trifluoromethoxybenzoyl)oxy]phenyl}prop-2-anoyl)oxy]}hexyl 3,5-Diaminobenzoate 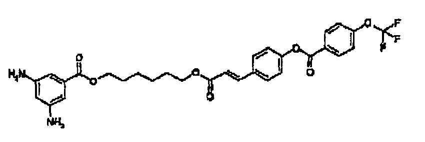 yield Polyamic acid 10 as white powder; [η] = 0.71 dUg
6-{[((2E)-3-{4-[(4-trifluoromethylbenzoyl)oxy]phenyl}prop-2-enoyl)oxy]}hexyl 3,5-Diaminobenzoate 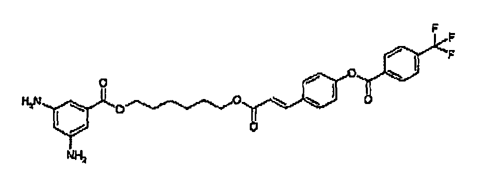 yield Polyamic acid 11 as white powder, [η] = 1.21 dUg 6-{[((2E)-3-{4-[(4-(2,2,3,3-tetrafluoropropoxy)benzoyl)oxy]phenyl}prop-2-enoyl)oxy]}hexyl 3,5-Diaminobenzoate 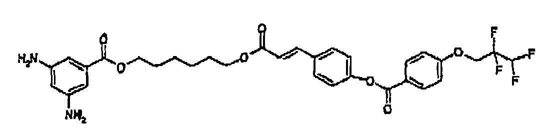 yield Polyamic acid 12 as white powder, [η] = 0.48 dL/g
6-{[((2E)-3-{4-[(4-(2,2,3,3-tetrafluoroethoxy)benzoy)oxy]phenyl}prop-2-enoyl)oxy]}hexyl 3,5-Diaminobenzoate 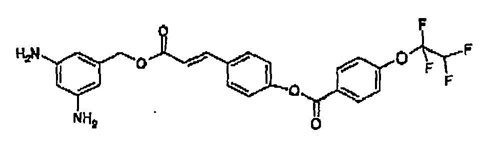 yield Polyamic acid 13 as white powder; [η] = 0.48 dL/g
3,5-Diaminobenzyl (2E) 3-{4-[(4-(4,4,5,5,5-pentafluoropentyloxy)benzoyl)oxy] phenyl}acrylate 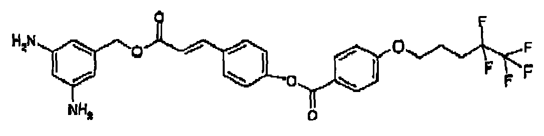 yield Polyamic acid 14 as white powder; [η] = 0.59 dUg
3,5-Diaminobenzyl (2E) 3-{4-[{4-(4,4,5,5,6,6,6-heptafluorohexyloxy)benzoyl}oxy] phenyl}acrylate 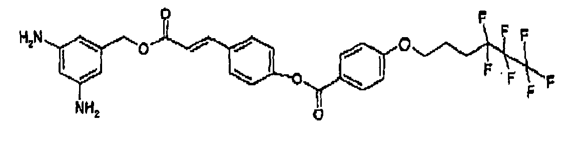 yield Polyamic acid 15 as white powder, [η] = 0.20 dUg
3,5-Diaminobenzyl (2E) 3-{4-[(4-(5,5,5-trifluorobutoxy)benzoyl)oxy]phenyl}acrylate 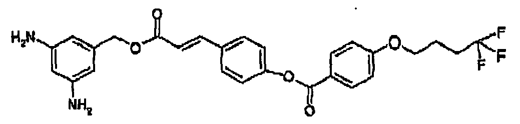 yield Polyamic acid 16 as white powder, **[**η] = 0.38 dUg
2-(2,4-Diaminophenyl)ethyl (2E) 3-{4-[(4-(4,4,4-trifluorobutoxy)benzoyl)oxy]phenyl} acrylate 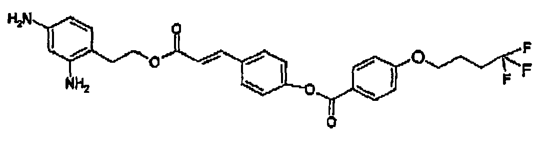 yield Polyamic acid 17 as white powder, [η] = 0.50 dUg
2-(2,4-Diaminophenyl)ethyl (2E) 3-{4[(4-(4,4,5,5,5-pentafluoropentyloxy)benzoyl)oxy] phenyl} acrylate 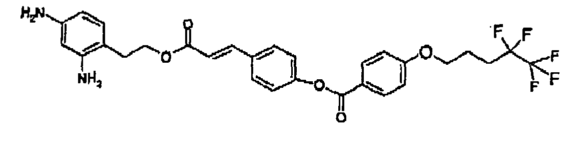 yield Polyamic acid 18 as white powder, [η] = 0.27 dUg
2-(2,4-Diaminophenyl)ethyl (2E) 3-{4-[(4-(4,4,5,5,6,6,6-heptafluorohexyloxy) benzoyl)oxy]phenyl} acrylate 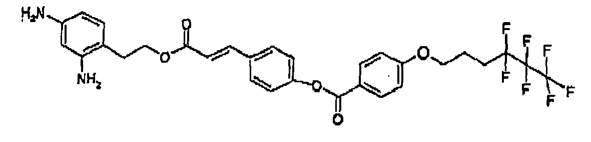 yield Polyamic acid 19 as white powder, [η] = 0.19 dUg
2-(2.4-DiaminophenyJ)ethyl (2E) 3-{4-[(4-(2,2,3,3-tetrafluoroethoxy)benzoyl)oxy] phenyl} acrylate 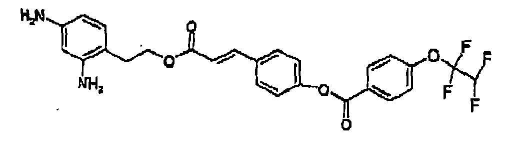 yield Polyamic acid 20 as white powder, [η] = 0.28 dUg
2,2-bis(4-aminobenzyl)-1,3 di[(2E)-3-{4-[(4-(4,4,4-trifluorobutoxy)benzoyl)oxy]phenyl}prop-2-enoyl] propanediol 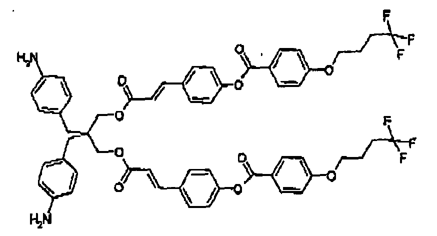 yield Polyamic acid 21 as white powder, [η] = 0.54 dUg

### EXAMPLE 6

Analogous to EXAMPLE 6 the following diamines were used for the preparation of Polyamic Acid with 2,3,5-tricarboxycyclopentylacetic acid dianhydride
3,5-Diaminobenzyl (2E) 3-{4[(4-(5,5,5-trifluorobutoxy)benzoyl)oxy]phenyl}acrylate 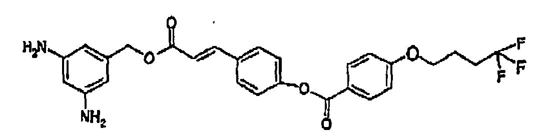 yield Polyamic acid 22 as white powder, [η] = 0.40 dUg
2,2-bis(4-aminobenzyl)-1,3 di[(2E)-3-{4-[(4-(4,4,4-trifluorobutoxy)benzoyl)oxy]phenyl}prop-2-enoyl] propanediol 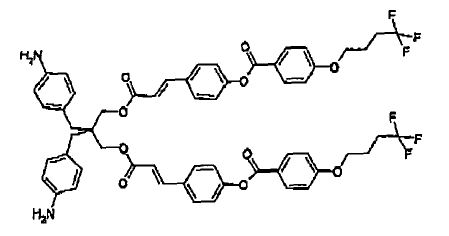 yield Polyamic acid 23 as white powder; [η]= 0.47 dL/g
2-(2,4-Diaminophenyl)ethyl (2E) 3-{4-[(4-(4,4,4-trifluorobutoxy)benzoyl)oxy]phenyl} acrylate 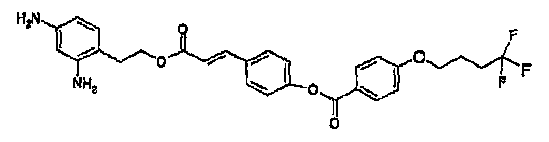 yield Polyamic acid 24 as white powder; [η] = 0.23 dUg
2-(2,4-Diaminophenyl)ethyl (2E) 3-{4-[(4-(4,4,5,5,5-pentafluoropentyloxy)benzoyl)oxy] phenyl} acrylate 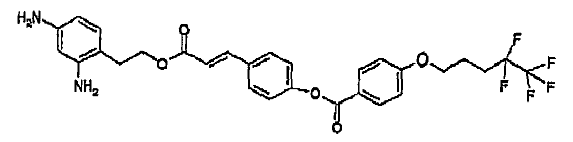 yield Polyamic acid 25 as white powder, [η] = 0.14 dL/g
3,5-Diaminobenzyl (2E) 3-{4-[(4-(5,5,5-trifluorobutoxy)benzoyl)oxy]phenyl}acrylate 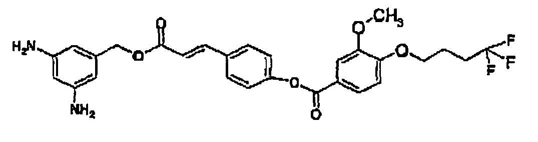 yield Polyamic acid 26 as white powder, [η] = 0.45 dL/g

### EXAMPLE 7

Analogous to EXAMPLE 6 the following tetracarboxylic acid dianhydride were used for the preparation of Polyamic Acid with of 6-{[((2E)-3-{4-[(4-(4,4,4-trifluorobutoxy)benzoyl)oxy]phenyl}prop-2-enoyl)oxy]}hexyl 3,5-Diaminobenzoate.
4-(2,5-dioxotetrahydrofuran-3-yl)tetrahydronaphthalene-1,2-dicarboxylicacid dianhydrid e Diaminobenzoate yield Polyamic acid 27 as white powder; [η]=0.15 dUg.
bicyclo[2.2.2]oct-7-ene-2,3,5,6-tetracarboxylic acid dianhydride yield Polyamic acid 28 as white powder; [η] = 0.11 dUg
2,3,5-tricarboxycyclopentylacetic acid dianhydride yield Polyamic acid 29 as white powder; [η] = 0.43 dUg
5-(2,5-dioxotetrahydrofuran-3-yl)-3-methyl-3-cyclohexene-1,2-dicarboxylic-acid dianhydride yield Polyamic acid 30 as white powder, [η] = 0.16 dL/g 4,4'-(hexafluoroisopropylidene)diphthalic acid dianhydride yield Polyamic acid 31 as white powder [n] = 0.51 dUg

### EXAMPLE 8

Analogous to EXAMPLE 6 the following tetracarboxylic acid dianhydride mixture were used for the preparation of Polyamic; Acid with of 6-{[((2E)-3-{4-[(4-(4,4,4-trifluorobutoxy)benzoyl)oxy]phenyl}prop-2-enoyl)oxy]}hexyl 3,5-Diaminobenzoate.

A mixture of 1 ,2,3.4-cyclobutantetracarboxylic acid dianhydride and 4-(2,5-dioxotetrahydrofuran-3-yl)tetrahydronaphthalene-1,2-dicarboxylic acid dianhydride 25;75 (mole ratio) yield Polyamic acid 32 as white powder, [η] =0.16 dUg

A mixture of 1,2,3,4-cyclobutantetracarboxylic acid dianhydride and 4-(2,5-dioxotetrahydrofuran-3-yl)tetrahydronaphthalene-1,2-dicarboxylic acid dianhydride 1:1 (mole ratio) yield Polyamic acid 33 as white powder, [η] = 0.20 dUg

A mixture of 1,2,3,4-cyclobutantetracarboxylic acid dianhydride and 4-(2,5-dioxotetrahydrofuran-3-yl)tetrahydronaphthalene-1,2-dicarboxylic acid dianhydride 75:25 (mole ratio) yield Polyamic acid 34 as white powder; [η] = 0.20 dL/g

A mixture of 1,2,3,4-cyclobutantetracarboxylic acid dianhydride and 4-(2,5-dioxotetrahydrofuran-3-yl)tetrahydronaphthalene-1,2-dicarboxylic acid dianhydride 90:10 (mole ratio) yield Polyamic acid 35 as white powder; [η] = 0.17 dUg

A mixture of 1,2,3,4-cydobutantetracarboxylic acid dianhydride and 5-(2,5-dioxotetrahydrofuran-3-yl)-3-methyl-3-cyclohexone-1,2-dicarboxylic-acid dianhydride 25:75 (mole ratio) yield Polyamic add 36 as white powder; [η] = 0.16 dUg

A mixture of 1,2,3,4-cyclobutantetracarboxylic acid dianhydride and 5-(2,5-dioxotatrahydrofuran-3-yl)-3-methyl-3-cyctohexene-1,2-dicarboxylic-acid dianhydride 1:1 (mole ratio) yield Polyamic acid 37 as white powder, [η] = 0.16 dUg

A mixture of 1,2,3,4-cyclobutantetracarboxylic acid dianhydride and 5-(2,5-dioxotatrahydrofuran-3-yl)-3-methyl-3-cyclohexene-1,2-dicarboxylic-acid dianhydride 75:25 (mole ratio) yield Polyamic acid 40 as white powder; [η] = 0.16 dUg

### EXAMPLE 9

Analogous to EXAMPLE 6 a mixture of 1,2,3,4-cyclobutantetracarboxylic acid dianhydride and
4-(2,5-dioxotetrahydrofuran-3-yl)tetrahydronaphthalene-1,2-dicarboxylic acid dianhydride 75:25 (mole ratio) and 3,5-Diaminobenzyl (2E) 3-{4-[(4-(4,4,5,5,5-pentafluoropentyloxy)benzoyl)oxy] phenyl}acrylate were used for the preparation to yield Polyamic acid 41 as white powder; [η] = 0.17 dUg

### EXAMPLE 10

Analogous to EXAMPLE 6 a mixture of 1,2,3,4-cyclobutantetracarboxylic acid dianhydride and
4-(2,5-dioxotetrahydrofuran-3-yl)tetrahydronaphthalene-1,2-dicarboxylic acid dianhydride 75:25 (mole ratio) and 3,5-Diaminobenzyl (2E) 3-{4-[(4-(4,4,4-trifluorebutoxy)benzoyl)oxy] phenyl}acrylate were used for the preparation to yield Polyamic acid 42 as white powder; [η] = 0.24 dUg

### EXAMPLE 11

Analogous to EXAMPLE 6 a mixture of 1,2,3,4-cyclobutantetracarboxylic acid dianhydride and
4-(2,5-dioxotetrahydrofuran-3-yl)tetrahydronaphthalene-1,2-dicarboxylic acid dianhydride 75:25 (mole ratio) and 2-(2,4-Diaminophenyl)ethyl (2E) 3{4-[(4-{4,4,5,5,5-pentafluoropentyloxy)benzoyl)oxy] phenyl} acrylate were used for the preparation to yield Polyamic acid 43 as white powder; [η] = 0.11 dL/g

### EXAMPLE 12

Analogous to EXAMPLE 6 a mixture of 2,2-bis(4-aminobenzyl)-1,3 di[(2E)-3-{4-[(4-(4,4,4-trifluorobutoxy)benzoyl)oxy]phenyl}prop-2-enoyl] propanediol and 6-{[((2E)-3-{4-[(4-(4,4,4-trifluorobutoxy)benzoyl)oxy]phenyl}prop-2-enoyl)oxy]}hexyl 3,5-Diaminobenzoate1:1 (mole ratio) and 1,2,3,4-cyclobutantetracarboxylic acid dianhydride were used for the preparation to yield Polyamic acid 45 as white powder; [η] = 0.98 dUg

### EXAMPLE 13

Analogous to EXAMPLE 6 a mixture of 2,2-bis(4-aminobenzyl)-1,3 di[(2E)-3-{4-[(4-(4,4,4-trifluorobutoxy)benzoyl)oxy]phenyl}prop-2-enoyl] propanediol and 4,4'-Diaminodiphenylmethane 80:20 (mole ratio) and 1,2,3,4-cyclobutantetracarboxylic acid dianhydride were used for the preparation to yield Polyamic acid 46 as white powder, [η] = 1.00 dUg

### EXAMPLE 14

### Polymerisation Step B (Formation of the Polyimide)

0.50 g of Polyamic Acid No. 1 obtained in above EXAMPLE 6 were dissolved in 3 ml of 1-methyl-2-pyrrolidon (NMP). Thereto were added 0.28 g (3.57 mmol) of pyridine and 364 mg (3.57 mmol) acetic acid anhydride, and the dehydration and ring closure was carried out at 80°C for 2 h. The polymer mixture was diluted with 1.5 ml NMP, precipitated into 100 ml diethyl ether and collected by filtration. The polymer was reprecipitated from THF (10 ml) into 200 ml water to yield, after drying at room temperature under vacuum, 0.55 g Polyimide No 1; [η] = 0.50 dUg

Analogous to EXAMPLE 14 the following polyamic acid were used for the preparation of polyimide
Polyamic acid 2 yield Polyimide 2 as white powder; [η] = 0.24 dUg
Polyamic acid 5 yield Polyimide 5 as white powder; [η] = 0.36 dUg
Polyamic acid 13 yield Polyimide 14 as white powder; [η] = 0.88 dUg
Polyamic acid 14 yield Polyimide 13 as white powder, [η] = 0.48 dUg
Polyamic, acid 15 yield Polyimide 15 as white powder; [η] = 0.20 dUg
Polyamic acid 16 yield Polyimide 16 as white powder; [η] = 0.27 dL/g
Polyamic acid 17 yield Polyimide 17 as white powder, [η] = 0.29 dUg
Polyamic acid 18 yield Polyimide 18 as white powder, [η] = 0,28 dL/g
Polyamic acid 19 yield Polyimide 19 as white powder, [η] = 0.19 dUg
Polyamic acid 20 yield Polyimide 20 as white powder; [η] = 0.28 dUg
Polyamic acid 21 yield Polyimide 21 as white powder, [η] = 0.63 dUg
Polyamic acid 22 yield Polyimide 22 as white powder, [η] = 0.43 dUg
Polyamic acid 24 yield Polyimide 24 as white powder, [η] = 0.20 dUg
Polyamic acid 25 yield Polyimide 25 as white powder; [η] = 0.14 dUg
Polyamic acid 29 yield Polyimide 29 as white powder; [η] = 0.40 dUg
Polyamic acid 34 yield Polyimide 34 as white powder; [η] = 0.21 dL/g
Polyamic acid 41 yield Polyimide 41 as white powder; [η] = 0.14 dUg
Polyamic acid 42 yield Polyimide 42 as white powder; [η] = 0.12 dL/g

### Comparative Synthesis Example 1

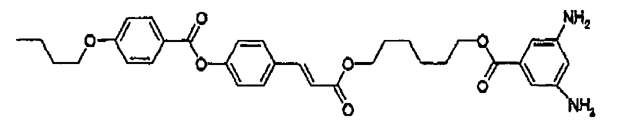

Analogous to example 21 15{[((2E)-3-{4-[(4-butoxybenzoyl)oxy]phenyl}pop-2-enoyl)oxy]}hexyl 3,5-Diaminobenzoate were synthesized.

### Comparative Polymerisation 1

The preparation was carried out analogously to *Synthesis Example* 6 using 920.2 mg (1.683. mmol) 6-{[((2E)-3-{4-[(4-butoxybenzoyl)oxy]phenyl}prop-2-enoyl)oxy]}hexyl 3,5-Diaminobenzoate, 330.1 mg 1.683 mmol) 1,2,3,4-cyclobutantetracarboxylic acid dianhydride to yield 1.01 g *Comparative Polyamic Acid* 1; [η] = 0. 25 dUg

### Comparative Synthesis Example 2

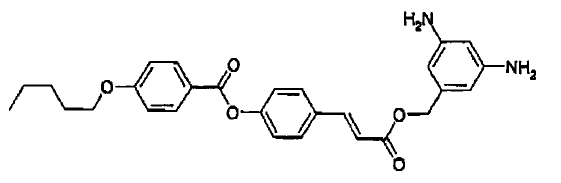

Analogous to example 3 3,5-Diaminobenzyl (2E) 3-{4-[(4-pentyloxy)benzoyl]oxy} phenyl}acrylate were synthesized.

### Comparative Polymerisation 2

The preparation was carried out analogously to *Synthesis Example* 6 using 1.0390 g (2.15 mmol) 3,5-Diaminobenzyl (2E) 3-{4-[(4-pentyloxy)benzoyl]oxy} phenyl}acrylate 422.2 mg (2.15 mmol) 1,2,3,4-cyclobutantetracarboxylic acid dianhydride to yield 1.349 g *Comparative Polyamic Acid 2;* [η] = 0. 87 dL/g

### Example for the Production of an orientation layer having a defined angle of tilt

2% solution of *Polyamic acid* 1 in cyclopentanone was filtered over a 0.2 µm Teflon filter and applied to a glass plate, which had been coated with indium-tin oxide (ITO), in a spin-coating apparatus at 3000 rev./min. in the course of 60 seconds. The resulting film was then predried for 15 minutes at 130 °C and then imidized for 1 hour at 200 °C to form a polyimide film. The so obtained LPP film was irradiated for 30mJ/cm2 with linearly polarised UV light, the direction of incidence of the light being inclined by 20° to 40° relative to the plate normal. The direction of polarisation of the light was kept in the plane defined by the direction of incidence of the light and the plate normal. From both plates a cell of 20 µm spacing was built such that the illuminated surfaces were facing each other and the previous polarisation directions of illumination were parallel. The cell was then filled with liquid crystal mixture MLC6609 from Merck in the isotropic phase at 100°C. The cell was then gradually cooled to room temperature at a rate ranging from 0.1°C/min to 2 C/min. Between crossed polarisers a uniformly oriented liquid crystal layer was observed. The tilt angle of this parallel cell, by crystal rotation method, was 88.7°.

### Example for the Determination of the voltage holding ratio (VHR)

Two glass plates coated in accordance with the above example were irradiated perpendicularly during 4 minutes with linearly polarised . UV light. From both plates a cell of 10µm spacing was built such that the illuminated surfaces were facing each other and the previous polarisation directions of illumination were parallel. This cell was then maintained at 120° C under high vacuum for 14 hours and thereafter filled with TFT liquid crystal mixture MLC6610 from Merck *in vacuum* at room temperature. Between crossed polarisers a uniformly oriented liquid crystal layer was observed. Prior to testing the voltage holding ratio (VHR) the cell was first subjected to ageing for 50 hours at 120 C The voltage decay V (at T=20ms) of a voltage surge of 64 µs with Vₒ (V at t=0)=0.2V was then measured over a period of T=20ms. The voltage holding ratio then determined, given by VHR=Vᵣₘₛ(t=T)V₀,

**Results**

| | VHR=Vᵣₘₛ(t=T)/V₀ at room temperature | VHR=Vᵣₘₛ(t=T)/V₀ at 80°C |
|---|---|---|
| Polyamic acid 1 | 97% | 92 % |
| Comparative Polyamic acid 1 | 85% | 62 % |
| Polyamic acid 6 | 99% | 93 % |
| Comparative Polyamic acid 2 | 99% | 71% |
| Blend between Polyamic acid 1 and Polyimide 1 (75:25 Weight ratio) | 95% | 92% |

## Claims

1. Diamine compound of formula (1): 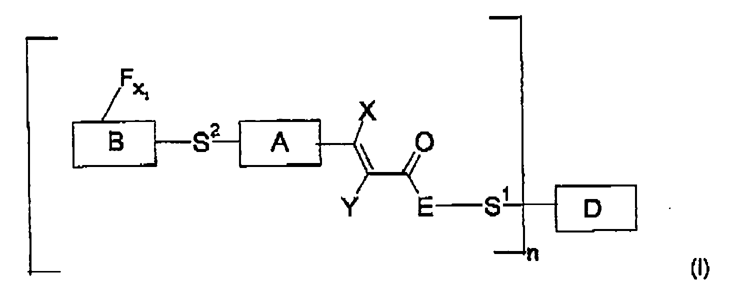 wherein,
A represents a unsubstituted or substituted carbocyclic or heterocyclic aromatic group selected from a monocyclic ring of five or six atoms, two adjacent monocyclic rings of five or six atoms, a bicyclic ring system of eight, nine or ten atoms, or a tricyclic ring system of thirteen or fourteen atoms;
F is fluorine, and
x₁ is an integer from 1 to 15,
B represents a straight-chain or branched C₁-C₁₆alkyl, which is unsubstituted or substituted by di-(C₁-C₁₆alkyl)amino, C₁-C₆alkyloxy, nitro, cyano and/or chlorine or fluorine; and wherein one or more -CH₂- group may independently be replaced by a linking group;
D represents unsubstituted or substituted aliphatic, aromatic or alicyclic diamine group having from 1 to 40 carbon atoms,
E represents an aromatic group, an oxygen atom, a sulphur atom, -NH-, -N(C₁-C₆alkyl)-, -CR²R³,
wherein R² and R³ are independently from each other hydrogen or a cyclic, straight-chain or branched, substituted or unsubstituted C₁-C₂₄alkyl, wherein one or more -CH₂- groups may be replaced by a linking group, and with the proviso that at least one of R² and R³ is not hydrogen;
S¹, S² each independently from each other represents a spacer unit;
X, Y each independently from each other represents hydrogen, fluorine, chlorine, cyano, unsubstituted or with fluorine substituted C₁-C₁₂alkyl, in which one or more -CH₂- groups may be replaced by a linking group;
n is 1, 2, 3 or 4,
with the proviso that if n is 2, 3, or 4, each A, B, x₁, D, E, S¹_{,} S², X, Y may be identical or different.

2. Diamine compound according to claim 1, wherein the linking group is selected from -O-, -CO, -CO-O-, -O-CO-, 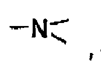 -NR¹-, -NR¹-CO-, -CO-NR¹-, -NR¹-CO-O-, -O-CO-NR -, -NR -CO-NR -, -CH=CH-, -C≡C-, -O-CO-O-, and -Si(CH₃)₂-O-Si(CH₃)₂-, and wherein:
R¹ represents a hydrogen atom or C₁-C₆alkyl;
with the proviso that oxygen atoms of linking groups are not directly linked to each other.

3. Diamine compound according to claim 1, wherein the spacer unit is a single bond, a C₁-C₂₄alkylen, wherein one or more -CH₂- groups may be replaced by a linking group and/or an alicyclic, aromatic, unsubstituted or substituted carbocyclic or heterocyclic group connected via bridging groups.

4. Diamine compound according to claim 3, wherein the bridging group is selected from -CH(OH)-, -CH₂-, -CH(CH₃)-, -C(CH₃)₂-, -CO-, -CH₂(CO)-, -SO-, -CH₂(SO)-, -SO₂-,-CH₂(SO₂)-, -COO-, -OCO-, -COCF₂-, -CF₂CO, -S-CO-, -CO-S-, -SOO-, -OSO-, -SOS-, -O-CO-O, -CH₂-CH₂-_{'} -OCH₂-, -CH₂O-, -CH=CH-, -C≡C-, -CH=CH-COO-, -OCO-CH=CH-, -CH=N-, -C(CH₃)=N-, -N=Nor a single bond.

5. Diamine compound according to any of the preceding claims, wherein
D is preferably selected from formula (III):
HN(R⁵)-(Sp¹)ₖ₁-(X¹)ₜ₁-(Z³-C³)ₐ₃-(Z⁴-C⁴)ₐ₄-(X²)ₜ₂-(Sp²)ₖ₂-N(R⁶)H (III)
wherein:
R5, R⁶ each independently from each other represents a hydrogen atom or C₁-C₆alkyl;
Sp¹, Sp² each independently represents an optionally substituted straight-chain or branched C₁-C₂₀alkylene, in which one or more CH₂-atoms may be replaced by a linking group, and
k¹, k² each independently is an integer having a value of 0 or 1; and
X¹, X² each independently represents a linking spacer, preferably selected from -O-, -S-, -NH-, N(CH₃)-, -CH(OH)-, -CO-, -CH₂(CO)-, -SO-, -CH₂(SO)-, -SO₂-, -CH₂(SO₂)-, -COO-, -OCO-, -OCO-O-, -S-CO-, -CO-S-, -SOO-, -OSO-, -SOS-, -CH₂-CH₂-, -OCH₂-, -CH₂O- -CH=CH-, or -C≡C- or a single bond; and
t¹, t² each independently is an integer having a value of 0 or 1; and
C³, C⁴ each independently represents a non-aromatic, aromatic, substituted or unsubstituted carbocyclic or heterocyclic group, and
Z³ represents a bridging group; and
Z⁴ represents a bridging group, or represents a substituted or unsubstituted straight-chain or branched C₁-C₂₀alkylene group, in which one or more -CH₂- groups may be replaced by a heteroatom and/or by a bridging group; and
a³, a⁴ are independently integers from 0 to 3, such that a³+a⁴ ≤ 4; and wherein
D is at least once linked to at least once group S¹ in formula (I) via group Sp¹ and/or group C³ and/or group Z⁴ and/or group C⁴ and/or group Sp²; and at least one of k¹, k², a³ and a⁴ is not equal to zero; and wherein
linking group is as defined in claim 2, and bridging group is as defined in claim 4; and wherein preferably, if n>1, then the side chains [i.e. structures (I) without the group D] can either be linked to the group D at one atomic position within group D, or they can be linked to group D at different atomic positions within group D.

6. Diamine compound according to claim 5, wherein C³, C⁴ independently from each other are selected from a compound of group G², wherein G² is: 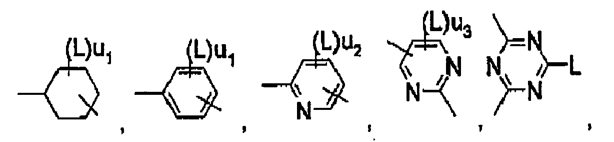 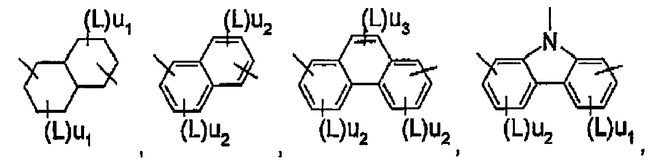 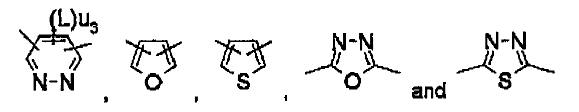 wherein:
"___" denotes the connecting bonds of C³ and C⁴ to the adjacent groups; and
L Is -CH₃, -COCH₃, -OCH₃, nitro, cyano, halogen, CH₂=CH-, CH₂=C(CH₃)-, CH₂=CH-(CO)O-, CH₂=CH-O-, CH₂=C(CH₃)-(CO)O- or CH₂=C(CH₃)-O-,
U₁ is an integer from 0 to 4; and
U₂ is an integer from 0 to 3; and
u₃ is an integer from 0 to 2.

7. Diamine compound according to any of the preceding claims, wherein D is a selected from a compound of the following group: 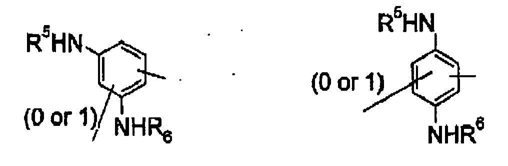 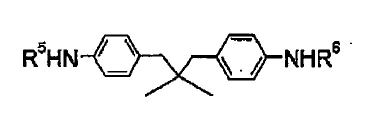 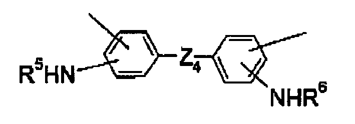 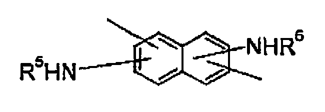 wherein
R⁵, R⁶ and Z⁴ are as defined in claim 5.

8. Diamine compound according to any of the preceding claims, wherein
S¹, S² each independently from each other represents a single bond or a cyclic, straight-chain or branched, substituted or unsubstituted C₁-C₂₄alkylen, in which one or more -CH₂- group may be replaced by a linking group; or a non-aromatic, aromatic, unsubstituted or substituted carbocyclic or heterocyclic group of formula (IV):
-(Z¹-C¹)ₐ₁-(Z²-C²)ₐ₂- (IV)
wherein:
C¹, C² each independently represents a non-aromatic, aromatic, optionally substituted carbocyclic or heterocyclic group, connected to each other via the bridging groups Z¹ and Z², and
Z¹, Z² each independently represents a bridging group, and
a¹, a² each independently represents an integer from 0 to 3, such that a¹+ a²≤ 4,
wherein the bridging groups Z¹ and Z² are as defined in claim 4.

9. Diamine compound (I) according to any preceding claim, wherein
A represents phenanthrylene, biphenylene, naphthylene, or phenylene, which is unsubstituted or mono- or poly-substituted by a halogen atom, hydroxy group and/or by a polar group, preferably nitro, cyano, carboxy; and/or by acryloylaxy, methacryloyloxy, vinyl, vinyloxy, allyl, allyloxy, and/or by a cyclic, straight-chain or branched C₁-C₁₂alkyl residue, which is unsubstituted, mono- or poly-substituted by fluorine and/or chlorine, wherein one or more -CH₂-groups may independently be replaced by a linking group and or an aromatic or an alicyclic group,
F is fluorine, and
x₁ is an integer from 1 to 10;
B represents a straight-chain or branched C₁-C₁₂alkylen, which is unsubstituted or substituted by di-(C₁-C₁₆alkyl)amino, C₁-C₆alkyloxy, nitro, cyano and/or chlorine or fluorine,
wherein one or more -CH₂- group may independently be replaced by a linking group selected from -O-, -CO-, -CO-O-, -O-CO-, -NR¹-, -NR¹-CO-, -CO-NR¹- and -CH=CH-, wherein:
R¹ represents a hydrogen atom or C₁-C₆alkyl;
with the proviso that oxygen atoms are not directly linked to each other;
D represents an optionally substituted aliphatic, aromatic or alicyclic diamine group having from 1 to 40 carbon atoms selected from formula (III), wherein:
k¹, k² are 0 or 1, and
t¹, t² are 0, and
R⁵, R⁶ are identical and represent a hydrogen atom, a methyl, an ethyl or an isopropyl group; and
C³, C⁴ Independently from each other are selected from compound of a group G²;
Z³ represents a group selected from -CH(OH)-, -CH(CH₃)-, -C(CH₃)₂-, -CO-,-COO-, -COCF₂-, -CF₂CO- or a single bond; and
Z⁴ has one of the meanings of Z³ or represents a substituted or unsubstituted straight-chain or branched C₁-C₂₀alkylene, in which one or more, preferably non-adjacent, -CH₂- groups may be replaced by a heteroatom and/or by an oxygen atom; and/or one or more carbon-carbon single bonds are replaced by a carbon-carbon double or a carbon-carbon triple bond;
a3, a⁴ each independently represents an integer from 0 to 2 such that a³ +-a⁴ ≤ 3:
E represents an phenylene, an oxygen atom or a -N(H)- group;
S¹ represents a single bond or a straight-chain or branched C₁-C₂₄alkylen,
wherein one or more -CH₂- groups may independently be replaced by a group represented by the formula (IV), wherein:
C¹, C² are selected from a compound of group G¹
and
Z¹, Z² each independently represents -O-, -CO-, -COO-, -OCO-, -COCF₂-, -CF₂CO-, -CH₂-CH₂-, -OCH₂-, -CH₂O-, -CH=CH-, -C≡C-, -CH=CH-COO-,-OCO-CH=CH- or a single bond; with the proviso that heteroatoms are not directly linked to each other, and
a¹, a² each independently represents an integer from 0 to 3, such that a¹ + a² ≤ 4;
S² represents a spacer unit such as a straight-chain or branched C₁-C₂₄alkylen, wherein one or more -CH₂- groups is independently replaced by a linking grouop or a group represented by the formula (IV), wherein:
C¹, C² are selected from a compound of group G¹ wherein group G¹ is: 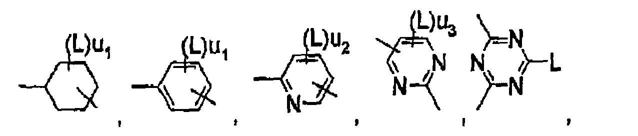 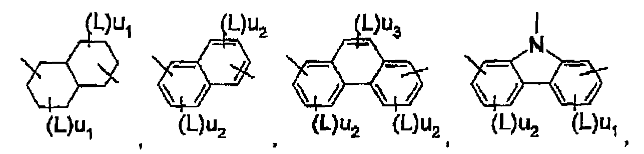 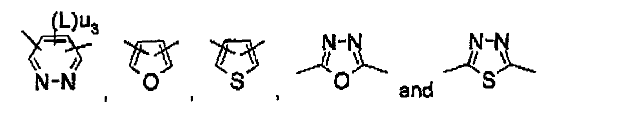 wherein:
"___" denotes the connecting bonds of C¹ and C² to the adjacent groups; and
L is -CH₃, -OCH₃, -COCH₃, nitro, cyano, halogen, CH₂=CH-, CH₂=C(CH₃)-, CH₂=CH-(CO)O-, CH₂=CH-O-, CH₂=C(CH₃)-(CO)O-, or CH₂=C(CH₃)-O-,
u₁ Is an integer from 0 to 4; and
u₂ is an integer from 0 to 3; and
u₃ is an integer from 0 to 2; and
Z¹, Z² each independently represents -O-, -CO-, -COO-, -OCO-, -COCF₂-, -CF₂CO-, -CH₂-CH₂-, -OCH₂-, -CH₂O-, -CH=CH-, -C≡C-, -CH=CH-COO-, -OCO-CH=CH- or a single bond; with the proviso that heteroatoms are not directly linked to each other, and
a¹, a² each independently represents an integer from 0 to 3, such that a¹+a²≤4;
X, Y are hydrogen atoms, and
n is 1, 2 or 3,
wherein diamine compound of formula (III) is as defined in claim 5; and compound of a group G² is as defined in claim 6; and group represented by the formula (IV), is as defined in claim 8;
With the proviso that if n is 2 or 3 each A, B, x₁, D, E, S¹ and S² may be identical or different.

10. Diamine compound (I) according to any of the preceding claim, wherein
A represents 1,4-phenylene, which is unsubstituted or mono- or poly-substituted by a halogen atom, and/or by acryloyloxy or methacryloyloxy, and/or by an alkoxy, alkylcarbonyloxy or an alkyloxycarbonyl group, having from 1 to 10 carbon atoms,
F is fluorine, and
x₁ is 1,2, 3,4. 5, 6. 7, 8 or 9;
B represents a straight-chain or branched C₁-C₆alkylen, wherein one -CH₂- group may independently be replaced by a group selected from -0-, -CO-, -CO-O-, -O-CO-, - and -CH=CH-, with the proviso that oxygen atoms are not directly linked to each other,
D represents an unsubstituted o-phenylenediamine, p-phenylenediamine, m-phenylenediamine, biphenyldiamine, aminophenyl-Z⁴-phenylamino, naphthylenediamine,
wherein
Z⁴ is as defined in claim 5;
E represents an oxygen atom;
S¹ represents a single bond or a straight-chain C₁-C₈alkylene group, wherein one -CH₂- groups may be may be replaced by -0-, -O(CO)-, -(CO)O-, -NR₁ CO-, -CONR₁-, wherein R₁ is hydrogen or C₁-C₆alkyl, or may be replaced by a group of formula (IV), wherein:
C¹, C² each independently represents 1,4-phenylene; and
Z¹, Z² each independently represents -COO-, -OCO-, -CH₂-CH₂-, -OCH₂-, -CH₂O-, -CH=CH-, -C=C-, -CH=CH-COO-, -OCO-CH=CH-, or a single bond; and
a¹, a² each independently represents 0 or 1, and
S² is replaced by a group of formula (IV), wherein:
C¹ represents a 1,4-phenylene which is unsubstituted or mono or poly-substituted by a halogen atom, and/or by an alkoxy, alkylcarbonyloxy or an alkyloxycarbonyl group, having form 1 to 10 Carbon atoms,
Z¹ represents -COO-, -OCO-, -CH₂-CH₂-,-CCH₂-, -CH₂O-, -CH=CH-, -C≡C-, -CH=CH-COO-, -OCO-CH=CH-, or a single bond;
a¹ represents 1, and
S² is linked to A via Z¹
X, Y are hydrogen atoms, and
n is 1 or 2,
with the proviso that if n is 2 each A, B, x₁, D, E, S¹ and S² may be identical or different.

11. Diamine compound according to any preceding claim, which is a compound of formulae (VI), (VII), (VIII), (IX), (X) and (XI) 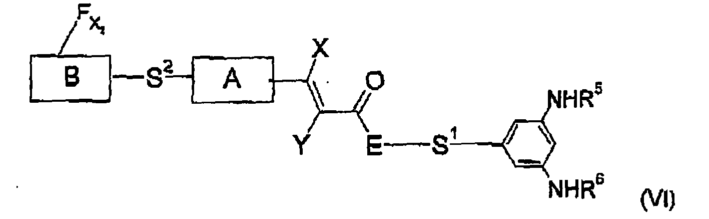 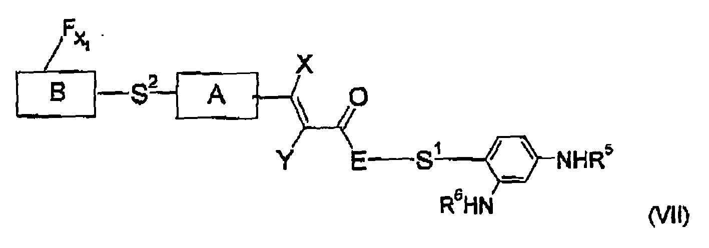 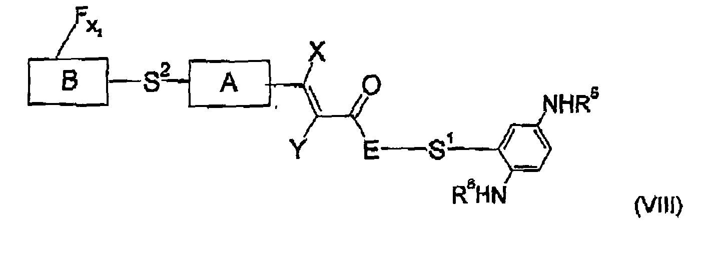 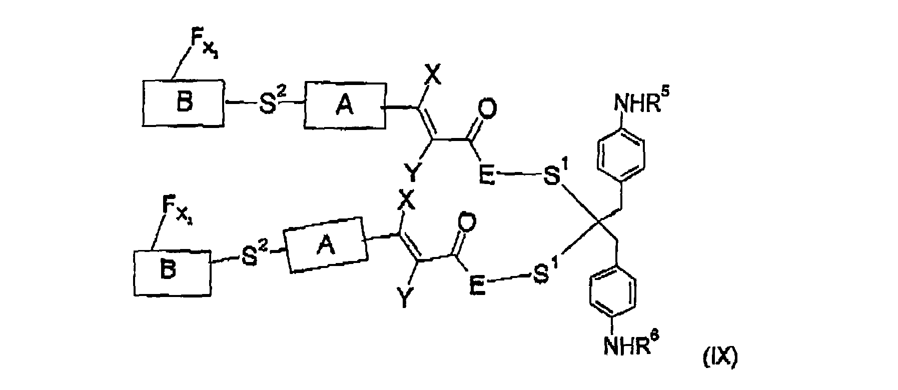 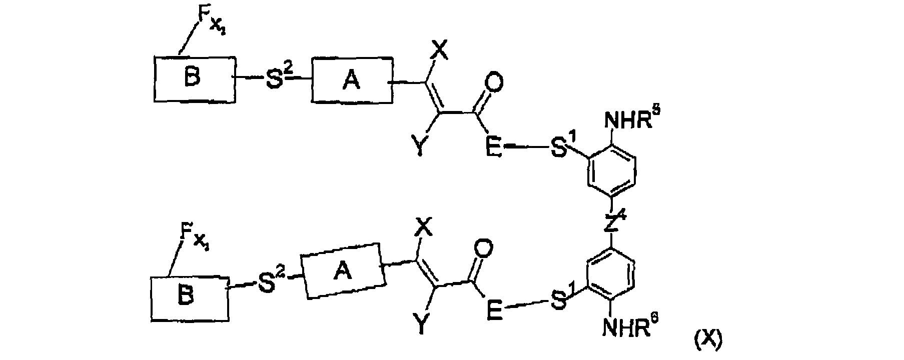 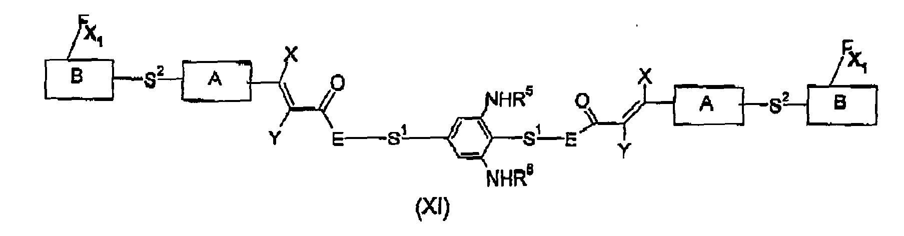 Wherein
A, B, x₁, n, D, E, S²,S¹, X and Y have the above given meanings as In claim 1 and R⁵, R⁶ have the same meanings as in claim 5; and Z⁴ has the same meaning as in claim 4.

12. Diamine compound according to any preceding claim, which is a compound of formula (XII) 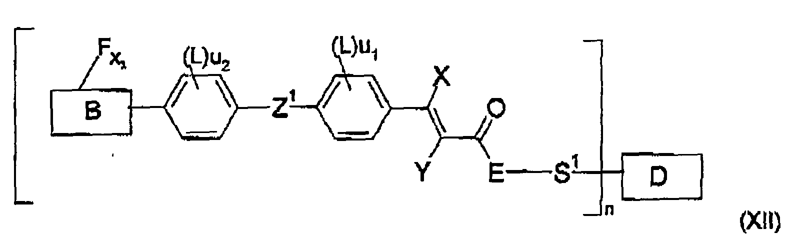 wherein X₁, n, D, E, S¹, X and Y have the above given meanings as in claim 1 and Z¹. L, u₁ and u₂ have the same meanings as in claim 9.

13. Diamine compound according to any preceding claim, which is a compound of formula (XIII) 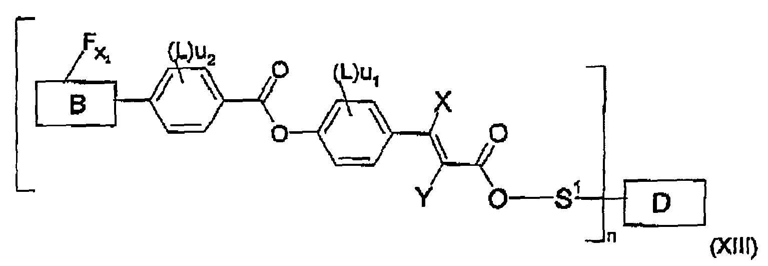 wherein x₁, n, D, S¹, X and Y have the above given meanings as in claim 1 and L, u₁ and u₂ have the same meanings as in claim 9.

14. A process for the preparation of diamine compounds (XII) and (XIII) as defined in claims 12 and 13 comprising contacting a acrylic acid compound of formula (XIV) 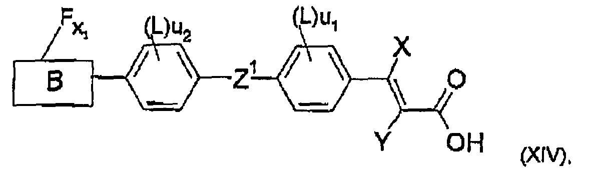 preferably 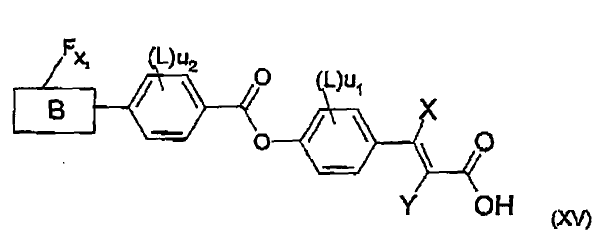 with a compound of formula (XVI) 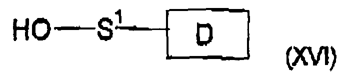 and optionally an additional other diamine,
wherein
F, x₁, B, S², X, Y, S¹ and D have the same meanings as in claim 1, and Z¹, L, u₁ and u₂ have the same meanings as in claim 9.

15. Acrylic acid of formulae (XIV) and (XV) as defined In claim 14.

16. Composition comprising diamine of compound (I) according to any of the preceding claims and optionally a further diamine or an additive.

17. Polymer material or oligomer material obtainable by reaction with a diamine of compound (I) according to any of the preceding claims.

18. Polymer material or oligomer material from the class of polyamic acids, polyamic acid esters or polyimides, and any mixtures thereof obtainable by the reaction of at least one Diamine compound represented by the formula (I) and optionally of one or more additional other Diamines with one or more tetracarboxylic acid anhydrides of the general formula (V) 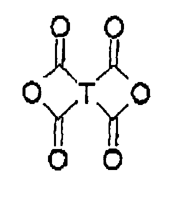 wherein:
T represents a tetravalent organic radical.

19. Polymer material or oligomer material according to claim 18, wherein the tetracarboxylic acid anhydrides of the general formula (V), wherein T is selected from:
1,1,4,4-butanetetracarboxylic acid dianhydride,
ethylenemaleic acid dianhydride,
1,2,3,4-cyclobutanetetracarboxylic acid dianhydride,
1,2,3,4-cyclopentanetetracarboxylic acid dianhydride,
2,3,5-tricarboxycyclopentylacetic acid dianhydride,
3,5,6-trioarboxynorbornylacetic acid dianhydride,
2,3,4,5-tetrahydrofurantetracarboxylic acid dianhydride,
rel-[1 S,5R,6R]-3-oxabicyclo[3.2.1]octane-2,4-dione-6-spiro-3'. (tetrahydrofuran2',5'-dione),
4-(2,5-dioxotetrahydrofuran-3-yl)tetrahydronaphthalene-1,2-dicarboxylicacid dianhydride,
5-(2,5-dioxotetrahydrofuran-3-yl)-3-methyl-3-cyclohexene-1,2-dicarboxylic-add dianhydride,
bicyclo[2.2.2]oct-7-ene-2,3,5,6-tetracarboxylic acid dianhydride,
bicyclo[2.2.2]octane-2,3,6,6-tetracarboxylic acid dianhydride,
1,8-dimethylbicyclo[2.2.2]oct-7-ene-2,3,5,6-tetracarboxylic acid dianhydride,
pyromellitic acid dianhydride,
3,3',4,4'-banzophenonetetracarboxylic acid dianhydride,
4,4'-oxydiphthalic add dianhydride,
3,3',4,4'-diphenylsulfonetetracarboxylic acid dianhydride,
1,4,5,8-naphthalenetetracarboxylic add dianhydride,
2,3,6,7-naphthalenetetracarboxylic acid dianhydride,
3,3',4,4'-dimethyldiphenylsilanetetracarboxylic acid dianhydride,
3,3',4,4'-tetraphenylsilanetetracarboxylic acid dianhydride,
1,2,3,4-furantetracarboxylic acid dianhydride,
4,4'-bis(3,4-dicarboxyphenoxy)diphenyl sulfide dianhydride,
4,4'-bis(3,4-dicarboxyphenoxy)diphenyl sulfone dianhydride,
4,4'-bis(3,4-dicarboxyphenoxy)dlphenylpropane dianhydride,
3,3',4,4'-blphenyltetracarboxylic acid dianhydride,
ethylene glycol bis(trimellitic acid) dianhydride,
4,4'-(1,4-phenylene)bis(phthalic acid) dianhydride,
4,4'-(1,3-phenylene)bis(phthalic acid) dianhydride,
4,4'-(hexafluoroisopropylidene)diphthalic acid dianhydride,
4,4'-oxydi(1,4-phenylene)bis(phthalic acid) dianhydride, and
4,4'-methylenedi(1,4-phenylene)bis(phthalic acid) dianhydride.

20. Composition comprising a polymer material or oligomer material according to claims 17, 18 or 19 diamine and an additive and/or other polymers, oligomers, monomers, photo-active polymers, photo-actlve oligomers and/or photo-active monomers.

21. Polymer or oligomer according to claims 17 to 19, wherein the one or more additional other diamine is selected from the group: ethytenediamine,
1,3-propylanediamine, 1,4-butylanediamine, 1,5-pentylenediamine,
1,6-hexylenediamine, 1,7-heptylenediamine, 1,8-octylenediamine,
1,9-nonylenediamine, 1,10-decylenediamine, 1,11-undecylenediamine,
1,12-dodecylenediamine, α,α'-diarmino-*m*-xylene, α,α'-diamino-*p*-xylene, (5-amino-2,2,4-trimethylcyclopentyl)methylamine, 1,2-diaminocyclohexane,
4,4'-diaminodicyclohexylmethane, 1,3-bis(methylamino)cyclahexane,
4,9-dioxadodecane-1,12-diamine, 3,5-diaminobenzoic acid methyl ester,
3,5-diaminobenzoic acid hexyl ester, 3,5-diaminobenzoic acid dodecyl ester,
3,5-diaminobenzoic acid isopropyl ester, 4,4'-methylenedianiline, 4,4'-ethylenedianiline,
4,4'-diamino,3,3'-dimethyldiphenylmethane, 3,3',5,5'-tetramethylbenzidine,
4,4'-diaminodiphenyl sulfone, 4,4'-diaminodiphenyl ether,
1,5-diaminonaphthalene,3,3'-dimethyl-4,4'-diaminobiphenyl,
3.4'-diaminodiphenyl ether, 3,3'-diaminobenzophenone, 4,4'-diaminobenzophenone,
4,4'-diamino-2,2'-dimethylbibenzyl, bis[4-(4-aminophenoxy)phenyl] sulfone,
1,4-bis(4-aminophenoxy)benzene, 1,3-bis(4-aminophenoxy)benzene,
1,3-bis(3-aminophenoxy)benzene, 2,7-diaminofluorene,
9,9-bis(4-aminophenyl)fluorene, 4,4'-methylene bis(2-chloroaniline),
4,4'-bis(4-aminophenoxy)biphenyl, 2,2',5,5'-tetrachloro-4,4'-diaminobiphenyl,
2,2'-dichloro-4,4'-diamino-5,5'-dimethoxybiphenyl, 3,3'-dimethoxy-4,4'-diaminobiphenyl,
4,4'-(1,4-phenyleneisopropylidene)bisaniline,
4,4'-(1,3-phenylensisopropylidene)bisaniline,
2,2-bis[4-(4-aminophenoxy)phenyl]propane,
2,2-bis[3-(4-aminophenoxy)phenyl]hexafluoropropane,
2,2-bis[3-amino-4-methylphenyl]hexafluoropropane,
2,2-bis(4-aminophenyl)hexafluoropropane.
2,2'-bis[4-(4-amino-2-trifluoromethylphenoxy)phenyl]hexafluoropropane,
4,4'-diamino-2,2'-bis(trifluoromethyl)biphenyl, and
4,4'-bis[(4-amino-2-trifluoromethyl)phenoxy]-2,3,5,6,2',3',5',6'-octafluorobiphenyl.

22. Polymer or oligomer according to any of claims 17 to 19 and 21, comprising as side-chains a photo-reactive group that can be photo-isomerized and/or photo-dimerized on exposure to visible light, UV light or laser light.

23. Polymer or oligomer according to claims 17 to 19 and 21 and 22, wherein at least 30%, preferably at least 75 % of the repeating units include a side chain with a photo-reactive group.

24. Polymer or oligomer according to any of claims 22 and 23, wherein the photo-reactive groups are able to undergo photo-cyclization, in particular [2+-2]-photo-cyclisation.

25. Polymer or oligomer according to any of claims 17 to 19 and 21 and 24, wherein the polymer or oligomer is a polymer gel or a polymer network, or an oligomer gel or an oligomer network, respectively.

26. Polymer or oligomer according to any of claims 17 to 19 and 21 and 25, with an intrinsic viscosity in the range of 0.05 to 10 dUg, preferably in the range of 0.05 to 5 dL/g.

27. Polymer or oligomer according to any of claims 17 to 19 and 21 and 26, containing from 2 to 2000 repeating units, especially from 3 to 200 repeating units.

28. Polymer or oligomer according to any of claims 17 to 19 and 21 and 27, in the form of a homopolymer or of a copolymer, preferably of a statistical copolymer.

29. Polymer or oligomer according to any of claims 17 to 19 and 21 and 28, wherein the polymer or oligomer is cross-linkable or cross-linked.

30. Polymer or oligomer according to any of claims 17 to 19 and 21 and 29, additionally comprising additives such as silane-containing compounds, one or more photo-sensitizers and/or one or more photo-radical generators and/or one or more cationic photo-initiators and/or cross-linking agents, preferably epoxy-containing cross-linking agents, most preferably selected from the group;
4,4'-methylene-bis-(N,N-diglycidylaniline), trimethylolpropane triglycidyl ether, benzene-1,2,4,5-tetracarboxylic acid 1,2,4,5-N,N'-diglycidyldiimide, polyethylene glycol diglycidyl ether, N,N-diglycidylcyclohexylamine.

31. Method for the preparation of a polymer or oligomer according to any of the claims 17 to 30, wherein in a polycondensation reaction a diamine compound according to any of claims 1-16 is reacted with one or more tetracarboxylic acid anhydrides of the general formula (V), optionally in the presence of one or more additional other diamines.

32. Method according to claim 31, wherein a poly-condensation reaction for the preparation of the polyamic acids is carried out in solution in a polar aprotic organic solvent, preferably selected from γ-butyrolactone, N,N-dimethylacetamide, N-methylpyrrolidone or N,N-dimethylformamide,

33. Method according to any of claims 31 and 32, wherein subsequent to the poly-condensation cyclisation with removal of water is carried out thermally under formation of a polyimide.

34. Method according to claim 33, wherein imidisation is carried out prior or after the application of the polymer or oligomer to a support.

35. Polymer or oligomer layer, in particular orientation layer, comprising at least one polymer or oligomer according to any of claims 17 to 19 and 21 and 30.

36. Method for the preparation of a polymer layer or oligomer layer according to claim 34, wherein one or more polymers or oligomers according to any of claims 17 to 19 and 21 and 29 is applied to a support, preferably from a solution of the polymer or oligomer material and subsequent evaporation of the solvent, and wherein, after any imidisation step which may be necessary, the polymer or oligomer or polymer mixture or oligomer mixture is cross-linked by irradiation with linearly polarized light.

37. Method according to claim 36, wherein the direction of orientation and the tilt angle within the polymer layer or oligomer layer is varied by controlling the direction of the irradiation of the linearly polarized light, and/or wherein by selectively irradiating specific regions of the polymer layer or oligomer layer specific regions of the layer are aligned.

38. Use of a polymer layer or oligomer layer according to claim 34, preferably in cross-linked form, as an orientation layer for liquid crystals.

39. Use according to claim 38 for the induction of vertical alignment of adjacent liquid crystalline layers, in particular for operating a cell in MVA mode.

40. Optical and electro-optical unstructured or structured constructional elements, preferably liquid crystal display calls, multi-layer and hybrid layer elements, comprising at least one polymer layer or oligomer layer according to claim 34.

41. Orientation layer, comprising at least one polymer layer or oligomer layer according to claim 34.
